(19) Europäisches Patentamt
European Patent Office
Office européen des brevets

(11) EP 2 993 239 A1

(12) EUROPEAN PATENT APPLICATION

(43) Date of publication:
**09.03.2016 Bulletin 2016/10**

(51) Int Cl.:
***C12Q 1/68** (2006.01)*

(21) Application number: **15175240.9**

(22) Date of filing: **18.05.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **16.05.2008 US 54098 P**

(62) Document number(s) of the earlier application(s) in accordance with Art. 76 EPC:
**09747766.5 / 2 281 069**

(71) Applicants:
- **AstraZeneca AB**
**151 85 Södertälje (SE)**
- **AstraZeneca UK Limited**
**London W1K 1LN (GB)**

(72) Inventors:
- **EMISON, Eileen**
**Princeton, NJ 08543 (US)**
- **DELMONTE, Terrye Aigeldinger**
**Princeton, NJ 08543 (US)**
- **RANADE, Koustubh**
**Princeton, NJ 08543 (US)**
- **RODRIGUES, Lisa Renee**
**Princeton, NJ 08543 (US)**
- **SIMONSEN, Katy Lynn Moore**
**Princeton, NJ 08543 (US)**

(74) Representative: **Vossius & Partner**
**Patentanwälte Rechtsanwälte mbB**
**Siebertstrasse 3**
**81675 München (DE)**

Remarks:
- The complete document including Reference Tables and the Sequence Listing can be downloaded from the EPO website
- This application was filed on 03-07-2015 as a divisional application to the application mentioned under INID code 62.

# (54) METHODS FOR IDENTIFYING SUBJECTS WITH AN INCREASED LIKELIHOOD OF RESPONDING TO DPP-IV INHIBITORS

(57) The invention provides novel *in vitro* diagnostic methods for identifying subjects or patients who may have an increased likelihood of responding to DPP-IV inhibitor therapy. The invention also provides novel polynucleotides associated with increased responsiveness of a patient to DPP-IV inhibition. Polynucleotide fragments which comprise at least one polymorphic locus, are also provided. Allele-specific primers and probes which hybridize to these polymorphic regions, and/or which comprise at least one polymorphic locus are also provided. The polynucleotides, primers, and probes of the invention are useful in phenotype correlations, medicine, and genetic analysis.

EP 2 993 239 A1

## Description

### PRIORITY

The present invention claims priority to United States Provisional Patent Application No. 61/054098, filed in the United States Patent and Trademark Office on May 16, 2008, the entire contents of which are incorporated herein by reference.

### FIELD OF THE INVENTION

The invention provides novel *in vitro* diagnostic methods for identifying subjects who may have an increased likelihood of responding to DPP-IV inhibitor therapy. The invention also provides novel polynucleotides associated with increased responsiveness of a subject to DPP-IV inhibition. Polynucleotide fragments corresponding to the genomic and/or coding regions of these polynucleotides, that comprise at least one polymorphic locus per fragment, are also provided. Allele-specific primers and probes that hybridize to these polymorphic regions, and/or that comprise at least one polymorphic locus are also provided. The polynucleotides, primers, and probes of the invention are useful in diagnostic methods, phenotype correlations, medicine, and genetic analysis.

### BACKGROUND OF THE INVENTION

At least 171 million people worldwide suffer from type II diabetes (NIDDM), which is characterized by hyperglycemia due to excessive hepatic glucose production and peripheral insulin resistance. Hyperglycemia is considered to be the major risk factor for the development of diabetic complications, and is likely to contribute directly to the impairment of insulin secretion seen in advanced NIDDM. Thus, consistent control of plasma glucose levels in NIDDM patients can offset the development of diabetic complications and beta cell failure seen in advanced disease.

Maturity Onset Diabetes of the Young (MODY) is characterized by an autosomal dominantly-inherited, early onset form of non-insulin dependent diabetes mellitus. The mean age at time of diagnosis is 23 years, and approximately one-third of patients with MODY develop progressive β-cell failure requiring insulin-replacement therapy (Pearson et al., 2000, Diabet Med. 17:543-545).

Incretins are hormones released from the gastrointestinal tract in response to nutrient ingestion that potentiate glucose-stimulated insulin secretion from islet beta cells. The two predominant incretins are glucagon-like peptide (GLP)-1 and glucose-dependent insulinotropic peptide (GIP). Unlike other insulinotropic agents, these two peptides stimulate insulin secretion in a glucose-dependent manner. In light of their beneficial actions, GLP-1 and GIP represent potential therapeutic agents for the treatment of type 2 diabetes. However, because exogenous GIP is comparatively less effective than GLP-1 at stimulating insulin secretion in type 2 diabetics, much of the current research has focused on enhancing GLP-1 action for the treatment of type 2 diabetes.

GLP-1 exerts a number of other biological actions that contribute to its ability to lower glucose, including inhibition of gastric emptying, which reduces meal-associated increases in glycemic excursion. GLP-1 also inhibits glucagon secretion and suppresses food intake in both diabetic and nondiabetic humans. Furthermore, due to its ability to stimulate beta-cell proliferation and neogenesis, as well as its ability to inhibit apoptosis, GLP-1 has the potential to preserve or enhance beta-cell function in human subjects with type 2 diabetes,. The therapeutic potential of native GLP-1 is limited, however, by its short physiologic half-life following exogenous administration, due in part to its rapid inactivation by the enzyme dipeptidyl peptidase (DPP)-IV and to renal clearance. As a result, preventing the degradation of native GLP-1 by inhibiting the activity of the DPP-IV enzyme has emerged as a therapeutic strategy for enhancing endogenous GLP-1 action in vivo.

DPP-IV is a ubiquitously expressed serine protease that exhibits postproline or alanine peptidase activity, thereby generating biologically inactive peptides via cleavage at the N-terminal region after X-proline or X-alanine. Because both GLP-1 and GIP have an alanine residue at position 2, they are substrates for DPP-IV. Thus, DPP-IV inhibitors interfere with the degradation of incretins like GLP-1, thereby increasing the amount of insulin secreted by the pancreas. By enhancing endogenous incretin action, DPP-IV inhibitors are able to (1) lower blood glucose in a glucose-dependent manner; (2) enhance beta-cell mass by promoting proliferation, neogenesis, and survival; and (3) promote satiety, thereby reducing food intake and, subsequently, body weight.

Currently, DPP-IV inhibitors are being developed to treat type II diabetes, however, many factors remain to be addressed, including the relative importance of their effects on GLP-1 versus GIP, long-term efficacy, side effects, whether they are weight-neutral or associated with weight loss in certain circumstances, and their effects on other DPP-IV substrates.

It is known that individual phenotypes with respect to drug response or toxicity often result from genetic variations that alter drug metabolism. Thus, in addition to other studies performed with DPP-IV inhibitors, there is a need in the art

to identify genetic polymorphisms of genes known to be associated with insulin sensitivity that can predict patient responsiveness to DPP-IV inhibitors, in an effort to improve treatment and to avoid unnecessary side-effects or ineffective treatment of diabetes or other metabolic diseases and disorders.

## SUMMARY OF THE INVENTION

The invention provides reagents and methods relating to detecting genetic polymorphisms in SEQ ID NO: 1, SEQ ID NO: 3, SEQ ID NO: 5, and SEQ ID NO: 63 that can be used to identify patients most likely to respond to DPP-IV inhibition. Genotypes of such polymorphisms can be predictive of an individual's likelihood of responding to DPP-IV inhibition and can be used to establish a treatment regimen optimized for each individual, wherein the presence of variant gene sequences at particular positions of certain genes has been associated as set forth herein with a decreased likelihood of favorable responsiveness to administration of a DPP-IV inhibitor or a DPP-IV inhibitor in combination with other oral antidiabetic therapy in an individual with NIDDM bearing any of these various variant gene sequences.

The invention provides methods for determining individuals more likely to have a favorable response to the administration of a therapeutically-effective amount of a DPP-IV inhibitor or a DPP-IV inhibitor in combination with other oral antidiabetic therapy, comprising the step of determining whether the individual harbors either the reference allele or variant allele at one or more polymorphic loci of SEQ ID NO: 1, SEQ ID NO: 3, SEQ ID NO: 5, and SEQ ID NO: 63, wherein an individual harboring the reference allele(s) of a TEKT5, GRINL1A, HSD11B1 or CHST10 gene would be more likely to have a favorable response to an administered DPP-IV inhibitor relative to an individual harboring a variant allele(s) of a TEKT5, GRINL1A, HSD11B1 or CHST10 gene as disclosed herein.

In a particular embodiment, an individual harboring the reference thymidine allele at position 87,992 of SEQ ID NO: 1 (the GRINL1A gene) is more likely to have a favorable response to an administered DPP-IV inhibitor or a DPP-IV inhibitor in combination with other oral antidiabetic therapy relative to an individual harboring the variant cytidine allele at position 87,992 of SEQ ID NO: 2. Thus, in certain embodiments of the invention, methods for determining whether an individual is more likely to have a favorable response to the administration of a therapeutically-effective amount of a DPP-IV inhibitor or a DPP-IV inhibitor in combination with other oral antidiabetic therapy comprise the step of determining whether the individual harbors the reference thymidine allele at position 87,992 of SEQ ID NO: 1 of the GRINL1A gene.

In another embodiment, an individual harboring the reference cytidine allele at position 34,403 of SEQ ID NO: 3 (the HSD11B1 gene) is more likely to have a favorable response to an administered DPP-IV inhibitor or a DPP-IV inhibitor in combination with other oral antidiabetic therapy relative to an individual harboring the variant thymidine allele at position 34,403 of SEQ ID NO: 4. Thus, in certain embodiments of the invention, methods for determining whether an individual is more likely to have a favorable response to the administration of a therapeutically-effective amount of a DPP-IV inhibitor or a DPP-IV inhibitor in combination with other oral antidiabetic therapy comprise the step of determining whether the individual harbors the reference cytidine allele at position 34,403 of SEQ ID NO: 3 of the HSD11B1 gene.

In other embodiments,, an individual harboring the reference guanosine allele at position 24,707 of SEQ ID NO: 5 (the CHST10 gene) is more likely to have a favorable response to an administered DPP-IV inhibitor or a DPP-IV inhibitor in combination with other oral antidiabetic therapy relative to an individual harboring the variant adenosine allele at position 24,707 of SEQ ID NO: 6 (the CHST10 gene). Likewise, as disclosed herein, an individual harboring the reference cytidine allele at position 34,078 of SEQ ID NO: 5 is more likely to have a favorable response to an administered DPP-IV inhibitor or a DPP-IV inhibitor in combination with other oral antidiabetic therapy relative to an individual harboring the variant thymidine allele at position 34,078 of SEQ ID NO: 7 (the CHST10 gene). Also, as disclosed herein, an individual harboring the reference guanosine allele at position 35,799 of SEQ ID NO: 5 is more likely to have a favorable response to an administered DPP-IV inhibitor or a DPP-IV inhibitor in combination with other oral antidiabetic therapy relative to an individual harboring the variant cytidine allele at position 35,799 of SEQ ID NO: 8 (the CHST10 gene). Also, as disclosed herein, an individual harboring the reference adenosine allele at position 38,709 of SEQ ID NO: 5 is more likely to have a favorable response to an administered DPP-IV inhibitor or a DPP-IV inhibitor in combination with other oral antidiabetic therapy relative to an individual harboring the variant guanosine allele at position 38,709 of SEQ ID NO: 9 (the CHST10 gene). In addition, as disclosed herein, an individual harboring the reference cytidine allele at position 38,947 of SEQ ID NO: 5 his more likely to have a favorable response to an administered DPP-IV inhibitor or a DPP-IV inhibitor in combination with other oral antidiabetic therapy relative to an individual harboring the variant adenosine allele at position 38,947 of SEQ ID NO: 10 (the CHST10 gene). In addition, as disclosed herein, an individual harboring the reference thymidine allele at position 41,180 of SEQ ID NO: 5 is more likely to have a favorable response to an administered DPP-IV inhibitor or a DPP-IV inhibitor in combination with other oral antidiabetic therapy relative to an individual harboring the variant cytidine allele at position 41,180 of SEQ ID NO: 11 (the CHST10 gene). Thus, in certain embodiments of the invention, methods for determining whether an individual is more likely to have a favorable response to the administration of a therapeutically-effective amount of a DPP-IV inhibitor or a DPP-IV inhibitor in combination with other oral antidiabetic therapy comprise the step of determining whether the individual harbors the reference allele at position 24,707, 34,078, 35,799, 38,709, 38,947, and/or 41,180 of SEQ ID NO: 5 of the CHST10 gene.

In further embodiments, an individual harboring the reference thymidine allele at position 26,472 of SEQ ID NO: 63 (the TEKT5 gene) is more likely to have a favorable response to an administered DPP-IV inhibitor or a DPP-IV inhibitor in combination with other oral antidiabetic therapy relative to an individual harboring the variant cytidine allele at position 26,472 of SEQ ID NO: 64. Thus, in certain embodiments of the invention, methods for determining whether an individual is more likely to have a favorable response to the administration of a therapeutically-effective amount of a DPP-IV inhibitor or a DPP-IV inhibitor in combination with other oral antidiabetic therapy comprise the step of determining whether the individual harbors the reference thymidine allele at position 26,472 of SEQ ID NO: 63 of the TEKT5 gene.

The methods also can be used to determine whether the individual may respond to a lower level of administered DPP-IV inhibitor or a DPP-IV inhibitor in combination with other oral antidiabetic therapy comprising the step of determining whether the individual harbors either the reference allele or variant allele at one or more polymorphic loci of TEKT5, GRINL1A, HSD11B1 or CHST10 gene, wherein an individual harboring the reference allele of TEKT5, GRINL1A, HSD11B1, or CHST10 gene, including, for example, the reference allele ("T") found at position 26,472 of SEQ ID NO: 63 (the TEKT5 gene), the reference allele ("A") found at position 87,712 of SEQ ID NO: 1 (the GRINL1A gene), the reference allele ("T") found at position 87,992 of SEQ ID NO: 1, the reference allele ("A") found at position 89,441 of SEQ ID NO: 1, the reference allele ("G") found at position 89,662 of SEQ ID NO: 1, the reference allele ("G") found at position 89,853 of SEQ ID NO: 1, the reference allele ("G") found at position 93,598 of SEQ ID NO: 1, the reference allele ("G") found at position 93,955 of SEQ ID NO: 1, the reference allele ("C") found at position 34,403 of SEQ ID NO: 3 (the HSD11B1 gene), or the reference allele ("G") found at position 24,707 of SEQ ID NO: 5 (the CHST10 gene), the reference allele ("C") found at position 34,078 of SEQ ID NO: 5, the reference allele ("G") found at position 35,799 of SEQ ID NO: 5, the reference allele ("A") found at position 38,709 of SEQ ID NO: 5, the reference allele ("C") found at position 38,947 of SEQ ID NO: 5, and/or the reference allele ("T") found at position 41,180 of SEQ ID NO: 5, is more likely to respond to a lower level of administered DPP-IV inhibitor relative to an individual harboring the corresponding variant allele of a TEKT5, GRINL1A, HSD11B1 or CHST10 gene, including, for example, the variant allele ("C") found at position 26,472 of SEQ ID NO: 64 (the TEKT5 gene), the variant allele ("G") found at position 87,712 of SEQ ID NO: 73 (the GRINL1A gene), the variant allele ("C") found at position 87,992 of SEQ ID NO: 2 (the GRINL1A gene), the variant allele ("G") found at position 89,441 of SEQ ID NO: 69 (the GRINL1A gene), the variant allele ("A") found at position 89,662 of SEQ ID NO: 70 (the GRINL1A gene?), the variant allele ("A") found at position 89,853 of SEQ ID NO: 71 (the GRINL1A gene), the variant allele ("A") found at position 93,598 of SEQ ID NO: 74 (the GRINL1A gene) the variant allele ("C") found at position 93,955 of SEQ ID NO: 72 (the GRINL1A gene), the variant allele ("T") found at position 34,403 of SEQ ID NO: 4 (the HSD11B1 gene), the variant allele ("A") found at position 24,707 of SEQ ID NO: 6 (the CHST10 gene), the variant allele ("T") found at position 34,078 of SEQ ID NO: 7 (the CHST10 gene), the variant allele ("C") found at position 35,799 of SEQ ID NO: 8 (the CHST10 gene), the variant allele ("G") found at position 38,709 of SEQ ID NO: 9 (the CHST10 gene), the variant allele ("A") found at position 38,947 of SEQ ID NO: 10 (the CHST10 gene)and/or the variant allele(s) ("C") found at position 41,180 of SEQ ID NO: 11 (the CHST10 gene).

Likewise, the invention provides methods for determining whether an individual is less likely to have a favorable response to the administration of a therapeutically-effective amount of a DPP-IV inhibitor or a DPP-IV inhibitor in combination with other oral antidiabetic therapy, comprising the step of determining whether the individual harbors either the reference allele or variant allele at one or more polymorphic loci of a TEKT5, GRINL1A, HSD11B1 or CHST10 gene, wherein an individual harboring the variant allele at one or more polymorphic loci of a TEKT5, GRINL1A, HSD11B1 or CHST10 gene is less likely to have a favorable response to an administered DPP-IV inhibitor or a DPP-IV inhibitor in combination with other oral antidiabetic therapy relative to an individual harboring the reference allele of a TEKT5, GRINL1A, HSD11B1 or CHST10 gene.

As disclosed herein, an individual harboring the variant thymidine allele at position 34,403 of SEQ ID NO: 4 (the HSD11B1 gene) is less likely to have a favorable response to an administered DPP-IV inhibitor or a DPP-IV inhibitor in combination with other oral antidiabetic therapy relative to an individual harboring the reference cytidine allele at position 34,403 of SEQ ID NO: 3. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant thymidine allele at position 34,403 of SEQ ID NO: 4 of the HSD11B1 gene.

As disclosed herein, an individual harboring the variant adenosine allele at position 24,707 of SEQ ID NO: 6 (the CHST10 gene) is less likely to have a favorable response to an administered DPP-IV inhibitor relative to an individual harboring the reference guanosine allele at position 24,707 of SEQ ID NO: 5. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant adenosine allele at position 24,707 of SEQ ID NO: 6 of the CHST10 gene.

As disclosed herein, an individual harboring the variant thymidine allele at position 34,078 of SEQ ID NO: 7 (the CHST10 gene) is less likely to have a favorable response to an administered DPP-IV inhibitor or a DPP-IV inhibitor

in combination with other oral antidiabetic therapy relative to an individual harboring the reference cytidine allele at position 34,078 of SEQ ID NO: 5. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant thymidine allele at position 34,078 of SEQ ID NO: 7 of the CHST10 gene.

As disclosed herein, an individual harboring the variant cytidine allele at position 35,799 of SEQ ID NO: 8 (the CHST10 gene) is less likely to have a favorable response to an administered DPP-IV inhibitor or a DPP-IV inhibitor in combination with other oral antidiabetic therapy relative to an individual harboring the reference guanosine allele at position 35,799 of SEQ ID NO: 5. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant cytidine allele at position 35,799 of SEQ ID NO: 8 of the CHST10 gene.

As disclosed herein, an individual harboring the variant guanosine allele at position 38,709 of SEQ ID NO: 9 (the CHST10 gene) is less likely to have a favorable response to an administered DPP-IV inhibitor or a DPP-IV inhibitor in combination with other oral antidiabetic therapy relative to an individual harboring the reference adenosine allele at position 38,709 of SEQ ID NO: 5. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant guanosine allele at position 38,709 of SEQ ID NO: 9 of the CHST10 gene.

As disclosed herein, an individual harboring the variant adenosine allele at position 38,947 of SEQ ID NO: 10 (the CHST10 gene) is less likely to have a favorable response to an administered DPP-IV inhibitor or a DPP-IV inhibitor in combination with other oral antidiabetic therapy relative to an individual harboring the reference cytidine allele at position 38,947 of SEQ ID NO: 5. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant adenosine allele at position 38,947 of SEQ ID NO: 10 of the CHST10 gene.

As disclosed herein, an individual harboring the variant cytidine allele at position 41,180 of SEQ ID NO: 11 (the CHST10 gene) is less likely to have a favorable response to an administered DPP-IV inhibitor or a DPP-IV inhibitor in combination with other oral antidiabetic therapy relative to an individual harboring the reference thymidine allele at position 41,180 of SEQ ID NO: 5. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant cytidine allele at position 41,180 of SEQ ID NO: 11 of the CHST10 gene.

As disclosed herein, an individual harboring the variant allele(s) at any one or more of nucleotide positions 24,707, 34,078, 35,799, 38,709, 38,947 and 41,180 of SEQ ID NO: 12 is less likely to have a favorable response to an administered DPP-IV inhibitor or a DPP-IV inhibitor in combination with other oral antidiabetic therapy relative to an individual harboring the reference allele at any one or more positions 24,707, 34,078, 35,799, 38,709, 38,947, and 41,180 of SEQ ID NO: 5. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant allele(s) at one or more of nucleotide positions 24,707, 34,078, 35,799, 38,709, 38,947 and 41,180 of SEQ ID NO: 12 of the CHST10 gene.

As disclosed herein, an individual harboring the variant cytidine allele at position 26,472 of SEQ ID NO: 64 (the TEKT5 gene) is less likely to have a favorable response to an administered DPP-IV inhibitor or a DPP-IV inhibitor in combination with other oral antidiabetic therapy relative to an individual harboring the reference thymidine allele at position 26,472 of SEQ ID NO: 63. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant cytidine allele at position 26,472 of SEQ ID NO: 64 of the TEKT5 gene.

As disclosed herein, an individual harboring the variant allele(s) at any one or more of nucleotide positions 87,712, 87,992, 89,441, 89,662, 89,853, 93,598 and 93,955 of SEQ ID NO: 2 (the GRINL1A gene) is less likely to have a favorable response to an administered DPP-IV inhibitor or a DPP-IV inhibitor in combination with other oral antidiabetic therapy relative to an individual harboring the reference allele at any one or more positions 87,712, 87,992, 89,441, 89,662, 89,853, 93,598 and 93,955 of SEQ ID NO: 1. Thus, in certain embodiments of the invention, methods for determining whether an individual is less likely to have a favorable response to the administration of a therapeutically-effective amount of a DPP-IV inhibitor or a DPP-IV inhibitor in combination with other oral antidiabetic therapy comprise the step of determining whether the individual harbors the variant allele(s) at one or more of nucleotide positions 87,712, 87,992, 89,441, 89,662, 89,853, 93,598 and 93,955 of SEQ ID NO: 77 of the GRINL1A gene.

The methods also can be used to determine whether the individual may require a higher level of administered DPP-IV inhibitor or a DPP-IV inhibitor in combination with other oral antidiabetic therapy comprising the step of determining whether the individual harbors either the reference allele or variant allele at one or more polymorphic loci of SEQ ID NO: 1, SEQ ID NO: 3, SEQ ID NO: 5, and SEQ ID NO: 63, wherein an individual harboring the variant allele of a TEKT5, GRINL1A, HSD11B1 or CHST10 gene, including, for example, the variant allele ("C") found at position 26,472 of SEQ ID NO: 64 (the TEKT5 gene), the variant allele ("C") found at position 87,992 of SEQ ID NO: 2 (the GRINL1A gene), the variant allele ("G") found at position 87,712 of SEQ ID NO: 73 (the GRINL1A gene), the variant allele ("G") found at position 89,441 of SEQ ID NO: 69 (the GRINL1A gene), the variant allele ("A") found at position 89,662 of SEQ ID NO: 70 (the GRINL1A gene), the variant allele ("A") found at position 89,853 of SEQ ID NO: 71 (the GRINL1A gene), the variant allele ("A") found at position 93,598 of SEQ ID NO: 74 (the GRINL1A gene), the variant allele ("C") found at position 94,074 of SEQ ID NO: 72 (the GRINL1A gene), the variant allele ("T") found at position 34,403 of SEQ ID NO: 4 (the HSD11B1 gene), the variant allele ("A") found at position 24,707 of SEQ ID NO: 6 (the CHST10 gene), the variant allele ("T") found at position 34,078 of SEQ ID NO: 7 (the CHST10 gene), the variant allele ("C") found at position 35,799 of SEQ ID NO: 8 (the CHST10 gene), the variant allele ("G") found at position 38,709 of SEQ ID NO: 9 (the CHST10 gene), the variant allele ("A") found at position 38,947 of SEQ ID NO: 10 (the CHST10 gene) and/or the variant allele ("C") found at position 41,180 of SEQ ID NO: 11 (the CHST10 gene), is more likely to require a higher level of administered DPP-IV inhibitor relative to an individual harboring the reference allele of a TEKT5, GRINL1A, HSD11B1 or CHST10 gene including, for example, the reference allele ("T") found at position 26,472 of SEQ ID NO: 63, the reference allele ("T") found at position 87,992 of SEQ ID NO: 1, the reference allele ("G") found at position 87,712 of SEQ ID NO: 1, the reference allele ("G") found at position 89,441 of SEQ ID NO: 1, the reference allele ("A") found at position 89,662 of SEQ ID NO: 1, the reference allele ("A") found at position 89,853 of SEQ ID NO: 1, the reference allele ("A") found at position 93,598 of SEQ ID NO: 1, the reference allele ("C") found at position 94,074 of SEQ ID NO: 1, the reference allele ("T") found at position 87,992 of SEQ ID NO: 1, the reference allele ("C") found at position 34,403 of SEQ ID NO: 3, the reference allele ("G") found at position 24,707 of SEQ ID NO: 5, the reference allele ("C") found at position 34,078 of SEQ ID NO: 5, the reference allele ("G") found at position 35,799 of SEQ ID NO: 5, the reference allele ("A") found at position 38,709 of SEQ ID NO: 5, the reference allele ("C") found at position 38,947 of SEQ ID NO: 5, and/or the reference allele ("T") found at position 41,180 of SEQ ID NO: 5.

The invention further provides nucleic acid molecules comprising at least one single nucleotide polymorphism (SNP) within SEQ ID NO: 1, SEQ ID NO: 3, SEQ ID NO: 5, and/or SEQ ID NO: 63 at a particular polymorphic locus. In certain embodiments, the invention provides the reference allele of a human TEKT5, GRINL1A, HSD11B1, or CHST10 gene. In certain embodiments, the invention provides a human TEKT5, GRINL1A, HSD11B1, or CHST10 polynucleotide comprising at least one single nucleotide polymorphism having a reference allele, which reference allele differs from a variant allele by one nucleotide at nucleotide position 26,472 of SEQ ID NO: 63 for the TEKT5 gene, at nucleotide positions 87,693, 87,992, 89,441, 89,662, 89,853, 93,598, and/or 94,074 of SEQ ID NO: 1 for the GRINL1A gene, at nucleotide position 34,403 of SEQ ID NO: 3 for the HSD11B1 gene, and/or at nucleotide positions 24,707, 34,078, 35,799, 38,709, 38,947, and/or 41,180 of SEQ ID NO: 5 for the CHST10 gene. The invention further relates to the complementary sequences of each of the sequences in SEQ ID NOs: 1, 3, 5, and 63. In certain embodiments, the invention provides a TEKT5 polynucleotide comprising SEQ ID NO: 63. SEQ ID NO: 63 contains a single nucleotide polymorphism having a reference allele, wherein the reference allele comprises a thymidine nucleotide at position 26,472. In further embodiments, the invention provides a GRINL1A polynucleotide comprising SEQ ID NO: 1. SEQ ID NO: 1 contains a single nucleotide polymorphism having one or more references allele(s), wherein the reference allele(s) comprises an adenosine nucleotide at position 87,712, a thymidine nucleotide at position 87,992, an adenosine nucleotide at position 89,441, a guanosine nucleotide at position 89,662, a guanosine nucleotide at position 89,853, a guanosine nucleotide at position 93,598, and a guanosine at position 94,074. In further embodiments, the invention provides an HSD11B1 polynucleotide comprising SEQ ID NO: 3. SEQ ID NO: 3 contains a single nucleotide polymorphism having a reference allele, wherein the reference allele comprises a cytidine nucleotide at position 34,403. In further embodiments, the invention provides a CHST10 polynucleotide comprising SEQ ID NO: 5. SEQ ID NO: 5 contains a single nucleotide polymorphism having one or more reference allele(s), wherein the reference allele(s) comprises a guanosine nucleotide at position 24,707, a cytidine nucleotide at position 34,078, a guanosine nucleotide at position 35,799, an adenosine nucleotide at position 38,709, a cytidine nucleotide at position 38,947, and a thymidine nucleotide at position 41,180.

In other embodiments, the invention provides the variant allele of a human TEKT5, GRINL1A, HSD11B1 or CHST10 gene. Also, in other embodiments, the invention provides a human TEKT5, GRINL1A, HSD11B1 or CHST10 polynucleotide comprising at least one single nucleotide polymorphism having a variant allele, which variant allele differs from a reference allele by one nucleotide at nucleotide position 26,472 of SEQ ID NO: 64 for the TEKT5 gene, at nucleotide position 87,712 of SEQ ID NO: 73, nucleotide position 87,992 of SEQ ID NO: 2, nucleotide position 89,441 of SEQ ID NO: 69, nucleotide position 89,662 of SEQ ID NO: 70; nucleotide position 89,853 of SEQ ID NO: 71; nucleotide position 93,598 of SEQ ID NO: 74; and/or nucleotide position 93,994 of SEQ ID NO: 72 for the GRINL1A gene, at nucleotide position 34,403 of SEQ ID NO: 4 for the HSD11B1 gene, and/or at nucleotide position 24,707 of SEQ ID NO:

6, nucleotide position 34,078 of SEQ ID NO: 7, nucleotide position 35,799 of SEQ ID NO: 8, nucleotide position 38,709 of SEQ ID NO: 9, nucleotide position 38,947 at SEQ ID NO: 10, and/or nucleotide position 41,180 of SEQ ID NO: 11 for the CHST10 gene. The invention further relates to the complementary sequences of each of the sequences in SEQ ID NOs: 2, 4, 6, 7, 8, 9, 10, 11, 64, and 70. In certain embodiments, the invention provides a TEKT5 polynucleotide comprising SEQ ID NO: 64. SEQ ID NO: 64 contains a single nucleotide polymorphism having a variant allele, wherein the variant allele is a cytidine nucleotide at position 26,472. In further embodiments, the invention provides a GRINL1A polynucleotide comprising any of SEQ ID NOs: 70-77. SEQ ID NO: 2 contains a single nucleotide polymorphism having a variant allele, wherein the variant allele is a cytidine nucleotide at position 87,992; SEQ ID NO: 69 contains a single nucleotide polymorphism having a variant allele, wherein the variant allele is a guanosine nucleotide at position 89,441; SEQ ID NO: 70 contains a single nucleotide polymorphism having a variant allele, wherein the variant allele is an adenosine nucleotide at position 89,662; SEQ ID NO: 71 contains a single nucleotide polymorphism having a variant allele, wherein the variant allele is an adenosine nucleotide at position 89,853; SEQ ID NO: 72 contains a single nucleotide polymorphism having a variant allele, wherein the variant allele is a cytidine nucleotide at position 94,074; SEQ ID NO: 73 contains a single nucleotide polymorphism having a variant allele, wherein the variant allele is a guanosine nucleotide at position 87,712; SEQ ID NO: 74 contains a single nucleotide polymorphism having a variant allele, wherein the variant allele is an adenosine nucleotide at position 93,598; SEQ ID NO: 77 contains a single nucleotide polymorphism at one or more sites, including variant alleles, wherein the variant allele is present at position(s) 87,712, 87,992, 89,441, 89,662, 89,853, 93,598, and/or 94,074. In further embodiments, the invention provides an HSD11B1 polynucleotide comprising SEQ ID NO: 4. SEQ ID NO: 4 contains a single nucleotide polymorphism having a variant allele, wherein the variant allele is a thymidine nucleotide at position 34,403. In further embodiments, the invention provides a CHST10 polynucleotide comprising any of SEQ ID NOs: 6 - 12. SEQ ID NO: 6 contains a single nucleotide polymorphism having a variant allele, wherein the variant allele is an adenosine nucleotide at position 24,707; SEQ ID NO: 7 contains a single nucleotide polymorphism having a variant allele, wherein the variant allele is a thymidine at position 34,078; SEQ ID NO: 8 contains a single nucleotide polymorphism having a variant allele, wherein the variant allele is a cytidine at position 35,799; SEQ ID NO: 9 contains a single nucleotide polymorphism having a variant allele, wherein the variant allele is a guanosine at position 38,709; SEQ ID NO: 10 contains a single nucleotide polymorphism having a variant allele, wherein the variant allele is an adenosine at position 38,947; SEQ ID NO: 11 contains a single nucleotide polymorphism having a variant allele, wherein the variant allele is an cytidine at position 41,180, SEQ ID NO: 12 contains a single nucleotide polymorphism at one or more sites, including variant alleles, wherein the variant allele is present at position(s) 24,707, 34,078, 35,799, 38,709, 38,947 and/or 41,180.

The invention further provides variant and reference allele-specific oligonucleotides that hybridize to a nucleic acid sequence comprising at least one polymorphic locus, in addition to the complement of said nucleic acid sequence. These oligonucleotides can be probes or primers, for example, oligonucleotide primers used to amplify a nucleic acid sequence across a polymorphic locus and oligonucleotide primers used to sequence the amplified nucleic acid sequences. Thus, the invention further provides oligonucleotides that can be used to amplify a portion of a polynucleotide of the invention, or fragment thereof, comprising either a variant or reference allele(s) at one or more polymorphic loci. The invention also provides oligonucleotides that can be used to sequence said amplified sequence(s). Methods for using such oligonucleotides to amplify and sequence target nucleic acid sequences are described herein and also well-known in the art.

In certain embodiments, the invention provides oligonucleotides between 15 and 40 nucleotides in length that hybridize to a nucleic acid sequence that spans a polymorphic locus (i.e., SNP-containing fragment) found in a human TEKT5, GRINL1A, HSD11B1, or CHST10 gene. In particular embodiments, the invention provides oligonucleotides that hybridize to a nucleic acid sequence that spans a polymorphic locus found in SEQ ID NOs: 1, 3, 5, 63, or 69, including, for example, a nucleic acid sequence that spans the polymorphic locus containing a reference allele at nucleotide position 26,472 of SEQ ID NO: 63, a nucleic acid sequence that spans the polymorphic locus containing a reference allele at nucleotide position 87,712 of SEQ ID NO: 1, a nucleic acid sequence that spans the polymorphic locus containing a reference allele at nucleotide position 87,992 of SEQ ID NO: 1, a nucleic acid sequence that spans the polymorphic locus containing a reference allele at nucleotide position 89,441 of SEQ ID NO: 1, a nucleic acid sequence that spans the polymorphic locus containing a reference allele at nucleotide position 89,662 of SEQ ID NO: 1, a nucleic acid sequence that spans the polymorphic locus containing a reference allele at nucleotide position 89,853 of SEQ ID NO: 1, a nucleic acid sequence that spans the polymorphic locus containing a reference allele at nucleotide position 93,598 of SEQ ID NO: 1, a nucleic acid sequence that spans the polymorphic locus containing a reference allele at nucleotide position 94,074 of SEQ ID NO: 1, a nucleic acid sequence that spans the polymorphic locus containing a reference allele at nucleotide position 34,403 of SEQ ID NO: 3, a nucleic acid sequence that spans the polymorphic locus containing a reference allele at nucleotide position 24,707 of SEQ ID NO: 5, a nucleic acid sequence that spans the polymorphic locus containing a reference allele at nucleotide position 34,078 of SEQ ID NO: 5, a nucleic acid sequence that spans the polymorphic locus containing a reference allele at nucleotide position 35,799 of SEQ ID NO: 5, a nucleic acid sequence that spans the polymorphic locus containing a reference allele at nucleotide position 38,709 of SEQ ID NO:

5, a nucleic acid sequence that spans the polymorphic locus containing a reference allele at nucleotide position 38,947 of SEQ ID NO: 5, and a nucleic acid sequence that spans the polymorphic locus containing a reference allele at nucleotide position 41,180 of SEQ ID NO: 5.

In certain other embodiments, the invention provides oligonucleotides between 12 and 25 nucleotides in length that hybridize to a nucleic acid sequence that spans a polymorphic locus found in SEQ ID NOs: 2, 4, 6, 7, 8, 9, 10, 11, 12, 64, 69, 70, 71, 72, 73, 74, or 75, including, for example, a nucleic acid sequence that spans the polymorphic locus containing a variant allele at nucleotide position 26,472 of SEQ ID NO: 64, a nucleic acid sequence that spans the polymorphic locus containing a variant allele at nucleotide position 87,992 of SEQ ID NO: 2 or 75, a nucleic acid sequence that spans the polymorphic locus containing a variant allele at nucleotide position 89,441 of SEQ ID NO: 69 or 75, a nucleic acid sequence that spans the polymorphic locus containing a variant allele at nucleotide position 89,662 of SEQ ID NO: 70 or 75, a nucleic acid sequence that spans the polymorphic locus containing a variant allele at nucleotide position 89,853 of SEQ ID NO: 71 or 75, a nucleic acid sequence that spans the polymorphic locus containing a variant allele at nucleotide position 94,074 of SEQ ID NO: 72 or 75, a nucleic acid sequence that spans the polymorphic locus containing a variant allele at nucleotide position 87,712 of SEQ ID NO: 73 or 75, a nucleic acid sequence that spans the polymorphic locus containing a variant allele at nucleotide position 93,598 of SEQ ID NO: 74 or 75, a nucleic acid sequence that spans the polymorphic locus containing a variant allele at nucleotide position 34,403 of SEQ ID NO: 4, a nucleic acid sequence that spans the polymorphic locus containing a variant allele at nucleotide position 24,707 of SEQ ID NO: 6 or 12, a nucleic acid sequence that spans the polymorphic locus containing a variant allele at nucleotide position 34,078 of SEQ ID NO: 7 or 12, a nucleic acid sequence that spans the polymorphic locus containing a variant allele at nucleotide position 35,799 of SEQ ID NO: 8 or 12, a nucleic acid sequence that spans the polymorphic locus containing a variant allele at nucleotide position 38,709 of SEQ ID NO: 9 or 12, a nucleic acid sequence that spans the polymorphic locus containing a variant allele at nucleotide position 38,947 of SEQ ID NO: 10 or 12, and a nucleic acid sequence that spans the polymorphic locus containing a variant allele at nucleotide position 41,180 of SEQ ID NO: 11 or 12. In other particular embodiments, the oligonucleotides comprise any of SEQ ID NOs: 13 - 44, 66-68, and 76-99. The oligonucleotides can be used to amplify the above-described nucleic acid sequences spanning a polymorphic locus.

The invention further provides oligonucleotide primers for sequencing any of the amplified nucleic acid sequences described herein. In particular embodiments, the oligonucleotides for sequencing the amplified nucleic acid sequences comprise any of SEQ ID NOs: 13-14, 21-22, 25-26, 29-30, 33-34, 37-38, 41-42, 65-66, 76-77, 80-81, 84-85, 88-89, 92-93, and 96-97.

The probe sequences for each polymorphism disclosed herein were selected to provide optimal melting temperature criteria. Occasionally, a probe directed to the antisense strand was selected because the melting temperature of the antisense probe was more beneficial for the assay conditions used. Nonetheless, the present invention contemplates probe sequences directed to the opposite strand of each allele for each polymorphism. Aside from the probe sequences disclosed herein, one skilled in the art would be able to design appropriate sense and antisense probe sequences to detect which allele is present at the polymorphic locus for each polymorphism of the present invention.

Specifically, for the rs2541508 (TEKT5 gene) SNP, the alleles at the polymorphic locus were determined to be T/C (reference/variant). Probe sequences directed to the opposing strand at this polymorphic locus would contain A/G (reference/variant) accordingly.

Specifically, for the rs11853270 (GRINL1A gene) SNP, the alleles at the polymorphic locus were determined to be T/C (reference/variant). Probe sequences directed to the opposing strand at this polymorphic locus would contain A/G (reference/variant) accordingly.

Specifically, for the rs4774281 (GRINL1A gene) SNP, the alleles at the polymorphic locus were determined to be A/G (reference/variant). Probe sequences directed to the opposing strand at this polymorphic locus would contain T/C (reference/variant) accordingly.

Specifically, for the rs8037000 (GRINL1A gene) SNP, the alleles at the polymorphic locus were determined to be G/A (reference/variant). Probe sequences directed to the opposing strand at this polymorphic locus would contain C/T (reference/variant) accordingly.

Specifically, for the rs6493943 (GRINL1A gene) SNP, the alleles at the polymorphic locus were determined to be G/A (reference/variant). Probe sequences directed to the opposing strand at this polymorphic locus would contain C/T (reference/variant) accordingly.

Specifically, for the rs4774282 (GRINL1A gene) SNP, the alleles at the polymorphic locus were determined to be G/C (reference/variant). Probe sequences directed to the opposing strand at this polymorphic locus would contain C/G (reference/variant) accordingly.

Specifically, for the SNP2 (GRINL1A gene) SNP, the alleles at the polymorphic locus were determined to be A/G (reference/variant). Probe sequences directed to the opposing strand at this polymorphic locus would contain T/C (reference/variant) accordingly.

Specifically, for the SNP12 (GRINL1A gene) SNP, the alleles at the polymorphic locus were determined to be G/A (reference/variant). Probe sequences directed to the opposing strand at this polymorphic locus would contain C/T

(reference/variant) accordingly.

Specifically, for the rs17015076 (HSD11B1 gene) SNP, the alleles at the polymorphic locus were determined to be C/T (reference/variant). Probe sequences directed to the opposing strand at this polymorphic locus would contain G/A (reference/variant) accordingly.

Specifically, for the rs1370627 (CHST10 gene) SNP, the alleles at the polymorphic locus were determined to be G/A (reference/variant). Probe sequences directed to the opposing strand at this polymorphic locus would contain C/T (reference/variant) accordingly.

Specifically, for the rs6716367 (CHST10 gene) SNP, the alleles at the polymorphic locus were determined to be C/T (reference/variant). Probe sequences directed to the opposing strand at this polymorphic locus would contain G/A (reference/variant) accordingly. Alleles in Figure 4 are given relative to the opposing strand.

Specifically, for the rs 7585898 (CHST10 gene) SNP, the alleles at the polymorphic locus were determined to be G/C (reference/variant). Probe sequences directed to the opposing strand at this polymorphic locus would contain C/G (reference/variant) accordingly. Alleles in Figure 5 are given relative to the opposing strand.

Specifically, for the rs17024136 (CHST10 gene) SNP, the alleles at the polymorphic locus were determined to be A/G (reference/variant). Probe sequences directed to the opposing strand at this polymorphic locus would contain T/C (reference/variant) accordingly. Alleles in Figure 6 are given relative to the opposing strand.

Specifically, for the rs17024129 (CHST10 gene) SNP, the alleles at the polymorphic locus were determined to be C/A (reference/variant). Probe sequences directed to the opposing strand at this polymorphic locus would contain G/T (reference/variant) accordingly. Alleles in Figure 7 are given relative to the opposing strand.

Specifically, for the rs7574684 (CHST10 gene) SNP, the alleles at the polymorphic locus were determined to be T/C (reference/variant). Probe sequences directed to the opposing strand at this polymorphic locus would contain A/G (reference/variant) accordingly. Alleles in Figure 8 are given relative to the opposing strand.

The invention further provides methods for analyzing nucleic acid from a biological sample obtained from a subject to determine whether the nucleic acid contains a reference or variant nucleotide (allele) at one or more polymorphic loci of a gene or genes, said method comprising the steps of (1) isolating nucleic acid (i.e., DNA or RNA) from a biological sample obtained from a subject, (2) amplifying a nucleotide sequence comprising a polymorphic locus of a gene from the isolated nucleic acid of the biological sample using allele specific oligonucleotides (ASO) and/or non-allele specific oligonucleotides (non-ASO), (3) sequencing the amplified nucleotide sequence comprising the polymorphic locus using allele specific oligonucleotides (ASO) and/or non-allele specific oligonucleotides (non-ASO) and (4) determining whether the sequence of the amplified nucleotide sequence contains a reference or a variant nucleotide (allele) at the polymorphic locus of the gene. In certain embodiments, the described methods are used to determine whether nucleic acid from a biological sample of a subject contains a reference or a variant nucleotide (allele) at a polymorphic locus of a TEKT5, GRINL1A, HSD11B1 or CHST10 gene, including, for example, a polymorphic locus of any of SEQ ID NOs: 1-12, 63-64, and 69-75. In certain embodiments, the methods are used to determine whether nucleic acid from a biological sample of a subject contains a reference ("T") allele or a variant ("C") allele at position 26,472 of SEQ ID NO: 63 or 64, respectively. In further embodiments, the methods are used to determine whether nucleic acid from a biological sample of a subject contains a reference ("T") allele or a variant ("C") allele at position 87,992 of SEQ ID NO: 1 or 2, a reference ("A") allele or a variant ("G") allele at position 89,441 of SEQ ID NO: 1 or 69, a reference ("G") allele or a variant ("A") allele at position 89,662 of SEQ ID NO: 1 or 70, a reference ("G") allele or a variant ("A") allele at position 89,853 of SEQ ID NO: 1 or 71, a reference ("G") allele or a variant ("C") allele at position 94,074 of SEQ ID NO: 1 or 72, a reference ("A") allele or a variant ("G") allele at position 87,712 of SEQ ID NO: 1 or 73, a reference ("G") allele or a variant ("A") allele at position 93,598 of SEQ ID NO: 1 or 74, and/or a combination thereof of the alleles described herein or otherwise known. In further embodiments, the methods are used to determine whether nucleic acid from a biological sample of a subject contains a reference ("C") allele or a variant ("T") allele at position 34,403 of SEQ ID NO: 3 or 4, respectively. In further embodiments, the methods are used to determine whether nucleic acid from a biological sample of a subject contains a reference ("G") allele or a variant ("A") allele at position 24,707 of SEQ ID NO: 5 or 6. In other embodiments, the methods are used to determine whether nucleic acid from a biological sample of a subject contains a reference allele ("C") or variant allele ("T") at position 34,078 of SEQ ID NO: 5 or 7, a reference allele ("G") or variant allele ("C") at position 35,799 of SEQ ID NO: 5 or 8, a reference allele ("A") or variant allele ("G") at position 38,709 of SEQ ID NO: 5 or 9, a reference allele ("C") or a variant allele ("A") found at position 38,947 of SEQ ID NO: 5 or 10, and/or a reference allele ("T") or a variant allele ("C") found at position 41,180 of SEQ ID NO: 5 or 11, and/or a combination thereof of the alleles described herein or otherwise known. In any of the above methods, one or more of the amplification primers described herein can be used, including, for example, in particular embodiments, oligonucleotides comprising any of SEQ ID NOs: 13 - 44, 65-68, and 76-99. In any of the above methods, one or more of the sequencing primers described herein can be used, including for example in particular embodiments, oligonucleotides comprising any of SEQ ID NOs: 21-22, 25-26, 29-30, 33-34, 37-38, 41-42, 65-66, 76-77, 80-81, 84-85, 88-89, 92-93, and 96-97.

The above-described methods and oligonucleotides can be used to identify a patient(s) or a patient population(s) that is more likely to have a favorable response to the administration of a therapeutically-effective amount of a DPP-IV

inhibitor or a DPP-IV inhibitor in combination with other oral antidiabetic therapy. In certain embodiments, wherein the described method(s) is used to determine whether nucleic acid from a biological sample(s) of a subject(s), patient(s), or patient population(s) contains a reference or variant nucleotide (allele) at one or more polymorphic loci of a TEKT5, GRINL1A, HSD11B1 or CHST10 gene (including, for example, a polymorphic locus found in SEQ ID NOs: 1-12, 63-64, and 69-75), the determination that the nucleic acid contains a reference allele identifies that subject(s), patient(s), or patient population(s) that is more likely to have a favorable response to the administration of a therapeutically-effective amount of a DPP-IV inhibitor or a DPP-IV inhibitor in combination with other oral antidiabetic therapy. In certain other embodiments, wherein the described method(s) is used to determine whether nucleic acid from a biological sample(s) of a subject(s), patient(s), or patient population(s) contains a reference or variant nucleotide (allele) at one or more polymorphic loci of a TEKT5, GRINL1A, HSD11B1 or CHST10 gene (including, for example, a polymorphic locus of SEQ ID NOs: 1-12, 63-64, and 69-75), the determination that the nucleic acid contains a variant allele identifies that subject(s), patient(s), or patient population(s) that is less likely to have a favorable response to the administration of a therapeutically-effective amount of a DPP-IV inhibitor or a DPP-IV inhibitor in combination with other oral antidiabetic therapy.

The oligonucleotides and methods described herein can also be used to genotype a patient sample(s) to assess whether said sample(s) contains a reference or variant nucleotide (allele) at one or more polymorphic loci and/or the frequency of expression of a reference or variant nucleotide (allele) at one or more polymorphic loci, including a polymorphic locus of a TEKT5, GRINL1A, HSD11B1 or CHST10 gene (for example, a polymorphic locus found in SEQ ID NOs: 1-12, 63-64, and 69-75). In certain embodiments, the method comprises the steps of (1) isolating nucleic acid (i.e., DNA or RNA) from a biological sample obtained from a subject or patient, (2) amplifying a nucleotide sequence comprising a polymorphic locus of a gene from the isolated nucleic acid of the biological sample, and (3) subjecting the amplified nucleotide sequence to an analysis assay, such as a genetic bit analysis (GBA) reaction so as to determine the genotype. In certain embodiments, a nucleotide sequence comprising a polymorphic locus of a TEKT5, GRINL1A, HSD11B1 or CHST10 gene, including, for example, the polymorphic locus found at position 26,472 of SEQ ID NOs: 63 and 64, the polymorphic locus found at position 87,992 of SEQ ID NOs: 1 and 2, the polymorphic locus found at position 89,441 of SEQ ID NOs: 1 and 69, the polymorphic locus found at position 89,662 of SEQ ID NOs: 1 and 70, the polymorphic locus found at position 89,853 of SEQ ID NOs: 1 and 71, the polymorphic locus found at position 94,074 of SEQ ID NOs: 1 and 72, the polymorphic locus found at position 87,712 of SEQ ID NOs: 1 and 73, the polymorphic locus found at position 93,598 of SEQ ID NOs: 1 and 74, the polymorphic locus found at position 34,403 of SEQ ID NOs: 3 and 4, the polymorphic locus found at position 24,707 of SEQ ID NOs: 5 and 6, the polymorphic locus found at position 34,0787 of SEQ ID NOs: 5 and 7, the polymorphic locus found at position 35,799 of SEQ ID NOs: 5 and 8, the polymorphic locus found at position 38,709 of SEQ ID NOs: 5 and 9, the polymorphic locus found at position 38,947 of SEQ ID NOs: 5 and 10, and the polymorphic locus found at position 41,180 of SEQ ID NOs: 5 and 11 is amplified and analyzed.

The methods and oligonucleotides described herein can be used to determine the association between either a reference or variant allele at one or more polymorphic loci and the likelihood of a favorable response to the administration of a therapeutically-effective amount of a DPP-IV inhibitor or a DPP-IV inhibitor in combination with other oral antidiabetic therapy. In certain embodiments, the method comprises the steps of (1) administering a therapeutically effective amount of a DPP-IV inhibitor or a DPP-IV inhibitor in combination with other oral antidiabetic therapy to one or more subject(s), patient(s), or patient population(s) and measuring the individual response(s) to the DPP-IV inhibitor or a DPP-IV inhibitor in combination with other oral antidiabetic therapy to determine whether the response(s) is a favorable response(s) or a non-response(s), (2) obtaining a biological sample from the subject(s), patient(s), or patient population(s) treated with the DPP-IV inhibitor or a DPP-IV inhibitor in combination with other oral antidiabetic therapy, (3) isolating nucleic acid (i.e., DNA or RNA) from the obtained biological sample(s), (4) amplifying a nucleotide sequence comprising a polymorphic locus of a gene from the isolated nucleic acid, (5) determining whether said amplified nucleotide sequence contains a reference or variant nucleotide (allele), and (6) determining the association between either the reference or variant allele at the polymorphic locus and a favorable response to the administration of a therapeutically-effective amount of a DPP-IV inhibitor or a DPP-IV inhibitor in combination with other oral antidiabetic therapy.

This method can be used to associate a favorable response to a DPP-IV inhibitor or a DPP-IV inhibitor in combination with other oral antidiabetic therapy to the presence of a reference or variant allele at any polymorphic locus of any gene. In certain embodiments, the polymorphic locus is located in a TEKT5, GRINL1A, HSD11B1 or CHST10 gene (for example, a polymorphic locus found in SEQ ID NOs: 1-12, 63-64, and 69-75). In particular embodiments, the polymorphic locus is located in a TEKT5, GRINL1A, HSD11B1, or CHST10 gene at the nucleotide positions described herein including, for example, the polymorphic locus found at position 26,472 of SEQ ID NOs: 63 and 64, the polymorphic locus found at position 87,992 of SEQ ID NOs: 1 and 2, the polymorphic locus found at position 89,441 of SEQ ID NOs: 1 and 69, the polymorphic locus found at position 89,662 of SEQ ID NOs: 1 and 70, the polymorphic locus found at position 89,853 of SEQ ID NOs: 1 and 71, the polymorphic locus found at position 94,074 of SEQ ID NOs: 1 and 72, the polymorphic locus found at position 87,712 of SEQ ID NOs: 1 and 73, the polymorphic locus found at position 93,598 of SEQ ID NOs: 1 and 74, the polymorphic locus found at position 34,403 of SEQ ID NOs: 3 and 4, the polymorphic

locus found at position 24,707 of SEQ ID NOs: 5 and 6, the polymorphic locus found at position 34,0787 of SEQ ID NOs: 5 and 7, the polymorphic locus found at position 35,799 of SEQ ID NOs: 5 and 8, the polymorphic locus found at position 38,709 of SEQ ID NOs: 5 and 9, the polymorphic locus found at position 38,947 of SEQ ID NOs: 5 and 10, and the polymorphic locus found at position 41,180 of SEQ ID NOs: 5 and 11. The response to the administration of a DPP-IV inhibitor or a DPP-IV inhibitor in combination with other oral antidiabetic therapy can be measured using a variety of assays known in the art, including, for example, by measuring the level of glycosylated hemoglobin (HbA1c) levels in blood, measuring the fasting glucose levels, and measuring the levels of AUC glucose. Methods for isolating nucleic acid from a biological sample are well- known in the art. Methods and oligonucleotides for amplifying a nucleotide sequence comprising a polymorphic locus of a gene are described herein and also known in the art. In certain embodiments, the amplification oligonucleotides can comprise, for example, SEQ ID NOs: 13-44, 65-68, and 76-99. Whether an amplified nucleotide sequence contains a reference or variant nucleotide (allele) can be determined by subjecting the amplified nucleotide sequence to an analysis assay, for example, a genetic bit analysis (GBA) reaction. In certain embodiments where DPP-IV inhibitor is administered to one or more patient populations, the method further comprises subjecting the association between the reference or variant allele at the polymorphic locus and a favorable response to the administration of a DPP-IV inhibitor or a DPP-IV inhibitor in combination with other oral antidiabetic therapy to well-known statistical analyses. In further embodiments where DPP-IV inhibitor is administered to one or more patient populations, the method can be used to identify specific ethnic population(s).

In additional embodiments, the amplified nucleotide sequences produced by the methods of the invention are used to genotype patient samples, such as by genetic bit analysis (GBA). Additional applications for nucleic acid sequences comprising a polymorphic locus as provided by the inventive methods include identifying individual(s) more likely to have a favorable response following administration of a pharmaceutically-acceptable amount of a DPP-IV inhibitor or a DPP-IV inhibitor in combination with other oral antidiabetic therapy. Further applications include using said amplified nucleic acid sequences comprising a polymorphic locus as provided by the methods of this invention to identify ethnic population(s).

The methods, polynucleotides, and oligonucleotides provided herein can be used to analyze a nucleic acid from one or more individuals to determine whether the reference or variant nucleotide is present at any polymorphic site(s) found in any of the following genes: the TEKT5 gene (e.g., SEQ ID NOs: 63 and 64), the GRINL1A gene (e.g., SEQ ID NOs: 1, 2, and 69-75), the HSD11B1 gene (e.g. SEQ ID NOs: 3 and 4), and the CHST10 gene (e.g. SEQ ID NOs: 5 - 12). A nucleotide or set of nucleotides occupying the polymorphic locus or loci in any gene can be determined. In certain embodiments, a nucleotide or set of nucleotides occupying the polymorphic locus or loci found in the TEKT5 gene, the GRINL1A gene, the HSD11B1 gene, and the CHST10 gene is determined. This type of analysis can be performed on a number of individuals, who are also tested (previously, concurrently or subsequently) for an increased likelihood of a favorable response to administration of a therapeutically-effective amount of a DPP-IV inhibitor or a DPP-IV inhibitor in combination with other oral antidiabetic therapy. The nucleotide or set of nucleotides present at the polymorphic locus or loci in individuals having a favorable response to the administration of a therapeutically effective amount of a DPP-IV inhibitor or a DPP-IV inhibitor in combination with other oral antidiabetic therapy is determined. The increased likelihood of a favorable response to the administration of a pharmaceutically acceptable amount of a DPP-IV inhibitor or a DPP-IV inhibitor in combination with other oral antidiabetic therapy phenotype is then correlated with the nucleotide or set of nucleotides present at the polymorphic locus or loci in the individuals tested. The invention thus further provides methods for identifying individuals more likely to have a favorable response to administration of a therapeutically-effective amount of a DPP-IV inhibitor or a DPP-IV inhibitor in combination with other oral antidiabetic therapy based on the particular genotype found in the polymorphic locus or loci identified in any gene, including for example, the TEKT5, GRINL1A, HSD11B1, or CHST10 gene, particularly including, for example, the polymorphic locus found at nucleotide position 26,472 of SEQ ID NOs: 63 and 64, the polymorphic locus found at nucleotide position 87,992 of SEQ ID NOs: 1 and 2, the polymorphic locus found at nucleotide position 89,441 of SEQ ID NOs: 1 and 69, the polymorphic locus found at nucleotide position 89,662 of SEQ ID NOs: 1 and 70, the polymorphic locus found at nucleotide position 89,853 of SEQ ID NOs: 1 and 71, the polymorphic locus found at nucleotide position 94,074 of SEQ ID NOs: 1 and 72, the polymorphic locus found at nucleotide position 87,712 of SEQ ID NOs: 1 and 73, the polymorphic locus found at nucleotide position 93,598 of SEQ ID NOs: 1 and 74, the polymorphic locus found at nucleotide position 87,992 of SEQ ID NOs: 1 and 2, the polymorphic locus found at nucleotide position 34,403 of SEQ ID NOs: 3 and 4, the polymorphic locus found at nucleotide position 24,707 of SEQ ID NOs: 5 and 6, the polymorphic locus found at nucleotide position 34,078 of SEQ ID NOs: 5 and 7, the polymorphic locus found at nucleotide position 35,799 of SEQ ID NOs: 5 and 8, the polymorphic locus found at nucleotide position 38,709 of SEQ ID NOs: 5 and 9, the polymorphic locus found at nucleotide position 38,947 of SEQ ID NOs: 5 and 10, or the polymorphic locus found at nucleotide position 41,180 of SEQ ID NOs: 5 and 11. In certain embodiments, methods for identifying individuals more likely to have a favorable response to administration of a therapeutically-effective amount of a DPP-IV inhibitor or a DPP-IV inhibitor in combination with other oral antidiabetic therapy are based on determining the particular genotype found in the polymorphic loci found at one or more of the nucleotide positions 24,707, 34,078, 35,799, 38,709, 38,947 and 41,180 of SEQ ID NOs: 5 and 12.

The invention provides complementary oligonucleotides capable of hybridizing to the polynucleotides, and fragments thereof, of the invention. Preferably, such antisense oligonucleotides are capable of discriminating between the reference or variant allele of the polynucleotide, preferably at one or more polymorphic sites of said polynucleotide.

The invention provides siRNA or RNAi oligonucleotides capable of hybridizing to the polynucleotides, and fragments thereof, of the invention. Preferably, such siRNA or RNAi oligonucleotides are capable of discriminating between the reference or variant allele of the polynucleotide, preferably at one or more polymorphic sites of said polynucleotide.

The invention provides methods for analyzing one or more nucleic acid samples comprising the step of determining the nucleic acid sequence of one or more samples at one or more polymorphic loci in the human TEKT5, GRINL1A, HSD11B1 or CHST10 gene, including, for example, the polymorphic locus found at nucleotide position 26,472 of SEQ ID NOs: 63 and 64, the polymorphic locus found at nucleotide position 87,992 of SEQ ID NOs: 1 and 2, the polymorphic locus found at nucleotide position 89,441 of SEQ ID NOs: 1 and 69, the polymorphic locus found at nucleotide position 89,662 of SEQ ID NOs: 1 and 70, the polymorphic locus found at nucleotide position 89,853 of SEQ ID NOs: 1 and 71, the polymorphic locus found at nucleotide position 94,074 of SEQ ID NOs: 1 and 72, the polymorphic locus found at nucleotide position 87,712 of SEQ ID NOs: 1 and 73, the polymorphic locus found at nucleotide position 93,598 of SEQ ID NOs: 1 and 74, the polymorphic locus found at nucleotide position 34,403 of SEQ ID NOs: 3 and 4, the polymorphic locus found at nucleotide position 24,707 of SEQ ID NOs: 5 and 6, the polymorphic locus found at nucleotide position 34,078 of SEQ ID NOs: 5 and 7, the polymorphic locus found at nucleotide position 35,799 of SEQ ID NOs: 5 and 8, the polymorphic locus found at nucleotide position 38,709 of SEQ ID NOs: 5 and 9, the polymorphic locus found at nucleotide position 38,947 of SEQ ID NOs: 5 and 10, or the polymorphic locus found at nucleotide position 41,180 of SEQ ID NOs: 5 and 11, and the polymorphic loci found at one or more of the nucleotide positions 24,707, 34,078, 35,799, 38,709, 38,947 and 41,180 of SEQ ID NOs: 5 and 12 or the polymorphic loci found at one or more of the nucleotide positions 87,712, 87,992, 89,441, 89,662, 89,853, 93,598, and 94,074 of SEQ ID NOs: 1 and 75, wherein the presence of a reference allele at said one or more polymorphic loci is indicative of a increased likelihood of a favorable response to the administration of a therapeutically-effective amount of a DPP-IV inhibitor or a DPP-IV inhibitor in combination with other oral antidiabetic therapy.

The invention provides methods for analyzing one or more nucleic acid samples comprising the step of determining the nucleic acid sequence of one or more samples at one or more polymorphic loci in the human TEKT5, GRINL1A, HSD11B1 or CHST10 gene, including, for example, the polymorphic locus found at nucleotide position 26,472 of SEQ ID NOs: 63 and 64, the polymorphic locus found at nucleotide position 87,992 of SEQ ID NOs: 1 and 2, the polymorphic locus found at nucleotide position 89,441 of SEQ ID NOs: 1 and 69, the polymorphic locus found at nucleotide position 89,662 of SEQ ID NOs: 1 and 70, the polymorphic locus found at nucleotide position 89,853 of SEQ ID NOs: 1 and 71, the polymorphic locus found at nucleotide position 94,074 of SEQ ID NOs: 1 and 72, the polymorphic locus found at nucleotide position 87,712 of SEQ ID NOs: 1 and 73, the polymorphic locus found at nucleotide position 93,598 of SEQ ID NOs: 1 and 74, the polymorphic loci found at one or more nucleotide positions 87,712, 87,992, 89,441, 89,662, 89,853, 93,598, and 94,074 of SEQ ID NOs: 1 and 75, wherein the presence of a reference allele at said one or more polymorphic loci is indicative of a increased likelihood of a favorable response to the administration of a therapeutically-effective amount of a DPP-IV inhibitor or a DPP-IV inhibitor in combination with other oral antidiabetic therapy, the polymorphic locus found at nucleotide position 34,403 of SEQ ID NOs: 3 and 4, and the polymorphic locus found at nucleotide position 24,707 of SEQ ID NOs: 5 and 6, or the polymorphic locus found at nucleotide position 34,078 of SEQ ID NOs: 5 and 7, or the polymorphic locus found at nucleotide position 35,799 of SEQ ID NOs: 5 and 8, or the polymorphic locus found at nucleotide position 38,709 of SEQ ID NOs: 5 and 9, or the polymorphic locus found at nucleotide position 38,947 of SEQ ID NOs: 5 and 10, or the polymorphic locus found at nucleotide position 41,180 of SEQ ID NOs: 5 and 11, the polymorphic loci found at one or more nucleotide positions 24,707, 34,078, 35,799, 38,709, 38,947 and 41,180 of SEQ ID NO: 5 and 12, wherein the presence of the variant allele at said one or more polymorphic loci is indicative of an decreased likelihood of a favorable response to the administration of a therapeutically-effective amount of a DPP-IV inhibitor or a DPP-IV inhibitor in combination with other oral antidiabetic therapy.

The invention further provides methods for constructing haplotypes using the isolated nucleic acids of any of SEQ ID NOs: 1- 12, 63-64, and 69-75, or elsewhere herein, comprising the step of grouping at least two of the isolated nucleic acids. The invention further provides methods for constructing haplotypes further comprising the step of using said haplotypes to identify an individual more likely to have a favorable response to the administration of a therapeutically-effective amount of a DPP-IV inhibitor or a DPP-IV inhibitor in combination with other oral antidiabetic therapy phenotype, and correlating the presence of such a phenotype with said haplotype.

The invention further provides a library of nucleic acids, each of which comprises one or more polymorphic positions within a gene encoding the human TEKT5, GRINL1A, HSD11B1 or CHST10 protein, wherein said polymorphic positions are selected from the polymorphic positions found in SEQ ID NOs: 1- 12, 63-64, and 69-75. In certain embodiments, the polymorphic positions are selected from position 26,472 in SEQ ID NOs: 63 and 64; position 87,992 in SEQ ID NOs: 1 and 2; position 87,712 in SEQ ID NOs: 1 and 73; position 89,441 in SEQ ID NOs: 1 and 69; position 89,662

in SEQ ID NOs: 1 and 70; position 89,853 in SEQ ID NOs: 1 and 71; position 93,598 in SEQ ID NOs: 1 and 74; position 94,074 in SEQ ID NOs: 1 and 72; position 34,403 in SEQ ID NOs: 3 and 4; position 24,707 in SEQ ID NOs: 5 and 6, position 34,078 in SEQ ID NOs: 5 and 7, position 35,799 in SEQ ID NOs: 5 and 8, position 38,709 in SEQ ID NOs: 5 and 9, position 38,947 in SEQ ID NOs: 5 and 10, and position 41,180 in SEQ ID NOs: 5 and 11. In other embodiments, the invention provides a library of nucleic acids comprising the complementary sequences of those sequences comprising one or more polymorphic loci found in a human TEKT5, GRINL1A, HSD11B1 or CHST10 gene, including, for example, the complementary sequences of SEQ ID NOs: 1- 12, 63-64, and 69-77 and, in particular, the complementary sequences of those sequences comprising the polymorphic positions selected from position 26,472 in SEQ ID NOs: 63 and 64; position 87,992 in SEQ ID NOs: 1 and 2, position 89,441 in SEQ ID NOs: 1 and 69, position 89,662 in SEQ ID NOs: 1 and 70, position 89,853 in SEQ ID NOs: 1 and 71, position 94,074 in SEQ ID NOs: 1 and 72, position 87,712 in SEQ ID NOs: 1 and 73, position 93,598 in SEQ ID NOs: 1 and 74; position 34,403 in SEQ ID NOs: 3 and 4; position 24,707 in SEQ ID NOs: 5 and 6, position 34,078 in SEQ ID NOs: 5 and 7, position 35,799 in SEQ ID NOs: 5 and 8, position 38,709 in SEQ ID NOs: 5 and 9, position 38,947 in SEQ ID NOs: 5 and 10, and position 41,180 in SEQ ID NOs: 5 and 11. In other embodiments, the invention provides a library of nucleic acids comprising fragments of those sequences comprising one or more polymorphic loci found in a human TEKT5, GRINL1A, HSD11B1 or CHST10 gene, including, for example, polymorphic positions selected from position 26,472 in SEQ ID NOs: 63 and 64; position 87,992 in SEQ ID NOs: 1 and 2, position 89,441 in SEQ ID NOs: 1 and 69, position 89,662 in SEQ ID NOs: 1 and 70, position 89,853 in SEQ ID NOs: 1 and 71, position 94,074 in SEQ ID NOs: 1 and 72, position 87,712 in SEQ ID NOs: 1 and 73, position 93,598 in SEQ ID NOs: 1 and 74; position 34,403 in SEQ ID NOs: 3 and 4; position 24,707 in SEQ ID NOs: 5 and 6, position 34,078 in SEQ ID NOs: 5 and 7, position 35,799 in SEQ ID NOs: 5 and 8, position 38,709 in SEQ ID NOs: 5 and 9, position 38,947 in SEQ ID NOs: 5 and 10, and position 41,180 in SEQ ID NOs: 5 and 11, as well as the complementary sequences thereof.

The invention further provides kits for identifying a subject or patient more likely to have a favorable response to the administration of a therapeutically-effective amount of a DPP-IV inhibitor or a DPP-IV inhibitor in combination with other oral antidiabetic therapy, wherein said kit comprises oligonucleotides capable of identifying the nucleotide residing at one or more polymorphic loci of the human TEKT5, GRINL1A, HSD11B1 or CHST10 gene, wherein the presence of the reference allele at said one or more polymorphic loci is indicative of an increased likelihood of a favorable response to the administration of a therapeutically-effective amount of a DPP-IV inhibitor or a DPP-IV inhibitor in combination with other oral antidiabetic therapy and/or wherein the presence of the variant allele at said one or more polymorphic loci is indicative of a decreased likelihood of a favorable response to the administration of a therapeutically-effective amount of a DPP-IV inhibitor or a DPP-IV inhibitor in combination with other oral antidiabetic therapy. In certain embodiments, the kit comprises oligonucleotides primers that can amplify a portion of the reference and/or variant sequences comprising at least one polymorphic locus of the human TEKT5, GRINL1A, HSD11B1 or CHST10 gene, for example, oligonucleotide primers that amplify sequence across the polymorphic locus. In further embodiments, the kit additionally comprises oligonucleotides that can be used to sequence said amplified sequence(s).

In particular embodiments, the kit comprises oligonucleotides that hybridize to a nucleic acid sequence that spans a polymorphic locus found in SEQ ID NOs: 1, 3, 5, or 63, including, for example, the polymorphic locus containing a reference allele at nucleotide position 26,472 of SEQ ID NO: 63; the polymorphic locus containing a reference allele at nucleotide positions 87,712, 87,992, 89,441, 89,662, 89,853, 93,598, and 94,074 of SEQ ID NO: 1, the polymorphic locus containing a reference allele at nucleotide position 34,403 of SEQ ID NO: 3, and the polymorphic locus containing a reference allele at nucleotide positions 24,707, 34,078, 35,799, 38,709, 38,947, and 41,180 of SEQ ID NO: 5, as well as the complementary sequences thereof.

In certain other embodiments, the invention provides oligonucleotides that hybridize to a nucleic acid sequence that spans a polymorphic locus found in SEQ ID NOs: 2, 4, 6-11, or 64 including, for example, the polymorphic locus containing a variant allele at nucleotide position 26,472 of SEQ ID NO: 64; the polymorphic locus containing a variant allele at nucleotide positions 87,712, 87,992, 89,441, 89,662, 89,853, 93,598, and 94,074 of SEQ ID NOs: 2 and 69-74, the polymorphic locus containing a variant allele at nucleotide position 34,403 of SEQ ID NO: 4, and the polymorphic locus containing a variant allele at nucleotide positions 24,707, 34,078, 35,799, 38,709, 38,947, and 41,180 of SEQ ID NOs: 6 -11, respectively, as well as the complementary sequences thereof. In other particular embodiments, the oligonucleotides comprise any of SEQ ID NOs: 13-44, 65-68, and 78-105.

In certain embodiments, the oligonucleotides of the kit hybridize immediately adjacent to one or more of the described polymorphic positions or hybridize to said polymorphic positions such that the central position of the primer aligns with the polymorphic position of said gene. For example, in specific embodiments, the kit comprises the oligonucleotides of SEQ ID NOs: 23-24, 27-28, 31-32, 35-36, 39-40, 43-44, 78-79, 82-83, 86-87, 90-91, 94-95, 98-99, and 102-103.

The invention further provides methods for determining whether an individual will be more likely to have a favorable response to the administration of a therapeutically-effective amount of a DPP-IV inhibitor or a DPP-IV inhibitor in combination with other oral antidiabetic therapy comprising the step of determining the nucleotide present within at

least one or more nucleic acid sequence(s) from an individual to be assessed at one or more polymorphic position(s) of the human TEKT5 gene sequence selected from SEQ ID NO: 63 and/or SEQ ID NO: 64, wherein the presence of the reference nucleotide at the one or more polymorphic position(s) indicates that the individual is more likely to have a favorable response to the administration of a therapeutically-effective amount of a DPP-IV inhibitor or a DPP-IV inhibitor in combination with other oral antidiabetic therapy as compared to an individual having the variant allele at said polymorphic position(s). In certain embodiments, the polymorphic position of the human TEKT5 gene sequence is at nucleotide position 26,472 of SEQ ID NO: 63 and/or SEQ ID NO: 64, wherein the presence of the reference nucleotide at nucleotide position 26,472 indicates that the individual more likely to have a favorable response to the administration of a therapeutically-effective amount of a DPP-IV inhibitor or a DPP-IV inhibitor in combination with other oral antidiabetic therapy as compared to an individual having the variant allele at that polymorphic position.

The invention further provides methods for determining whether an individual is more likely to have a favorable response to the administration of a therapeutically-effective amount of a DPP-IV inhibitor or a DPP-IV inhibitor in combination with other oral antidiabetic therapy comprising the step of determining the nucleotide present within at least one or more nucleic acid sequence(s) from an individual to be assessed at one or more polymorphic position(s) of the human TEKT5 gene sequence selected from SEQ ID NO: 63 and/or SEQ ID NO: 64, wherein the presence of the variant nucleotide at the one or more polymorphic position(s) indicates that the individual is less likely to have a favorable response to the administration of a therapeutically-effective amount of a DPP-IV inhibitor or a DPP-IV inhibitor in combination with other oral antidiabetic therapy as compared to an individual having the reference allele at said polymorphic position(s). In certain embodiments, the polymorphic position of the human TEKT5 gene sequence is at nucleotide position 26,472 of SEQ ID NO: 63 and/or SEQ ID NO: 64, wherein the presence of the variant nucleotide at nucleotide position 26,472 indicates that the individual is less likely to have a favorable response to the administration of a therapeutically-effective amount of a DPP-IV inhibitor or a DPP-IV inhibitor in combination with other oral antidiabetic therapy as compared to an individual having the reference allele at that polymorphic position.

The invention further provides methods for determining the likelihood that an individual will have a favorable response to the administration of a therapeutically-effective amount of a DPP-IV inhibitor or a DPP-IV inhibitor in combination with other oral antidiabetic therapy comprising the step of determining the nucleotide present within at least one or more nucleic acid sequence(s) from an individual to be assessed at one or more polymorphic position(s) of the human GRINL1A gene sequence selected from SEQ ID NO: 1, SEQ ID NO: 2, SEQ ID NO: 69, SEQ ID NO: 70, SEQ ID NO: 71, SEQ ID NO: 72, SEQ ID NO: 73, SEQ ID NO: 74, and SEQ ID NO: 75, wherein the presence of the reference nucleotide at the one or more polymorphic position(s) indicates that the individual is more likely to have a favorable response to the administration of a therapeutically-effective amount of a DPP-IV inhibitor or a DPP-IV inhibitor in combination with other oral antidiabetic therapy as compared to an individual having the variant allele at said polymorphic position(s). In certain embodiments, the polymorphic position of the human GRINL1A gene sequence is at nucleotide position 87,992 of SEQ ID NOs: 1, 2 and/or 75, at nucleotide position 89,441 of SEQ ID NOs: 1, 69 and/or 75, at nucleotide position 89,662 of SEQ ID NOs: 1, 70 and/or 75, at nucleotide position 89,853 of SEQ ID NOs: 1, 71 and/or 75, at nucleotide position 94,074 of SEQ ID NOs: 1, 72 and/or 75, at nucleotide position 87,712 of SEQ ID Nos: 1, 73, and/or 75, or at nucleotide position 93,598 of SEQ ID NOs: 1, 74 and/or 75 wherein the presence of the reference nucleotide at nucleotide position(s) 87,712, 87,992, 89,441, 89,662, 89,853, 93,598, and/or 94,074 indicates that the individual is more likely to have a favorable response to the administration of a therapeutically-effective amount of a DPP-IV inhibitor or a DPP-IV inhibitor in combination with other oral antidiabetic therapy as compared to an individual having the variant allele at that polymorphic position.

The invention further provides methods for determining the likelihood that an individual will have a favorable response to the administration of a therapeutically-effective amount of a DPP-IV inhibitor or a DPP-IV inhibitor in combination with other oral antidiabetic therapy comprising the step of determining the nucleotide present within at least one or more nucleic acid sequence(s) from an individual to be assessed at one or more polymorphic position(s) of the human GRINL1A gene sequence selected from SEQ ID NO: 1 and/or SEQ ID NO: 2, wherein the presence of the variant nucleotide at the one or more polymorphic position(s) indicates that the individual is less likely to have a favorable response to the administration of a therapeutically-effective amount of a DPP-IV inhibitor or a DPP-IV inhibitor in combination with other oral antidiabetic therapy as compared to an individual having the reference allele at said polymorphic position(s). In certain embodiments, the polymorphic position of the human GRINL1A gene sequence is at nucleotide position 87,992 of SEQ ID NOs: 1, 2 and/or 75, at nucleotide position 89,441 of SEQ ID NOs: 1, 69 and/or 75, at nucleotide position 89,662 of SEQ ID NOs: 1, 70 and/or 75, at nucleotide position 89,853 of SEQ ID NOs: 1, 71 and/or 75, at nucleotide position 94,074 of SEQ ID NOs: 1, 72 and/or 75, at nucleotide position 87,712 of SEQ ID Nos: 1, 73, and/or 75, or at nucleotide position 93,598 of SEQ ID NOs: 1, 74 and/or 75, wherein the presence of the variant nucleotide at nucleotide position(s) 87,712, 87,992, 89,441, 89,662, 89,853, 93,598, and/or 94,074 indicates that the individual is less likely to have a favorable response to the administration of a therapeutically-effective amount of a DPP-IV inhibitor or a DPP-IV inhibitor in combination with other oral antidiabetic therapy as compared to an individual having the reference allele at that polymorphic position.

The invention further provides methods for determining the likelihood that an individual will have a favorable response to the administration of a therapeutically-effective amount of a DPP-IV inhibitor or a DPP-IV inhibitor in combination with other oral antidiabetic therapy comprising the step of determining the nucleotide present within at least one or more nucleic acid sequence(s) from an individual to be assessed at one or more polymorphic position(s) of the human HSD11B1 gene sequence selected from SEQ ID NO: 3 and/or SEQ ID NO: 4, wherein the presence of the reference nucleotide at the one or more polymorphic position(s) indicates that the individual is more likely to have a favorable response to the administration of a therapeutically-effective amount of a DPP-IV inhibitor or a DPP-IV inhibitor in combination with other oral antidiabetic therapy as compared to an individual having the variant allele at said polymorphic position(s). In certain embodiments, the polymorphic position of the human HSD11B1 gene sequence is at nucleotide position 34,403 of SEQ ID NO: 3 and/or SEQ ID NO: 4, wherein the presence of the reference nucleotide at nucleotide position 34,403 indicates that the individual is more likely to have a favorable response to the administration of a therapeutically-effective amount of a DPP-IV inhibitor or a DPP-IV inhibitor in combination with other oral antidiabetic therapy as compared to an individual having the variant allele at that polymorphic position.

The invention further provides methods for determining the likelihood that an individual will have a favorable response to the administration of a therapeutically-effective amount of a DPP-IV inhibitor or a DPP-IV inhibitor in combination with other oral antidiabetic therapy comprising the step of determining the nucleotide present within at least one or more nucleic acid sequence(s) from an individual to be assessed at one or more polymorphic position(s) of the human HSD11B1 gene sequence selected from SEQ ID NO: 3 and/or SEQ ID NO: 4, wherein the presence of the variant nucleotide at the one or more polymorphic position(s) indicates that the individual is more likely to have a favorable response to the administration of a therapeutically-effective amount of a DPP-IV inhibitor or a DPP-IV inhibitor in combination with other oral antidiabetic therapy as compared to an individual having the reference allele at said polymorphic position(s). In certain embodiments, the polymorphic position of the human HSD11B1 gene sequence is at nucleotide position 34,403 of SEQ ID NO: 3 and/or SEQ ID NO: 4, wherein the presence of the variant nucleotide at nucleotide position 34,403 indicates that the individual is less likely to have a favorable response to the administration of a therapeutically-effective amount of a DPP-IV inhibitor or a DPP-IV inhibitor in combination with other oral antidiabetic therapy as compared to an individual having the reference allele at that polymorphic position.

The invention further provides methods for determining the likelihood that an individual will have a favorable response to the administration of a therapeutically-effective amount of a DPP-IV inhibitor or a DPP-IV inhibitor in combination with other oral antidiabetic therapy comprising the step of determining the nucleotide present within at least one or more nucleic acid sequence(s) from an individual to be assessed at one or more polymorphic position(s) of the human CHST10 gene sequence selected from SEQ ID NO: 5, SEQ ID NO: 6, SEQ ID NO: 7, SEQ ID NO: 8, SEQ ID NO: 9, SEQ ID NO: 10, SEQ ID NO: 11 and SEQ ID NO: 12, wherein the presence of the reference nucleotide at the one or more polymorphic position(s) indicates that the individual is more likely to have a favorable response to the administration of a therapeutically-effective amount of a DPP-IV inhibitor or a DPP-IV inhibitor in combination with other oral antidiabetic therapy as compared to an individual having the variant allele at said polymorphic position(s). In certain embodiments, the polymorphic position of the human CHST10 gene sequence is at nucleotide position 24,707 of SEQ ID NOs: 5, 6 and/or 12, at nucleotide position 34,078 of SEQ ID NOs: 5, 7 and/or 12, at nucleotide position 35,799 of SEQ ID NOs: 5, 8 and/or 12, at nucleotide position 38,709 of SEQ ID NOs: 5, 9 and/or 12, at nucleotide position 38,947 of SEQ ID NOs: 5, 10 and/or 12, or at nucleotide position 41,180 of SEQ ID NOs: 5, 11 and/or 12 wherein the presence of the reference nucleotide at nucleotide position 24,707, 34,078, 35,799, 38,709, 38,947, and/or 41,180 indicates that the individual is more likely to have a favorable response to the administration of a therapeutically-effective amount of a DPP-IV inhibitor or a DPP-IV inhibitor in combination with other oral antidiabetic therapy as compared to an individual having the variant allele at that polymorphic position.

The invention further provides methods for determining the likelihood that an individual will have a favorable response to the administration of a therapeutically-effective amount of a DPP-IV inhibitor or a DPP-IV inhibitor in combination with other oral antidiabetic therapy comprising the step of determining the nucleotide present within at least one or more nucleic acid sequence(s) from an individual to be assessed at one or more polymorphic position(s) of the human CHST10 gene sequence selected from SEQ ID NO: 5 and/or SEQ ID NO: 6, wherein the presence of the variant nucleotide at the one or more polymorphic position(s) indicates that the individual is less likely to have a favorable response to the administration of a therapeutically-effective amount of a DPP-IV inhibitor or a DPP-IV inhibitor in combination with other oral antidiabetic therapy as compared to an individual having the reference allele at said polymorphic position(s). In certain embodiments, the polymorphic position of the human CHST10 gene sequence is at nucleotide position 24,707 of SEQ ID NOs: 5, 6 and/or 12, at nucleotide position 34,078 of SEQ ID NOs: 5, 7, and/or 12, at nucleotide position 35,799 of SEQ ID NOs: 5, 8, and/or 12, at nucleotide position 38,709 of SEQ ID NOs: 5, 9, and/or 12, at nucleotide position 38,947 of SEQ ID NOs: 5, 10, and/or 12, or at nucleotide position 41,180 of SEQ ID NOs: 5, 11, and/or 12, wherein the presence of the variant nucleotide at nucleotide position 24,707, 34,078, 35,799, 38,709, 38,947, and/or 41,180 indicates that the individual is less likely to have a favorable response to the administration of a therapeutically-effective amount of a DPP-IV inhibitor or a DPP-IV inhibitor in combination with other oral antidiabetic therapy as compared

to an individual having the reference allele at that polymorphic position.

Specific embodiments of the present invention will become evident from the following more detailed description of certain preferred embodiments and the claims.

## BRIEF DESCRIPTION OF THE DRAWINGS

**Figure 1** shows a statistical association between human GRINL1A SNP (nucleotide position 87,992 of SEQ ID NOs: 1 and 2) alleles "T" (reference) and "C" (variant) with a likelihood of a favorable response to the administration of a therapeutically-effective amount of a DPP-IV inhibitor. Results are shown in terms of incidence of each genotype residing in a patient that was part of non-responder (NR) and super responder (SR) DPP-IV inhibitor groups. As shown, homozygous "T" allele patients ("T/T") are more likely to have a favorable response to the administration of a therapeutically-effective amount of a DPP-IV inhibitor than patients having the heterozygous allele ("T/C") or homozygous "C" allele ("C/C"). P-values from the genotype association test are shown. Overall P-values value was calculated by pooling data from both the Phase II and Phase III studies.

**Figure 2** shows a statistical association between human HSD11B1 SNP (nucleotide position 34,403 of SEQ ID NOs: 3 and 4) alleles "C" (reference) and "T" (variant) with a likelihood of a favorable response to the administration of a therapeutically-effective amount of a DPP-IV inhibitor.. Results are shown in terms of fold incidence of each genotype residing in a patient that was part of non-responder (NR) and super responder (SR) DPP-IV inhibitor groups. As shown, homozygous "C" allele patients ("C/C") are more likely to have a favorable response to the administration of a therapeutically-effective amount of a DPP-IV inhibitor than patients having the heterozygous allele ("C/T") or homozygous "T" allele ("T/T"). P-values from the genotype association test are shown. Overall P-values value was calculated by pooling data from both the Phase II and Phase III studies.

**Figure 3** shows a statistical association between human CHST10 SNP (nucleotide position 24,707 of SEQ ID NOs: 5 and 6) alleles "G" (reference) and "A" (variant) with a likelihood of a favorable response to the administration of a therapeutically-effective amount of a DPP-IV inhibitor. Results are shown in terms of fold incidence of each genotype residing in a patient that was part of non-responder and super responder DPP-IV inhibitor groups. As shown, homozygous "G" allele patients ("G/G") are more likely to have a favorable response to the administration of a therapeutically-effective amount of a DPP-IV inhibitor than patients having the heterozygous allele ("G/A") or homozygous "A" allele ("A/A"). P-values from the genotype association test are shown. Overall P-values value was calculated by pooling data from both the Phase II and Phase III studies.

**Figure 4** shows a statistical association between human CHST10 SNP (nucleotide position 34,078 of SEQ ID NOs: 5 and 7) alleles "C" (reference) and "T" (variant) with a likelihood of a favorable response to the administration of a therapeutically-effective amount of a DPP-IV inhibitor. Results are shown in terms of fold incidence of each genotype residing in a patient that was part of non-responder and good responder DPP-IV inhibitor groups. As shown, homozygous "C" allele patients ("C/C") are more likely to have a favorable response to the administration of a therapeutically-effective amount of a DPP-IV inhibitor than patients having the heterozygous allele ("C/T") or homozygous "T" allele ("T/T"). P-values from the genotype association test are shown. Overall P-values value was calculated by pooling data from both the Phase II and Phase III studies.

**Figure 5** shows a statistical association between human CHST10 SNP (nucleotide position 35,799 of SEQ ID NOs: 5 and 8) alleles "G" (reference) and "C" (variant) with a likelihood of a favorable response to the administration of a therapeutically-effective amount of a DPP-IV inhibitor. Results are shown in terms of fold incidence of each genotype residing in a patient that was part of non-responder and super responder DPP-IV inhibitor groups. As shown, homozygous "G" allele patients ("G/G") are more likely to have a favorable response to the administration of a therapeutically-effective amount of a DPP-IV inhibitor than patients having the heterozygous allele ("G/C") or homozygous "C" allele homozygous patients ("C/C"). P-values from the genotype association test are shown. Overall P-values value was calculated by pooling data from both the Phase II and Phase III studies.

**Figure 6** shows a statistical association between human CHST10 SNP (nucleotide position 38,709 of SEQ ID NOs: 5 and 9) alleles "A" (reference) and "G" (variant) with a likelihood of a favorable response to the administration of a therapeutically-effective amount of a DPP-IV inhibitor. Results are shown in terms of fold incidence of each genotype residing in a patient that was part of non-responder and super responder DPP-IV inhibitor groups. As shown, homozygous "A" allele patients ("A/A") are more likely to have a favorable response to the administration of a

therapeutically-effective amount of a DPP-IV inhibitor than patients having the heterozygous allele ("A/G") or homozygous "G" allele ("G/G"). P-values from the genotype association test are shown. Overall P-values value was calculated by pooling data from both the Phase II and Phase III studies.

**Figure 7** shows a statistical association between human CHST10 SNP (nucleotide position 38,947 of SEQ ID NOs: 5 and 10) alleles "C" (reference) and "A" (variant) with a likelihood of a favorable response to the administration of a therapeutically-effective amount of a DPP-IV inhibitor. Results are shown in terms of fold incidence of each genotype residing in a patient that was part of non-responder and super responder DPP-IV inhibitor groups. As shown, homozygous "C" allele patients ("C/C") are more likely to have a favorable response to the administration of a therapeutically-effective amount of a DPP-IV inhibitor than patients having the heterozygous allele ("C/A") or homozygous "A" allele ("A/A"). P-values from the genotype association test are shown. Overall P-values value was calculated by pooling data from both the Phase II and Phase III studies.

**Figure 8** shows a statistical association between human CHST10 SNP (nucleotide position 41,180 of SEQ ID NOs: 5 and 11) alleles "T" (reference) and "C" (variant) with a likelihood of a favorable response to the administration of a therapeutically-effective amount of a DPP-IV inhibitor. Results are shown in terms of fold incidence of each genotype residing in a patient that was part of non-responder and super responder DPP-IV inhibitor groups. As shown, homozygous "T" allele patients ("T/T") are more likely to have a favorable response to the administration of a therapeutically-effective amount of a DPP-IV inhibitor than patients having the heterozygous allele ("T/C") or homozygous "C" allele ("C/C"). P-values from the genotype association test are shown. Overall P-values value was calculated by pooling data from both the Phase II and Phase III studies.

**Figure 9** shows a statistical association between human GRINL1A SNP (nucleotide position 87,992 of SEQ ID NOs: 1 and 2) alleles "T" (WT, reference) and "C" (SNP carriers, variant) with a likelihood of a favorable response to the administration of a therapeutically-effective amount of a DPP-IV inhibitor. The response to DPP-IV inhibitor is assessed by measuring fasting glycosylated hemoglobin (HbA1c) levels in blood. Results are shown in terms of mean change in HbAlc levels for each genotype patient. As shown, reference carriers have a higher reduction in mean glycosylated hemoglobin than SNP variant carriers in response to saxagliptin treatment.

**Figure 10** shows a statistical association between human GRINL1A SNP (nucleotide position 87,992 of SEQ ID NOs: 1 and 2) alleles "T" (WT, reference) and "C" (SNP carriers, variant) with a likelihood of a favorable response to the administration of a therapeutically-effective amount of a DPP-IV inhibitor. The response to DPP-IV inhibitor is assessed by measuring fasting blood glucose levels in blood. Results are shown in terms of mean change in fasting glucose levels for each genotype patient. As shown, reference carriers have a higher reduction in mean fasting glucose levels than SNP carriers in response to saxagliptin treatment.

**Figure 11** shows a statistical association between human GRINL1A (nucleotide position 87,992 of SEQ ID NOs: 1 and 2) alleles "T" (WT, reference) and "C" (SNP carriers, variant) with a likelihood of a favorable response to the administration of a therapeutically-effective amount of a DPP-IV inhibitor. The response to DPP-IV inhibitor is assessed by measuring AUC Glucose levels in blood. Results are shown in terms of mean change in AUC Glucose levels for each genotype patient. As shown, reference carriers have a higher reduction in AUC Glucose levels than SNP carriers in response to saxagliptin treatment.

**Figure 12** shows a statistical association between human HSD11B1 SNP (nucleotide position 34,403 of SEQ ID NOs: 3 and 4) alleles "C" (WT, reference) and "T" (SNP carriers, variant) with a likelihood of a favorable response to the administration of a therapeutically-effective amount of a DPP-IV inhibitor. The response to DPP-IV inhibitor is assessed by measuring glycosylated hemoglobin (HbA1c) levels in blood. Results are shown in terms of mean change in HbAlc levels for each genotype patient. As shown, reference carriers have a higher reduction in mean glycosylated hemoglobin than SNP carriers in response to saxagliptin treatment.

**Figure 13** shows a statistical association between human CHST10 SNP (nucleotide position 24,707 of SEQ ID NOs: 5 and 6) alleles "G" (WT, reference) and "A" (SNP carriers, variant) with a likelihood of a favorable response to the administration of a therapeutically-effective amount of a DPP-IV inhibitor. The response to DPP-IV inhibitor is assessed by measuring glycosylated hemoglobin (HbA1c) levels in blood. Results are shown in terms of mean change in HbAlc levels for each genotype patient. As shown, reference carriers have a higher reduction in mean glycosylated hemoglobin than SNP carriers in response to saxagliptin treatment.

**Figure 14** shows a statistical association between human CHST10 SNP (nucleotide position 34,078 of SEQ ID

NOs: 5 and 7) alleles "C" (WT, reference) and "T" (SNP carriers, variant) with a likelihood of a favorable response to the administration of a therapeutically-effective amount of a DPP-IV inhibitor by measuring mean change in HbAlc levels in blood. As shown, reference carriers have a higher reduction in mean glycosylated hemoglobin than SNP carriers in response to saxagliptin treatment.

**Figure 15** shows a statistical association between human CHST10 SNP (nucleotide position 35,799 of SEQ ID NOs: 5 and 8) alleles "G" (WT, reference) and "C" (SNP carriers, variant) with a likelihood of a favorable response to the administration of a therapeutically-effective amount of a DPP-IV inhibitor by measuring mean change in HbAlc levels in blood. As shown, reference carriers have a higher reduction in mean glycosylated hemoglobin than SNP carriers in response to saxagliptin treatment.

**Figure 16** shows a statistical association between human CHST10 SNP (nucleotide position 38,709 of SEQ ID NOs: 5 and 9) alleles "A" (WT, reference) and "G" (SNP carriers, variant) with a likelihood of a favorable response to the administration of a therapeutically-effective amount of a DPP-IV inhibitor by measuring mean change in HbAlc levels in blood. As shown, reference carriers have a higher reduction in mean glycosylated hemoglobin than SNP carriers in response to saxagliptin treatment.

**Figure 17** shows a statistical association between human CHST10 SNP (nucleotide position 38,947 of SEQ ID NOs: 5 and 10) alleles "C" (WT, reference) and "A" (SNP carriers, variant) with a likelihood of a favorable response to the administration of a therapeutically-effective amount of a DPP-IV inhibitor by measuring mean change in HbA1c levels in blood. As shown, reference carriers have a higher reduction in mean glycosylated hemoglobin than SNP carriers in response to saxagliptin treatment.

**Figure 18** shows a statistical association between human CHST10 SNP (nucleotide position 41,180 of SEQ ID NOs: 5 and 11) alleles "T" (WT, reference) and "C" (SNP carriers, variant) with a likelihood of a favorable response to the administration of a therapeutically-effective amount of a DPP-IV inhibitor by measuring mean change in HbA1c levels in blood. As shown, reference carriers have a higher reduction in mean glycosylated hemoglobin than SNP carriers in response to saxagliptin treatment.

**Figure 19** shows a statistical association between human TEKT5 SNP (nucleotide position 26,472 of SEQ ID NOs: 63 and 64) alleles "T" (reference) and "C" (variant) with a likelihood of a favorable response to the administration of a therapeutically-effective amount of a DPP-IV inhibitor. Results are shown as 95% confidence intervals of the mean HBA1C, both observed and as estimated by an ANCOVA model. The model-adjusted week 12 HbA1C values were compared between wild-type homozygous (genotype 0/0) and heterozygous (genotype 0/1) individuals on combination treatment. These values were found to be significantly different (Contrast p-value = 0.0042). This result and the pattern displayed in the plot indicate a a significant association between the wild-type homozygous genotype and lower HbA1C on treatment.

**Figure 20** shows a statistical association between human GRINL1A SNP (nucleotide position 87,992 of SEQ ID NOs: 1, 2 and 75) alleles "T" (reference) and "C" (variant) with a likelihood of a favorable response to the administration of a therapeutically-effective amount of a DPP-IV inhibitor or a DPP-IV inhibitor in combination with other oral antidiabetic therapy.In the plot, "Placebo" denotes Metformin arm, and "Drug" denotes Combination arm. The model-adjusted week 24 HbA1C values were compared between wild-type homozygous (genotype 0/0) and heterozygous (genotype 0/1) individuals on combination treatment. These values were found to be significantly different (Contrast p-value = 0.0536). This result and the pattern displayed in the plot indicate a likely association between the wild-type homozygous genotype and lower HbA1C on combination treatment, just outside our criteria for significance at 0.05.

**Figure 21** shows a statistical association between human GRINL1A SNP (nucleotide position 87,992 of SEQ ID NOs: 1, 2 and 75) alleles "T" (reference) and "C" (variant) with a likelihood of a favorable response to the administration of a therapeutically-effective amount of a DPP-IV inhibitor or a DPP-IV inhibitor in combination with other oral antidiabetic therapy.In the plot, "Placebo" denotes Glyburide arm, and "Drug" denotes Combination arm. The model-adjusted week 24 HbA1C values were compared between wild-type homozygous (genotype 0/0) and heterozygous (genotype 0/1) individuals on combination treatment. These values were found to be significantly different (Contrast p-value = 0.0571). This result and the pattern displayed in the plot indicate a likely association between the wild-type homozygous genotype and lower HbA1C on combination treatment, just outside our criteria for significance at 0.05.

## DETAILED DESCRIPTION OF THE INVENTION

As used herein, the skilled worker will understand the meaning of the following terms:

Glutamate Receptor, Ionotropic, N-Methyl D-Aspartate-Like 1A (GRINL1A) gene is a gene whose protein product facilitates signaling via the glutamatergic pathway through interaction with the NMDA receptor. Multiple splice isoforms have been identified, one of which is identified herein as SEQ ID NO 69 anmd by reference to GenBank Accession No. NM_001018090. In some instances, GRINL1A splice isoforms are annotated as GCOM1.Tektin 5 (TEKT5) gene is a gene whose product has been implicated in the cellular construction of cilia and flagella. It is identified herein as SEQ ID NO 63 and by reference to GenBank Accession No. NM_144674.1.

11β hydroxysteroid dehydrogenase type 1 (referred to herein as 11β-HSD1 or HSD11B1) gene is a gene having two isozymes of 11-HSD, 11β-HSD type 1 and type 2, that are the products of distinct genes and differ considerably in tissue distribution and function. 11β-HSD1 has been hypothesized to play a role in human obesity, adipose tissue formation and insulin resistance along with other diseases in that glucocorticoids are typically implicated, such as osteoporosis and glaucoma. (Tomlinson et al., 2004, 11ß-Hydroxysteroid Dehydrogenase Type 1: A Tissue-Specific Regulator of Glucocorticoid Response, Endocrine Reviews 25(5): 831-866). It is identified herein as SEQ ID NO 3 and by reference to GenBank Accession No. NM_005525.

The human cDNA encoding 11β-HSD1 comprises approximately 1.4 kb in length predicted an open reading frame of 876 bp and a protein of 292 amino acids, which is 77% identical at the amino acid level to the rat enzyme. The human gene has been designated HSD11β1 and is 30 kb in size, consisting of six exons (182, 130, 111, 185, 143, and 617 bp, respectively) and five introns (776, 767, 120, 25,300, and 1,700 bp, respectively). (Draper et al., 2002, J Clin Endocrinol Metab 87:4984-4990). Alternate 11ß-HSD1 mRNA transcripts, as a result of differential promoter usage and alternate splicing mechanisms, have been demonstrated, giving rise to three proteins referred to as 11ß-HSD1A, 11ß-HSD1B (or HSD11B1), and 11ß-HSD1C.

To date, there are few reports describing polymorphism in and around the HSD11B1 locus. A scan of the GenBank single-nucleotide polymorphism (SNP) database (db SNP on NCBI website) reveals a number of documented sequence variations detected primarily through human genome-sequencing projects.

A role for HSD11ß1 in regulating insulin sensitivity can occur at the level of the liver (hepatic gluconeogenesis), adipose tissue (central obesity), or muscle. Various *in vitro* and rodent studies suggest a modulatory role for 11ß-HSD1 in the control of hepatic gluconeogenesis. (Alberts et al., 2002, Diabetologia 45:1528-1532; Alberts et al., 2003, J Med Chem 45:3813-3815). Observational clinical studies in states of impaired glucose tolerance have, in almost all cases, demonstrated decreased hepatic 11ß-HSD1 reductase activity as measured by cortisol generation profiles after an oral dose of cortisone acetate (Smith et al., 1982, Biol Neonate 42:201-207; Andrews et al., 2003, J Clin Endocrinol Metab 87:5587-5593; Rask et al., 2002, J Clin Endocrinol Metab 87:3330-3336, Stewart et al., 1999, J Clin Endocrinol Metab 84:1022-1027) and urinary F/E metabolite ratios (Rask et al., 2001, J Clin Endocrinol Metab 86:1418-1421; Stewart et al., 1999, J Clin Endocrinol Metab 84:1022-1027); Paterson JM et al., 2004, Proc Natl Acad Sci USA 101:7088-7093. 11ß-HSD1 has also emerged as a therapeutic target in the metabolic syndrome.

Carbohydrate sulfotransferase (CHST10) is a gene whose protein product catalyzes transfer of sulfate to position 3 of terminal glucuronic acid of both protein- and lipid-linked oligosaccharides. Known also as HNK-1sulfotransferase and MGC17148, it plays a role in the biosynthesis of HNK-1, a neuronally expressed carbohydrate that contains a sulfoglucuronyl residue carried by many neural recognition molecules. It is identified herein as SEQ ID NO 5 and by reference to GenBank Accession No. NM_004854.

The invention provides nucleic acid molecules comprising a single nucleotide polymorphism (SNP) at a specific location, referred to herein as the polymorphic locus, and complements thereof. The nucleic acid molecules, e.g., a gene, which include the SNP has at least two alleles, referred to herein as the reference allele and the variant allele. The reference allele frequently, but not always, is the more common allele found in a heterogeneous population. Frequently, but not always, the reference allele is also the first identified allelic form. Other allelic forms are designated as variant alleles.

The TEKT5 SNPs were identified by whole genome scans of DNA from a large number of individuals that were subjected to DPP-IV inhibitor treatment and comparing the mean HBA1C response among SNP genotypes. These studies showed that the reference allele at nucleotide position 26,472 of SEQ ID NO: 63 is associated with a favorable response to administration of a therapeutically-effective amount of a DPP-IV inhibitor.. The TEKT5 SNPs were identified due to the significant differences in mean HBA1C response among individuals with different genotypes on treatment, taking into account covariates such as demographic differences. Apparent favored HBA1C response for individuals homozygous for the wild-type allele was observed. The invention also provides a variant allele of the described TEKT5 gene and complements of the variant allele. The variant allele differs from the reference allele by one nucleotide at the polymorphic locus found at nucleotide position 26,472 of SEQ ID NOs: 63 and 64.

The invention also provides novel polynucleotides of the human GRINL1A gene comprising at least one single nucleotide polymorphism (SNP) that was shown to be associated with an increased likelihood of a favorable response to the administration of a therapeutically-effective and pharmaceutically acceptable amount of a DPP-IV inhibitor or a DPP-IV inhibitor in combination with other oral antidiabetic therapy. One GRINL1A SNP, rs11853270, was identified by whole genome scan in a large number of individuals that were subjected to DPP-IV inhibitor treatment and comparing the genotype frequencies of those individuals who were non-responders to the nucleotide sequences of those individuals who were super responders of DPP-IV inhibition. Additional GRINL1A SNPs were identified by sequencing the GRINL1A genomic DNA from a large number of individuals that were subjected to DPP-IV inhibitor treatment and comparing the GRINL1A nucleotide sequences of those individuals who were non-responders to the nucleotide sequences of those individuals who were super responders of DPP-IV inhibition. These studies showed that the reference alleles at nucleotide position 87,992 of SEQ ID NO: 1, at nucleotide position 89,441 of SEQ ID NO: 1, at nucleotide position 89,662 of SEQ ID NO: 1, at nucleotide position 89,853 of SEQ ID NO: 1, at nucleotide position 94,074 of SEQ ID NO: 1, at nucleotide position 87,712 of SEQ ID NO: 1, or at nucleotide position 93,598 of SEQ ID NO: 1, are associated with an increased likelihood of a favorable response to administration of a therapeutically-effective amount of a DPP-IV inhibitor or a DPP-IV inhibitor in combination with other oral antidiabetic therapy. Thus, the invention provides a reference allele(s) of the described GRINL1A gene and complements of the reference allele(s).

The invention also provides variant alleles of the described GRINL1A gene and complements of the variant alleles. One of the variant alleles differs from the reference allele by one nucleotide at the polymorphic locus found at nucleotide position 87,992 of SEQ ID NOs: 1, 2 and/or 75, at nucleotide position 89,441 of SEQ ID NOs: 1, 69 and/or 75, at nucleotide position 89,662 of SEQ ID NOs: 1, 70 and/or 75, at nucleotide position 89,853 of SEQ ID NOs: 1, 71 and/or 75, at nucleotide position 94,074 of SEQ ID NOs: 1, 72 and/or 75, at nucleotide position 87,712 of SEQ ID Nos: 1, 73, and/or 75, or at nucleotide position 93,598 of SEQ ID NOs: 1, 74 and/or 75.

The invention also provides novel polynucleotides of the human HSD11B1 gene comprising at least one single nucleotide polymorphism (SNP) that was shown to be associated with an increased likelihood of a favorable response to the administration of a therapeutically-effective amount of a DPP-IV inhibitor. The HSD11B1 SNP was identified by whole genome scan in a large number of individuals that were subjected to DPP-IV inhibitor treatment and comparing the genotype frequencies of those individuals who were non-responders to the nucleotide sequences of those individuals who were super responders of DPP-IV inhibition. These studies showed that the reference allele at nucleotide position 34,403 of SEQ ID NO: 3 is associated with an increased likelihood of a favorable response to administration of a therapeutically-effective amount of a DPP-IV inhibitor or a DPP-IV inhibitor in combination with other oral antidiabetic therapy. Thus, the invention provides a reference allele of the described HSD11B1 gene and complements of the reference allele.

The invention also provides a variant allele of the described HSD11B1 gene and complements of the variant allele. The variant allele differs from the reference allele by one nucleotide at the polymorphic locus found at nucleotide position 34,403 of SEQ ID NOs: 3 and 4.

The invention also provides novel polynucleotides of the human CHST10 gene comprising at least one single nucleotide polymorphism (SNP) that was shown to be associated with an increased likelihood of a favorable response to the administration of a therapeutically-effective and pharmaceutically acceptable amount of a DPP-IV inhibitor or a DPP-IV inhibitor in combination with other oral antidiabetic therapy. The CHST10 SNPs were identified by whole genome scan in a large number of individuals that were subjected to DPP-IV inhibitor treatment and comparing the genotype frequencies of those individuals who were non-responders to the nucleotide sequences of those individuals who were super responders of DPP-IV inhibition. These studies showed that the reference alleles at nucleotide position 24,707 of SEQ ID NO: 5, position 34,078 of SEQ ID NO: 5, position 35,799 of SEQ ID NO: 5, position 38,709 of SEQ ID NO: 5, position 38,947 of SEQ ID NO: 5, and position 41,180 of SEQ ID NO: 5 are associated with an increased likelihood of a favorable response to administration of a therapeutically-effective amount of a DPP-IV inhibitor or a DPP-IV inhibitor in combination with other oral antidiabetic therapy. Thus, the invention provides a reference allele(s) of the described CHST10 gene and complements of the reference allele(s).

The invention also provides variant alleles of the described CHST10 gene and complements of the variant alleles. One of the variant alleles differs from the reference allele by one nucleotide at the polymorphic locus found at nucleotide position 24,707 of SEQ ID NOs: 5, 6, and/or 12, at nucleotide position 34,078 of SEQ ID NOs: 5, 7, and/or 12, at nucleotide position 35,799 of SEQ ID NOs: 5, 8, and/or 12, at nucleotide position 38,709 of SEQ ID NOs: 5, 9, and/or 12, at nucleotide position 38,947 of SEQ ID NOs: 5, 10, and/or 12, or nucleotide position 41,180 of SEQ ID NOs: 5, 11, and/or 12.

The nucleic acid molecules or polynucleotides of the invention can comprise DNA or RNA, can be double- or single-stranded, and can comprise SNP-containing fragments thereof. The invention further provides fragments of the variant alleles and fragments of complements of the variant alleles that comprise the site of the SNP (e.g., polymorphic locus) and are at least five nucleotides in length. The fragments can be about 5 nucleotides to about 100 nucleotides in length including, for example, about 5 nucleotides to about 10, 15, 20, 25, 30, 40, 50, 60, 70, 80, 90 or 100 nucleotides,

about 10 nucleotides to about 20, 25, 30, 35, 40, 50, 60, 70, 80, 90 or 100 nucleotides, about 15 nucleotides to about 25, 35, 45, 50, 60, 70, 80, 90, or 100 nucleotides, about 20 nucleotides to about 30, 40, 50, 60, 70, 80, 90, or 100 nucleotides, about 30 nucleotides to about 40, 50, 60, 70, 80, 90, or 100 nucleotides, about 40 nucleotides to about 50, 60, 70, 80, 90, or 100 nucleotides, about 50 nucleotides to about 60, 70, 80, 90, or 100 nucleotides, about 60 nucleotides to about 70, 80, 90, or 100 nucleotides, about 70 nucleotides to about 80, 90, or 100 nucleotides, about 80 nucleotides to about 90 or 100 nucleotides, and about 90 nucleotides to about 100 nucleotides, and preferably comprise at least one polymorphic allele.

Examples of polymorphisms include those found at nucleotide position 26,472 of SEQ ID NOs: 63 and 64, nucleotide position 87,992 of SEQ ID NOs: 1, 2 and/or 75, nucleotide position 89,441 of SEQ ID NOs: 1, 69 and/or 75, nucleotide position 89,662 of SEQ ID NOs: 1, 70 and/or 75, nucleotide position 89,853 of SEQ ID NOs: 1, 71 and/or 75, nucleotide position 94,074 of SEQ ID NOs: 1, 72 and/or 75, nucleotide position 87,712 of SEQ ID Nos: 1, 73, and/or 75, nucleotide position 93,598 of SEQ ID NOs: 1, 74 and/or 75, nucleotide position 34,403 of SEQ ID NOs: 3 and 4, nucleotide position 24,707 of SEQ ID NOs: 5, 6 and/or 12, nucleotide position 34,078 of SEQ ID NOs: 5, 7 and/or 12, nucleotide position 35,799 of SEQ ID NOs: 5, 8 and/or 12, nucleotide position 38,709 of SEQ ID NOs: 5, 9 and/or 12, nucleotide position 38,947 of SEQ ID NOs: 5, 10 and/or 12, and nucleotide position 41,180 of SEQ ID NOs: 5, 11 and/or 12.

In one particular embodiment, the invention provides human TEKT5 nucleic acid having the nucleotide sequence of SEQ ID NO: 63 or SEQ ID NO: 64 comprising a single nucleotide polymorphism at a polymorphic locus found at nucleotide 26.472 of SEQ ID NO: 63 or SEQ ID NO: 64. The reference nucleotide for the polymorphic locus at nucleotide 26,472 is "T". The variant nucleotide for the polymorphic locus at nucleotide 26,472 is "C". The nucleotide sequence(s) can be double- or single- stranded. The nucleotide sequence(s) can comprise the complementary sequence(s) of SEQ ID NOs: 63 and 64. The nucleotide sequence(s) can comprise the complement of the reference nucleotide for the polymorphic locus at nucleotide 26,472 ("A"). The nucleotide sequence(s) can comprise the complement of the variant nucleotide for the polymorphic locus at nucleotide 26,472 ("G"). In certain embodiments, the invention further provides a portion of the human TEKT5 gene comprising one or more polymorphic loci selected from nucleotide 26,472 of SEQ ID NO: 63 and/or SEQ ID NO: 64.

In another particular embodiment, the invention provides human GRINL1A nucleic acid having the nucleotide sequence of SEQ ID NOs: 1-2 and 69-75 comprising a single nucleotide polymorphism at a polymorphic locus at nucleotide position 87,712, 87,992, 89,441, 89,662, 89,853, 93,598, and/or 94,074 of SEQ ID NO: 1 or SEQ ID NOs: 2, 69-75. The reference nucleotide for the polymorphic locus at nucleotide position 87,712 of SEQ ID NO: 1 is "A", the reference nucleotide for the polymorphic locus at nucleotide position 87,992 of SEQ ID NO: 1 is "T", the reference nucleotide for the polymorphic locus at nucleotide position 89,441 of SEQ ID NO: 1 is "A", the reference nucleotide for the polymorphic locus at nucleotide position 89,662 of SEQ ID NO: 1 is "G", the reference nucleotide for the polymorphic locus at nucleotide position 89,853 of SEQ ID NO: 1 is "G", the reference nucleotide for the polymorphic locus at nucleotide position 93,598 pf SEQ ID NO: 1 is "G", and the reference nucleotide for the polymorphic locus at nucleotide position 94,074 of SEQ ID NO: 1 is "G".

The variant nucleotide for the polymorphic locus at nucleotide position 87,712 of SEQ ID NO: 71 or 75 is "G". The variant nucleotide for the polymorphic locus at nucleotide position 87,992 of SEQ ID NO: 2 or 75 is "C". The variant nucleotide for the polymorphic locus at nucleotide position 89,441 of SEQ ID NO: 69 or 75 is "G". The variant nucleotide for the polymorphic locus at nucleotide position 89,662 of SEQ ID NO: 70 or 75 is "A". The variant nucleotide for the polymorphic locus at nucleotide position 89,853 of SEQ ID NO: 71 or 75 is "A". The variant nucleotide for the polymorphic locus at nucleotide position 93,598 of SEQ ID NO: 74 or 75 is "A". The variant nucleotide for the polymorphic locus at nucleotide position 94,074 of SEQ ID NO: 72 or 75 is "C".

The nucleotide sequences of the invention can be double- or single- stranded. The nucleotide sequence(s) can comprise the complementary sequence(s) of SEQ ID NOs: 1, 2 and 69-75. The nucleotide sequence(s) can comprise the complement of the reference nucleotide for the polymorphic locus at nucleotide 87,712 ("A"). The nucleotide sequence(s) can comprise the complement of the variant nucleotide for the polymorphic locus at nucleotide 87,712 ("G"). The nucleotide sequence(s) can comprise the complement of the reference nucleotide for the polymorphic locus at nucleotide 87,992 ("T"). The nucleotide sequence(s) can comprise the complement of the variant nucleotide for the polymorphic locus at nucleotide 87,992 ("C"). The nucleotide sequence(s) can comprise the complement of the reference nucleotide for the polymorphic locus at nucleotide 89,441 ("A"). The nucleotide sequence(s) can comprise the complement of the variant nucleotide for the polymorphic locus at nucleotide 89,441 ("G"). The nucleotide sequence(s) can comprise the complement of the reference nucleotide for the polymorphic locus at nucleotide 89,662 ("G"). The nucleotide sequence(s) can comprise the complement of the variant nucleotide for the polymorphic locus at nucleotide 89,662 ("A"). The nucleotide sequence(s) can comprise the complement of the reference nucleotide for the polymorphic locus at nucleotide 89,853 ("G"). The nucleotide sequence(s) can comprise the complement of the variant nucleotide for the polymorphic locus at nucleotide 89,853 ("A"). The nucleotide sequence(s) can comprise the complement of the reference nucleotide for the polymorphic locus at nucleotide 93,598 ("G"). The nucleotide sequence(s) can comprise the complement of the variant nucleotide for the polymorphic locus at nucleotide 93,598 ("A"). The nucleotide sequence(s) can

comprise the complement of the reference nucleotide for the polymorphic locus at nucleotide 94,074 ("G"). The nucleotide sequence(s) can comprise the complement of the variant nucleotide for the polymorphic locus at nucleotide 94,074 ("C"). The invention further provides a portion of the human GRINL1A gene comprising one or more polymorphic loci selected from nucleotide 87,712, 87,992, 89,441, 89,662, 89,853, 93,598, and 94,074 of SEQ ID NO: 1 and/or SEQ ID NO: 2, 69-75.

In another particular embodiment, the invention provides human HSD11B1nucleic acid having the nucleotide sequence of SEQ ID NO: 3 or SEQ ID NO: 4 comprising a single nucleotide polymorphism at a polymorphic locus found at nucleotide 34,403 of SEQ ID NO: 3 or SEQ ID NO: 4. The reference nucleotide for the polymorphic locus at nucleotide 34,403 is "C". The variant nucleotide for the polymorphic locus at nucleotide 34,403 is "T". The nucleotide sequences of the invention can be double- or single- stranded. The nucleotide sequence(s) can comprise the complementary sequence(s) of SEQ ID NOs: 3 and 4. The nucleotide sequence(s) can comprise the complement of the reference nucleotide for the polymorphic locus at nucleotide 34,403 ("G"). The nucleotide sequence(s) can comprise the complement of the variant nucleotide for the polymorphic locus at nucleotide 34,403 ("A"). In certain embodiments, the invention further provides a portion of the human HSD11B1 gene comprising one or more polymorphic loci selected from nucleotide 34,403 of SEQ ID NO: 3 and/or SEQ ID NO: 4.

In another particular embodiment, the invention provides human CHST10 nucleic acid having the nucleotide sequence of SEQ ID NOs: 5 - 12 comprising a single nucleotide polymorphism at a polymorphic locus at nucleotide position 24,707, 34,078, 35,799, 38,709, 38,947, and/or 41,180 of SEQ ID NO: 5 or SEQ ID NOs: 6 -12. The reference nucleotide for the polymorphic locus at nucleotide position 24,707 of SEQ ID NO: 5 is "G", the reference nucleotide for the polymorphic locus at nucleotide position 34,078 of SEQ ID NO: 5 is "C", the reference nucleotide for the polymorphic locus at nucleotide position 35,799 of SEQ ID NO: 5 is "G", the reference nucleotide for the polymorphic locus at nucleotide position 38,709 of SEQ ID NO: 5 is "A", the reference nucleotide for the polymorphic locus at nucleotide position 38,947 of SEQ ID NO: 5 is "C", and the reference nucleotide for the polymorphic locus at nucleotide position 41,180 of SEQ ID NO: 5 is "T".

The variant nucleotide for the polymorphic locus at nucleotide position 24,707 of SEQ ID NO: 6 or 12 is "A". The variant nucleotide for the polymorphic locus at nucleotide position 34,078 of SEQ ID NO: 7 or 12 is "T". The variant nucleotide for the polymorphic locus at nucleotide position 35,799 of SEQ ID NO: 8 or 12 is "C". The variant nucleotide for the polymorphic locus at nucleotide position 38,709 of SEQ ID NO: 9 or 12 is "G". The variant nucleotide for the polymorphic locus at nucleotide position 38,947 of SEQ ID NO: 10 or 12 is "A". The variant nucleotide for the polymorphic locus at nucleotide position 41,180 of SEQ ID NO: 11 or 12 is "C".

The nucleotide sequences of the invention can be double- or single- stranded. The nucleotide sequence(s) can comprise the complementary sequence(s) of SEQ ID NOs: 5-12. The nucleotide sequence(s) can comprise the complement of the reference nucleotide for the polymorphic locus at nucleotide 24,707 ("C"). The nucleotide sequence(s) can comprise the complement of the variant nucleotide for the polymorphic locus at nucleotide 24,707 ("T"). The nucleotide sequence(s) can comprise the complement of the reference nucleotide for the polymorphic locus at nucleotide 34,078 ("G"). The nucleotide sequence(s) can comprise the complement of the variant nucleotide for the polymorphic locus at nucleotide 34,078 ("A"). The nucleotide sequence(s) can comprise the complement of the reference nucleotide for the polymorphic locus at nucleotide 35,799 ("C"). The nucleotide sequence(s) can comprise the complement of the variant nucleotide for the polymorphic locus at nucleotide 35,799 ("G"). The nucleotide sequence(s) can comprise the complement of the reference nucleotide for the polymorphic locus at nucleotide 38,709 ("T"). The nucleotide sequence(s) can comprise the complement of the variant nucleotide for the polymorphic locus at nucleotide 38,709 ("C"). The nucleotide sequence(s) can comprise the complement of the reference nucleotide for the polymorphic locus at nucleotide 38,947 ("G"). The nucleotide sequence(s) can comprise the complement of the variant nucleotide for the polymorphic locus at nucleotide 38,947 ("T"). The nucleotide sequence(s) can comprise the complement of the reference nucleotide for the polymorphic locus at nucleotide 41,180 ("A"). The nucleotide sequence(s) can comprise the complement of the variant nucleotide for the polymorphic locus at nucleotide 41,180 ("G"). The invention further provides a portion of the human CHST10 gene comprising one or more polymorphic loci selected from nucleotide 24,707, 34,078, 35,799, 38,709, 38,947, and 41,180 of SEQ ID NO: 5 and/or SEQ ID NO: 6 - 12.

The reference allele of the single nucleotide polymorphisms described herein derived from the TEKT5, GRINL1A, HSD11B1 or CHST10 gene are shown herein to be associated with an increased likelihood of a favorable response to the administration of a therapeutically-effective amount of a DPP-IV inhibitor or a DPP-IV inhibitor in combination with other oral antidiabetic therapy. The variant allele of the single nucleotide polymorphisms of the human TEKT5, GRINL1A, HSD11B1 or CHST10 gene described herein were shown to be associated with a decrease in the likelihood of a favorable response to the administration of a therapeutically-effective amount of a DPP-IV inhibitor or a DPP-IV inhibitor in combination with other oral antidiabetic therapy.

The invention further provides allele-specific oligonucleotides that hybridize to human TEKT5, GRINL1A, HSD11B1 or CHST10 gene sequences, and fragments and complements thereof, comprising the described nucleotide polymorphisms and/or polymorphic loci. Such oligonucleotides can be designed to specifically hybridize to one polymorphic allele of the nucleic acid molecules described herein without hybridizing to other allele(s). Such oligonucleotides

can be used to determine the presence or absence of a particular allele of the polymorphic sequences described herein and to distinguish between reference and variant allele for each form. These oligonucleotides can be probes or primers, such as the probes and primers provided herein.

The described polynucleotides and oligonucleotides of the invention, as well as the corresponding methods described herein, can be used to analyze a nucleic acid isolated from an individual to identify the presence or absence of a particular nucleotide at a given polymorphic locus and to distinguish between the reference and variant allele at each locus. In certain embodiments, the method of analyzing the nucleic acid comprises determining, by means of differential hybridization detection, which base is present at nucleotide position 26,472 of SEQ ID NOs: 63 and 64 for the TEKT5 gene, determining which base is present at nucleotide position 87,992 of SEQ ID NOs: 1 and 2 for the GRINL1A gene, determining which base is present at nucleotide position 89,441 of SEQ ID NOs: 1 and 69 for the GRINL1A gene, determining which base is present at nucleotide position 89,662 of SEQ ID NOs: 1 and 70 for the GRINL1A gene, determining which base is present at nucleotide position 89,853 of SEQ ID NOs: 1 and 71 for the GRINL1A gene, determining which base is present at nucleotide position 94,074 of SEQ ID NOs: 1 and 72 for the GRINL1A gene, determining which base is present at nucleotide position 87,712 of SEQ ID NOs: 1 and 73 for the GRINL1A gene, determining which base is present at nucleotide position 93,598 of SEQ ID NOs: 1 and 74 for the GRINL1A gene, determining which base is present at nucleotide position 34,403 of SEQ ID NOs: 3 and 4 for the HSD11B1 gene, determining which base is present at nucleotide position 24,707 of SEQ ID NOs: 5 and 6 for CHST10 gene, determining which base is present at nucleotide position 34,078 of SEQ ID NOs: 5 and 7 for CHST10 gene, determining which base is present at nucleotide position 35,799 of SEQ ID NOs: 5 and 8 for CHST10 gene, determining which base is present at nucleotide position 38,709 of SEQ ID NOs: 5 and 9 for CHST10 gene, determining which base is present at nucleotide position 38,947 of SEQ ID NOs: 5 and 10 for CHST10 gene, and determining which base is present at nucleotide position 41,180 of SEQ ID NOs: 5 and 11 for CHST10 gene. Optionally, a set of nucleotides present at the polymorphic loci described herein for the TEKT5 gene, the GRINL1A gene, the HSD11B1 gene, and the CHST10 gene is determined using the oligonucleotides and methods described herein. This type of analysis can also be performed on a number of individuals, who are additionally tested (previously, concurrently or subsequently) for the existence of an increased likelihood of a favorable response to the administration of a therapeutically-effective amount of a DPP-IV inhibitor or a DPP-IV inhibitor in combination with other oral antidiabetic therapy phenotype in the presence or absence of a DPP-IV protease inhibitor. The existence of an increased likelihood of a favorable response to the administration of a therapeutically-effective amount of a DPP-IV inhibitor or a DPP-IV inhibitor in combination with other oral antidiabetic therapy phenotype is then correlated with a nucleotide (allele) or set of nucleotides present at the polymorphic locus or loci in the patient and/or sample tested.

Thus, the invention further provides methods for determining the likelihood (e.g., increased or decreased of a favorable response to the administration of a therapeutically-effective amount of a DPP-IV inhibitor or a DPP-IV inhibitor in combination with other oral antidiabetic therapy. In a particular embodiments, the method comprises obtaining a nucleic acid sample from an individual and determining the identity of nucleotides at specified polymorphic loci of the nucleic acid molecules described herein, wherein the presence of a particular nucleotide is correlated with the incidence of an increased likelihood of a favorable response to the administration of a therapeutically-effective amount of a DPP-IV inhibitor or a DPP-IV inhibitor in combination with other oral antidiabetic therapy phenotype in the presence of a DPP-IV inhibitor or a DPP-IV inhibitor in combination with other oral antidiabetic therapy, thereby determining the likelihood of a favorable response to the administration of a therapeutically-effective amount of a DPP-IV inhibitor or a DPP-IV inhibitor in combination with other oral antidiabetic therapy in the individual or sample. The correlation between a particular polymorphic form of a gene and a phenotype can thus be used in methods of diagnosis of that phenotype, as well as in the development of various treatments for the phenotype.

As used herein, the term "oligonucleotide" refers to DNA or RNA, and single- or double-stranded nucleic acid. An oligonucleotide can be used, for example, as either a "primer" or a "probe". Oligonucleotides can be naturally occurring or synthetic, but are typically prepared by synthetic means. An oligonucleotide primer, for example, can be designed to hybridize to the complementary sequence of either the sense or antisense strand of a specific target sequence, and can be used alone or as a pair, such as in DNA amplification reactions. The oligonucleotide primer may or may not comprise one or more polymorphic loci of the invention. An oligonucleotide probe can also be designed to hybridize to the complementary sequence of either the sense or antisense strand of a specific target sequence, and can be used alone or as a pair, such as in DNA amplification reactions, but necessarily will comprise one or more polymorphic loci of the invention.

Preferred oligonucleotides of the invention include fragments of DNA, and complements thereof, of the human TEKT5, GRINL1A, HSD11B1 and CHST10 gene, and can comprise a polymorphic locus selected from nucleotide position 26,472 of SEQ ID NO: 63 or 64 for the TEKT5 gene, nucleotide position 87,992 of SEQ ID NO: 1 or 2 for the GRINL1A gene, nucleotide position 89,441 of SEQ ID NO: 1 or 69 for the GRINL1A gene, nucleotide position 89,662 of SEQ ID NO: 1 or 70 for the GRINL1A gene, nucleotide position 89,853 of SEQ ID NO: 1 or 71 for the GRINL1A gene, nucleotide position 94,074 of SEQ ID NO: 1 or 72 for the GRINL1A gene, nucleotide position 87,712 of SEQ ID NO: 1

or 73 for the GRINL1A gene, nucleotide position 93,598 of SEQ ID NO: 1 or 74 for the GRINL1A gene, nucleotide position 34,403 of SEQ ID NO: 3 or 4, nucleotide position 24,707 of SEQ ID NO: 5 or 6, nucleotide position 34,078 of SEQ ID NO: 5 or 7, nucleotide position 35,799 of SEQ ID NO: 5 or 8, nucleotide position 38,709 of SEQ ID NO: 5 or 9, nucleotide position 38,947 of SEQ ID NO: 5 or 10, and nucleotide position 41,180 of SEQ ID NO: 5 or 11 for the CHST10 gene. The polymorphic locus can occur within any nucleotide position of the fragment, including at either terminal position or any internal position, including directly in the middle of the fragment. The fragments can be from any of the allelic forms of DNA shown or described herein.

As used herein, the terms "nucleotide", "base" and "nucleic acid" are intended to be equivalent. The terms "nucleotide sequence", "nucleic acid sequence", "nucleic acid molecule" , "oligonucleotide", and "nucleic acid segment" are intended to be equivalent.

Hybridization probes are oligonucleotides that bind in a base-specific manner to a complementary strand of nucleic acid and as used herein are designed to identify an allele at polymorphic loci, for example, within the TEKT5, GRINL1A, HSD11B1 and CHST10 genes described herein. Such probes can include peptide nucleic acids, as described in Nielsen et al. (1991, Science 254: 1497-1500). Probes can be any length suitable for specific hybridization to the target nucleic acid sequence. The most appropriate length of the probe varies depending upon the hybridization method in which it is being used; for example, particular lengths may be more appropriate for use in microfabricated arrays, while other lengths may be more suitable for use in classical hybridization methods. Such hybridization optimizations are known to the skilled artisan. Suitable probes can range from about 6 nucleotides to about 40 nucleotides, including about 12 nucleotides to about 25 nucleotides in length. For example, probes and primers can be about 6, 8, 10, 12, 14, 16, 18, 20, 22, 24, 25, 26, 28, 30, 32, 34, 36, 38, or about 40 nucleotides in length. The probe can comprise the sequence or complementary sequence of any of the polymorphic loci described herein, including a polymorphic locus comprising a reference allele or a variant allele. The nucleotide sequence of the probe can correspond to the coding sequence of the allele or to the complement of the coding sequence of the allele, where applicable.

Probe hybridizations are usually performed under stringency conditions having a salt concentration of no more than 1 M and a temperature of at least 25°C, depending, *inter alia,* on the length and sequence complexity of the probe. For example, conditions of 5X SSPE (750 mM NaCl, 50 mM NaPhosphate, 5 mM EDT A, pH 7.4) and a temperature of 25-30°C, or equivalent conditions, are suitable for allele-specific probe hybridizations. Equivalent conditions can be determined by varying one or more of the parameters given as an example, as known in the art, while maintaining a similar degree of identity or similarity between the target nucleotide sequence and the primer or probe used. Hybridization methods are well-known in the art.

As used herein, the term "primer" refers to a single-stranded oligonucleotide which acts as a point of initiation of template-directed DNA synthesis under appropriate conditions. Such DNA synthesis reactions can be carried out using conventional conditions in the presence of a sufficient (excess) concentration of all four different nucleoside triphosphates (e.g., in the form of phosphoramidates, for example) corresponding to adenine, guanine, cytosine and thymine or uracil nucleotides, and a polymerization catalyst, such as DNA or RNA polymerase or reverse transcriptase in an appropriate buffer and at a suitable temperature. Alternatively, such a DNA synthesis reaction may utilize only a single nucleoside (e.g., for single base-pair extension assays). The appropriate length of a primer depends on the intended use of the primer, but typically ranges from about 10 to about 30 nucleotides. Short primer molecules generally require cooler temperatures to form sufficiently stable hybrid complexes with the template. A primer need not reflect the exact sequence of the template, but must be sufficiently complementary to hybridize with a template. The term "primer site" refers to the area of the target DNA to which a primer hybridizes. The term "primer pair" refers to a set of primers including a 5' (upstream) primer that hybridizes with the 5' end of the DNA sequence to be amplified and a 3' (downstream) primer that hybridizes with the complement of the 3' end of the sequence to be amplified; generally, primer pairs do not hybridize with each other under the reaction conditions used.

As used herein, "polymorphism" refers to the occurrence of two or more genetically determined alternative sequences or alleles in a population. A "polymorphic locus" is a marker or site at which divergence from a reference allele occurs. The phrase "polymorphic loci" is meant to refer to two or more markers or sites at which divergence from two or more reference alleles occurs. Preferred markers have at least two alleles, each occurring at frequency of greater than 1%, and more preferably at a frequency greater than 10% - 20% of a selected population. A polymorphic locus can be as small as one base pair (*i.e.,* a single nucleotide polymorphism, or SNP). Polymorphic loci include, for example, restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The "reference" allele is frequently, but not always, the more common allele found in a heterogeneous population and frequently, but not always, is also the first identified allelic form. Other allelic forms are designated as "variant" alleles. The allelic form occurring most frequently in a selected population is also sometimes referred to as the "wild type" form. Diploid organisms can be homozygous or heterozygous for allelic forms. A diallelic or biallelic polymorphism has two forms. A triallelic polymorphism has three forms.

A single nucleotide polymorphism occurs at a polymorphic locus occupied by a single nucleotide, which is the

site of variation between allelic sequences. The site is usually preceded by and followed by highly conserved sequences of the allele (e.g., sequences that vary in less than 1/100 or 1/1000 members of the populations).

A single nucleotide polymorphism usually arises due to substitution of one nucleotide for another at the polymorphic locus. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele. Typically the polymorphic locus is occupied by a base other than the reference base. For example, where the reference allele contains the base "C" at the polymorphic site, the altered allele can contain a "T", "G" or "A" at the polymorphic locus.

For the purposes of this invention the terms "polymorphic position", "polymorphic site", "polymorphic locus", and "polymorphic allele" shall be construed to be equivalent and are defined as the location of a sequence identified as having more than one nucleotide represented at that location in a population comprising at least one or more individuals, and/or chromosomes.

As used herein, the term "genotype" is meant to encompass the particular alleles present at a polymorphic locus of a nucleic acid sample, a gene, and/or chromosome.

As used herein, the term "haplotype" is meant to encompass the combination of genotypes across two or more polymorphic loci of a DNA sample, a gene, and/or chromosome, wherein the genotypes are closely linked, may be inherited together as a unit, and may be in linkage disequilibrium relative to other haplotypes and/or genotypes of other DNA samples, genes, and/or chromosomes.

As used herein, the term "linkage disequilibrium" refers to a measure of the degree of association between two alleles in a population. For example, when alleles at two distinctive loci occur in a sample more frequently than expected given the known allele frequencies and recombination fraction between the two loci, the two alleles may be described as being in "linkage disequilibrium".

As used herein, the terms "genotype assay" and "genotype determination", and the phrase "to genotype" or any verb usages of the term "genotype" are intended to be equivalent and refer to assays designed to identify the allele or alleles at a particular polymorphic locus or loci in a DNA sample, a gene, and/or chromosome. Such assays can employ, for example, single base extension reactions, DNA amplification reactions that amplify across one or more polymorphic loci, or may be as simple as sequencing across one or more polymorphic loci. A number of methods are known in the art for genotyping, with many of these assays being described or referred to herein.

The invention described herein discloses whole genome scans and resequencing of the human TEKT5, GRINL1A, HSD11B1 and/or CHST10 gene in a large number of individuals to identify polymorphisms that predispose individuals to an increased likelihood of a favorable response to an administered DPP-IV inhibitor. For example, polymorphisms in the HSD11B1 and/or CHST10 gene described herein are associated with an increased likelihood of a favorable response to an administered DPP-IV inhibitor and are useful for predicting the likelihood that an individual will have such a response upon the administration of a DPP-IV inhibitor or a DPP-IV inhibitor in combination with other oral antidiabetic therapy.

By altering amino acid sequence, SNPs may alter the function of the encoded proteins. The discovery of a SNP can facilitate biochemical analysis of the variants and the development of assays to characterize the variants and to screen for pharmaceutical compounds that would interact directly with one or another form of the protein. SNPs (including silent SNPs) can also alter regulation of gene expression at the transcriptional or post- transcriptional level. SNPs (including silent SNPs) also enable the development of specific DNA, RNA, or protein-based diagnostics that detect the presence or absence of the polymorphism in particular conditions.

The phrase "DPP-IV inhibitor" is meant to encompass compounds, including, but not limited to, saxagliptin; 2-[4-{{2-(2S,5R)-2-cyano-5-ethynyl-1-pyrrolidinyl]-2-oxoethyl]amino]-4-methyl-1-piperidinyl]-4-pyridinecarboxylic acid (ABT-279); 7-But-2-ynyl-9-(6-methoxy-pyridin-3-yl)-6-piperazin-1-yl-7,9-dihydro-purin-8-one; E3024, 3-but-2-ynyl-5-methyl-2-piperazin-1-yl-3,5-dihydro-4H-imidazo[4,5-d]pyridazin-4-one tosylate; Sitagliptin; cis-2,5-dicyanopyrrolidine; 2-[3-[2-[(2S)-2-Cyano-1-pyrrolidinyl]-2-oxoethylamino]-3-methyl-1-oxobutyl]-1,2,3,4-tetrahydroisoquinoline; 2-Cyano-4-fluoro-1-thiovalylpyrrolidine analogues; KR-62436, 6-{2-[2-(5-cyano-4,5-dihydropyrazol-1-yl)-2-oxoethylamino]ethylamino}nicotinonitrile; Glutamic acid analogues; Vildagliptin ((2S)-{[(3-hydroxyadamantan-1-yl)amino]acetyl}-pyrrolidine-2-carbonitrile; 1-((S)-gamma-substituted prolyl)-(S)-2-cyanopyrrolidine; (2R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine; aminomethylpyrimidine; Gamma-amino-substituted analogues of 1-[(S)-2,4-diaminobutanoyl]piperidine; 1-[[(3-hydroxy-1-adamantyl)amino]acetyl]-2-cyano-(S)-pyrrolidine; NVP-DPP728 (1-[[[2-[(5-cyanopyridin-2-yl)amino]ethyl]amino]acetyl]-2-cyano-(S)- pyrrolidine); 1-[2-[(5-Cyanopyridin-2-yl)amino]ethylamino]acetyl-2-(S)-pyrrolidinecarbonitrile; FE 999011; alogliptin; and alogliptin benzoate, in addition to any other DPP-IV inhibitor known in the art, as well as any salt, formulation and/or combination of the same.

"Saxagliptin" refers to the compound with the chemical name (1*S,* 3*S,* 5*S*) -2 -[(2*S*)-2 -amino -2 -(3-hydroxytricyclo [3.3.1.1 $^{3,7}$] dec -1 -yl) -1- oxoethyl] -2- azabicyclo [3.1.0] hexane -3-carbonitrile or the alternative chemical name (1*S*,3*S*,5*S*)-2-[(2*S*)-2-amino-2-(3-hydroxy-1-adamantyl)-1-oxoethyl]-2-azabicyclo[3.1.0]hexane-3-carbonitrile having

the formula provided as (I) below, as well as any pharmaceutically acceptable salt of this compound, any solvate or hydrate of the compound, any solvate of a pharmaceutically acceptable salt of the compound, and any crystal form of the compound or of a pharmaceutically acceptable salt of the compound, solvate of the compound, or solvate of a salt of the compound. Saxagliptin is disclosed in U.S. Patent No. 6,395,767 (exemplified in Example 60), which is incorporated in its entirety herein.

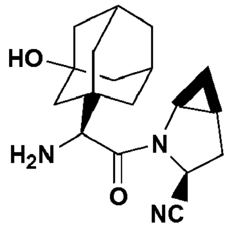

(I)

"Alogliptin" or its salt "alogliptin benzoate" is another example of a DPP-IV inhibitor or a DPP-IV inhibitor in combination with other oral antidiabetic therapy. Alogliptin benzoate" refers to the compound with the chemical name 2-[[6-[(3R)-3-amino-1-piperidinyl]-3,4-dihydro-3-methyl-2,4-dioxo-(2H)-pyrimidin-1-yl]methyl]-benzonitrile having the formula provided as (II) below, as well as any pharmaceutically acceptable salt of this compound, any solvate or hydrate of the compound, any solvate of a pharmaceutically acceptable salt of the compound, and any crystal form of the compound or of a pharmaceutically acceptable salt of the compound, solvate of the compound, or solvate of a salt of the compound. Alogliptin is disclosed in International Application No. WO2005/095381 (exemplified as compound 4), which is incorporated in its entirety herein.

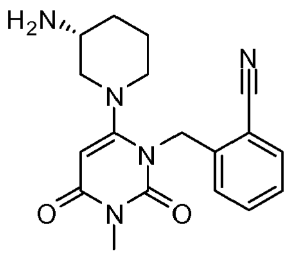

(II)

The term "isolated" is used herein to indicate that the material in question exists in a physical milieu distinct from that in which it occurs in nature, and thus is altered "by the hand of man" from its natural state.

As used herein, the term "polynucleotide" refers to a molecule comprising a nucleic acid of the invention. A polynucleotide can contain the nucleotide sequence of a full length cDNA sequence, including the 5' and 3' untranslated sequences, the coding region, with or without a signal sequence, and the secreted protein coding region, and can also contain the nucleotide sequence of the genomic sequence with or without the accompanying promoter and transcriptional termination sequences. The term "polynucleotide" also includes fragments, epitopes, domains, and variants of the cDNA and genomic nucleic acid sequences, as well as complements thereof. In specific examples, the polynucleotides of the invention include, among others, SEQ ID NOs: 1-12, 63-64, and 69-75.

A "polynucleotide" of the invention also includes those polynucleotides capable of hybridizing, under stringent hybridization conditions, to sequences described herein, or the complement(s) thereof. "Stringent hybridization conditions" refers to an overnight incubation (typically, of filters comprising DNA sequences to be hybridized to a detectable or detectably-labeled probe) at 42°C in a solution comprising 50% formamide, 5x SSC (750 mM NaCl, 75 mM trisodium citrate), 50 mM sodium phosphate (pH 7.6), 5x Denhardt's solution, 10% dextran sulfate, and 20 μg/ml denatured, sheared salmon sperm DNA, or equivalent solution, followed by washing the filters in 0.1x SSC at about 65°C.

A "polynucleotide" of the invention can be made up of any polyribonucleotide or polydeoxribonucleotide that can be unmodified RNA or DNA or modified RNA or DNA. For example, polynucleotides can be made up of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition,

the polynucleotide can be made up of triple-stranded regions comprising RNA or DNA or both RNA and DNA. A polynucleotide can also contain one or more modified bases, one or more sugar modifications, or DNA or RNA backbone modifications for stability or for other reasons. "Modified" bases include, for example, tritylated bases and atypical bases, such as inosine. A variety of modifications can be made to DNA and RNA; thus, the term "polynucleotide" embraces chemically, enzymatically, or metabolically modified forms.

As used herein, a "polypeptide" refers to a molecule having the translated amino acid sequence generated from the polynucleotide as defined.

Unless otherwise indicated, all nucleotide sequences determined by sequencing a DNA molecule herein were determined using an automated DNA sequencer (such as the Model 3730-XL or SOLiD System Sequencer from Applied Biosystems, Inc., the PE 9700 from Perkin Elmer, the GS FXL Titanium from 454 Sequencing, and/or the Genome Analyzer from Solexa), and all amino acid sequences of polypeptides encoded by DNA molecules determined herein were predicted by translation of a DNA sequence determined above. The nucleotide sequence can also be determined by other approaches including manual DNA sequencing methods well known in the art. As is also known in the art, a single insertion or deletion in a determined nucleotide sequence that is a protein-coding sequence, compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion. Since the invention provides methods for identifying single nucleotide polymorphisms whereby the novel sequence differs by as few as a single nucleotide from a reference sequence, identified SNPs were multiply verified to ensure each novel sequence represented a true SNP.

The phrase "favorable response to a DPP-IV inhibitor or a DPP-IV inhibitor in combination with other oral antidiabetic therapy" and the like, is meant to encompass a significant decrease in mean glycosylated hemoglobin (HbA1c) levels post administration of a DPP-IV inhibitor or a DPP-IV inhibitor in combination with other oral antidiabetic therapy, such as, for example, a decrease of at least about 0.5, and preferably a decrease of at least about 1.0, and more preferably a decrease of at least about 1.5 or more of HbA1c levels. The HbAlc units are reported as a standard unit, % HbA1c, as described in Colman et al., "Glycohaemoglobin- A crucial Measurement in Modern Diabetes Management. Progress Towards Standardization and Improved Precision of Measurement", Consensus Statement from the Australian Diabetes Society, Royal College of Australia Association of Clinical Biochemists, pp1-11. A favorable response to a DPP-IV inhibitor or a DPP-IV inhibitor in combination with other oral antidiabetic therapy can also be measured by measuring the level of fasting glucose or AUC glucose.

The phrase "therapeutically-effective amount of a DPP-IV inhibitor or a DPP-IV inhibitor in combination with other oral antidiabetic therapy" refers to a pharmaceutically acceptable amount of DPP-IV inhibitor administered to subjects or patients in accordance with standard medical practice for the treatment of or amelioration of symptoms arising from conditions or disorders relating to the impaired metabolism of glucose. The phrase "therapeutically-effective amount of a DPP-IV inhibitor or a DPP-IV inhibitor in combination with other oral antidiabetic therapy" also refers to that amount of DPP-IV inhibitor required to elicit a therapeutic effect in a subject or patient, such as the treatment of or amelioration of symptoms arising from conditions or disorders relating to the impaired metabolism of glucose, including, for example, but not limited to diabetes, type II diabetes, complications of diabetes, including retinopathy, neuropathy, nephropathy and delayed wound healing, diseases related to diabetes including insulin resistance, impaired glucose homeostatis, hyperglycaemia, hyperinsulinemia, elevated blood levels of fatty acids or glycerol, obesity, hyperlipidemia including hypertriglyceridemia, Syndrome X, atherosclerosis, and hypertension, among others. Therapeutically-effective amounts of various DPP-IV inhibitors are known. For example, saxagliptin can be administered at 2.5 mg, 5 mg, 10 mg, 20 mg, or 40 mg once daily, with high doses being up to 100 mg once daily (see J. Rosenstock, et al., Diabetes, Obesity and Metabolism, 10(5):376-386 (2008)).

The term "nucleotide position 26,472" is meant to refer to the allele at the polymorphic locus located at nucleotide 26,472 of SEQ ID NO: 63 and/or 64. One skilled in the art would recognize that reference to this allele is not limited to only SEQ ID NO: 63 and/or 64, but rather necessarily also includes any other polynucleotide that may include this sequence, or a portion of this sequence surrounding this polymorphic locus.

The term "nucleotide position 87,992" is meant to refer to the allele at the polymorphic locus located at nucleotide 87,992 of SEQ ID NO: 1, 2, and/or 75. One skilled in the art would recognize that reference to this allele is not limited to only SEQ ID NO: 1 and/or 2, but rather necessarily also includes any other polynucleotide that may include this sequence, or a portion of this sequence surrounding this polymorphic locus.

The term "nucleotide position 89,441" is meant to refer to the allele at the polymorphic locus located at nucleotide 89,441 of SEQ ID NO: 1, 69, and/or 75. One skilled in the art would recognize that reference to this allele is not limited to only SEQ ID NO: 1, 69, and/or 75, but rather necessarily also includes any other polynucleotide that may include this sequence, or a portion of this sequence surrounding this polymorphic locus.

The term "nucleotide position 89,662" is meant to refer to the allele at the polymorphic locus located at nucleotide 89,662 of SEQ ID NO: 1, 70, and/or 75. One skilled in the art would recognize that reference to this allele is not limited

to only SEQ ID NO: 1, 70, and/or 75, but rather necessarily also includes any other polynucleotide that may include this sequence, or a portion of this sequence surrounding this polymorphic locus.

The term "nucleotide position 89,853" is meant to refer to the allele at the polymorphic locus located at nucleotide 89,853 of SEQ ID NO: 1, 71, and/or 75. One skilled in the art would recognize that reference to this allele is not limited to only SEQ ID NO: 1, 71, and/or 75, but rather necessarily also includes any other polynucleotide that may include this sequence, or a portion of this sequence surrounding this polymorphic locus.

The term "nucleotide position 94,074" is meant to refer to the allele at the polymorphic locus located at nucleotide 94,074 of SEQ ID NO: 1, 72, and/or 75. One skilled in the art would recognize that reference to this allele is not limited to only SEQ ID NO: 1, 72, and/or 75, but rather necessarily also includes any other polynucleotide that may include this sequence, or a portion of this sequence surrounding this polymorphic locus.

The term "nucleotide position 87,712" is meant to refer to the allele at the polymorphic locus located at nucleotide 87,712 of SEQ ID NO: 1, 73, and/or 75. One skilled in the art would recognize that reference to this allele is not limited to only SEQ ID NO: 1, 73 and/or 75, but rather necessarily also includes any other polynucleotide that may include this sequence, or a portion of this sequence surrounding this polymorphic locus.

The term "nucleotide position 93,598" is meant to refer to the allele at the polymorphic locus located at nucleotide 93,598 of SEQ ID NO: 1, 74, and/or 75. One skilled in the art would recognize that reference to this allele is not limited to only SEQ ID NO: 1, 74 and/or 75, but rather necessarily also includes any other polynucleotide that may include this sequence, or a portion of this sequence surrounding this polymorphic locus.

The term "nucleotide position 34,403" is meant to refer to the allele at the polymorphic locus located at nucleotide 34,403 of SEQ ID NO: 3 and/or 4. One skilled in the art would recognize that reference to this allele is not limited to only SEQ ID NO: 3 and/or 4, but rather necessarily also includes any other polynucleotide that may include this sequence, or a portion of this sequence surrounding this polymorphic locus.

The term "nucleotide position 24,707" is meant to refer to the polymorphic locus located at nucleotide 24,707 of SEQ ID NO: 5, 6, and/or 12. One skilled in the art would recognize that reference to this allele is not limited to only SEQ ID NO: 5, 6 and/or 12, but rather necessarily also includes any other polynucleotide that may include this sequence, or a portion of this sequence surrounding this polymorphic locus.

The term "nucleotide position 34,078" is meant to refer to the polymorphic locus located at nucleotide 34,078 of SEQ ID NO: 5, 7, and/or 12. One skilled in the art would recognize that reference to this allele is not limited to only SEQ ID NO: 5, 7, and/or 12, but rather necessarily also includes any other polynucleotide that may include this sequence, or a portion of this sequence surrounding this polymorphic locus.

The term "nucleotide position 35,799" is meant to refer to the polymorphic locus located at nucleotide 35,799 of SEQ ID NO: 5, 8. and/or 12. One skilled in the art would recognize that reference to this allele is not limited to only SEQ ID NO: 5, 8, and/or 12, but rather necessarily also includes any other polynucleotide that may include this sequence, or a portion of this sequence surrounding this polymorphic locus.

The term "nucleotide position 38,709" is meant to refer to the polymorphic locus located at nucleotide 38,709 of SEQ ID NO: 5, 9, and/or 12. One skilled in the art would recognize that reference to this allele is not limited to only SEQ ID NO: 5, 9, and/or 12, but rather necessarily also includes any other polynucleotide that may include this sequence, or a portion of this sequence surrounding this polymorphic locus.

The term "nucleotide position 38,947" is meant to refer to the polymorphic locus located at nucleotide 38,947 of SEQ ID NO: 5, 10, and/or 12. One skilled in the art would recognize that reference to this allele is not limited to only SEQ ID NO: 5, 10, and/or 12, but rather necessarily also includes any other polynucleotide that may include this sequence, or a portion of this sequence surrounding this polymorphic locus.

The term "nucleotide position 41,180" is meant to refer to the polymorphic locus located at nucleotide 41,108 of SEQ ID NO: 5, 11, and/or 12. One skilled in the art would recognize that reference to this allele is not limited to only SEQ ID NO: 5, 11, and/or 12, but rather necessarily also includes any other polynucleotide that may include this sequence, or a portion of this sequence surrounding this polymorphic locus.

The term "biological sample" refers to any biological sample obtained from an organism, body fluids, cell lines, tissue culture, or other source that contains the nucleic acid, polypeptide, or mRNA of the invention. As indicated, biological samples include body fluids (such as the following non-limiting examples, sputum, amniotic fluid, urine, saliva, breast milk, secretions, interstitial fluid, blood, serum, spinal fluid, etc.) that contain the nucleic acid or polypeptide of the invention, and other tissue sources found to express the polypeptide of the invention. Methods for obtaining tissue biopsies and body fluids from organisms are well known in the art. Where the biological sample is to include mRNA, a tissue biopsy is the preferred source.

The terms "subject", "individual", "patient" and "patient population" as used herein is meant to include any eukaryotic organism, and preferably meant to encompass mammals, in particular humans.

The term "non-responder" refers to a subject, individual, patient or patient population that demonstrates little or no response to a therapeutically effective amount of a DPP-IV inhibitor or a DPP-IV inhibitor in combination with other oral antidiabetic therapy as measured by appropriate assay, including, but not limited to, those assays provided herein,

for example, by measuring the level glycosylated hemoglobin following DPP-IV treatment compared with placebo control.

The term "super-responder" refers to a subject, individual, patient or patient population that demonstrates substantial response to a therapeutically effective amount of a DPP-IV inhibitor or a DPP-IV inhibitor in combination with other oral antidiabetic therapy as measured by appropriate assay, including, but not limited to, those assays provided herein, for example, by measuring the level of glycosylated hemoglobin following DPP-IV treatment compared with placebo control.

**Polynucleotides and Polypeptides of the Invention**

**Features of TEKT SNP- Reference Allele**

The invention provides isolated nucleic acid molecules comprising all or a portion of one or more alleles of the human TEKT5 gene, as provided in SEQ ID NO: 63 (GenBank Accession No.: gi|NC_000015.8). The allele described in SEQ ID NO: 63 represents the reference allele for this SNP and is exemplified by a "T" at nucleotide position 26,472 of SEQ ID NO: 63. The reference allele described in SEQ ID NO: 1 can also be exemplified by the complementary nucleotide "A" at position 26,472. Fragments of this polynucleotide are at least about 10 nucleotides, at least about 20 nucleotides, at least about 40 nucleotides, or at least about 100 contiguous nucleotides and comprise the reference allele at the nucleotide position 26,472 in SEQ ID NO: 63.

The invention provides methods for determining whether an individual is more likely to have a favorable response to a therapeutically-effective amount of a DPP-IV inhibitor or a DPP-IV inhibitor in combination with other oral antidiabetic therapy. In certain embodiments, the methods comprise the step of identifying the nucleotide present at nucleotide position 26,472 of SEQ ID NO: 63, from a nucleic acid sample to be assessed, or the corresponding nucleotide at this position if only a fragment of the sequence provided as SEQ ID NO: 63 is assessed. The presence of the reference allele ("T" allele) at said position indicates that the individual from whom said nucleic acid sample or fragment was obtained is more likely to have a favorable response to a therapeutically-effective amount of a DPP-IV inhibitor or a DPP-IV inhibitor in combination with other oral antidiabetic therapy or DPP-IV inhibitor in combination with other oral anti-diabetic therapy (e.g., metformin, TZD, or glyburide). compared to an individual having the variant allele ("C" allele) at this position.

Importantly, the presence of the reference allele at said position in a nucleic acid sample provided by an individual indicates that said individual is more likely to have a favorable response to the administration of a correspondingly lower amount of a DPP-IV inhibitor or a DPP-IV inhibitor in combination with other oral antidiabetic therapy or DPP-IV inhibitor in combination with other oral anti-diabetic therapy relative to another individual having the variant allele(s) at said position. Therefore, such individuals may be candidates to have the level of administered DPP-IV inhibitor "titrated-down".

Representative disorders that can be detected, diagnosed, identified, treated, prevented, and/or ameliorated by analysis of the TEKT5 SNPs (e.g., SEQ ID NOs: 63 and 64 and fragments and complements thereof) using the methods of this invention include, but are not limited to, the following diseases and disorders: DPP-IV abnormalities, susceptibility to developing DPP-IV abnormalities, diabetes, disorders associated with aberrant TEKT5 expression, disorders associated with aberrant TEKT5 regulation, disorders associated with aberrant TEKT5 activity, disorders associated with aberrant HbAlc levels, disorders associated with elevated HbAlc plasma/serum levels, diabetes, type II diabetes, complications of diabetes, including retinopathy, neuropathy, nephropathy and delayed wound healing, diseases related to diabetes including insulin resistance, impaired glucose homeostatis, hyperglycaemia, hyperinsulinemia, elevated blood levels of fatty acids or glycerol, obesity, hyperlipidemia including hypertriglyceridemia, Syndrome X, atherosclerosis, and hypertension, among others.

**Features of TEKT SNP- Variant Allele**

The invention provides isolated nucleic acid molecules comprising all or a portion of one or more alleles of the human TEKT5 gene, as provided in SEQ ID NO: 64 (GenBank Accession No.: gi|NC_000015.8). The allele described in SEQ ID NO: 64 represents the variant allele for this SNP and is exemplified by a "C" at nucleotide position 26,472 of SEQ ID NO: 64. The variant allele described in SEQ ID NO: 64 can also be exemplified by the complementary nucleotide "G" at position 26,472. Fragments of this polynucleotide are at least about 10 nucleotides, at least about 20 nucleotides, at least about 40 nucleotides, or at least about 100 contiguous nucleotides and comprise the variant allele at the nucleotide position 26,472 in SEQ ID NO: 64.

The invention further provides methods for determining whether an individual is more likely to have a favorable response to the administration of a therapeutically-effective amount of a DPP-IV inhibitor or a DPP-IV inhibitor in combination with other oral antidiabetic therapy or DPP-IV inhibitor in combination with other oral anti-diabetic therapy. The inventive methods of the invention comprise the step of identifying the nucleotide present at position 26,472 of SEQ ID

NO: 64 from a nucleic acid sample to be assessed, or the corresponding nucleotide at this position if only a fragment of the sequence provided as SEQ ID NO: 64 is assessed. The presence of the variant allele ("C" allele) at nucleotide position 26,472 indicates that the individual from whom said nucleic acid sample or fragment was obtained is less likely to have a favorable response to a therapeutically-effective amount of a DPP-IV inhibitor or a DPP-IV inhibitor in combination with other oral antidiabetic therapy compared to an individual having the reference allele at said position.

Importantly, the presence of the variant allele at said position in a nucleic acid sample provided by an individual indicates that said individual is less likely to have a favorable response to a DPP-IV inhibitor or a DPP-IV inhibitor in combination with other oral antidiabetic therapy or DPP-IV inhibitor in combination with other oral anti-diabetic therapy and that the typical dose may not be sufficient relative to another individual having the reference allele at said position. In addition, the presence of the variant allele at position 26,472 of SEQ ID NO: 64 in a nucleic acid sample can indicate that a higher dose of DPP-IV inhibitor may be required to enable the subject or patient to achieve a favorable response to the administration of a therapeutically-effective amount of a DPP-IV inhibitor or a DPP-IV inhibitor in combination with other oral antidiabetic therapy.

**Features of GRINL1A SNP- Reference Allele**

The invention provides isolated nucleic acid molecules comprising all or a portion of one or more alleles of the human GRINL1A gene, as provided in SEQ ID NO: 1 (GenBank Accession No.: gi|NC_000015.8). The allele(s) described in SEQ ID NO: 1 represents the reference allele(s) for this SNP and is exemplified by a "A" at nucleotide position 87,712, "T" at nucleotide position 87,992, "A" at nucleotide position 89,441, "G" at nucleotide position 89,662, "G" at nucleotide position 89,853, "G" at nucleotide position 93,598, and "T" at nucleotide position 94,074 of SEQ ID NO: 1. The reference allele(s) described in SEQ ID NO: 1 can also be exemplified by the complementary nucleotide at each site, for example, "T" at nucleotide position 24,707, "C" at nucleotide position 34,078, "G" at nucleotide position 35,799, "A" at nucleotide position 38,709, "C" at nucleotide position 38,947 and "T" at nucleotide position 41,180 of SEQ ID NO: 1. Fragments of this polynucleotide are at least about 10 nucleotides, at least about 20 nucleotides, at least about 40 nucleotides, or at least about 100 contiguous nucleotides and comprise the reference allele at the nucleotide position(s) 87,712, 87,992, 89,441, 89,662, 89,853, 93,598, and 94,074 in SEQ ID NO: 1.

The invention provides methods for predicting whether an individual is more likely to have a favorable response to a therapeutically-effective and pharmaceutically acceptable amount of a DPP-IV inhibitor or a DPP-IV inhibitor in combination with other oral antidiabetic therapy or DPP-IV inhibitor in combination with other oral anti-diabetic therapy

. In these embodiments, the methods comprise the step of identifying the nucleotide present at nucleotide position(s) 87,712, 87,992, 89,441, 89,662, 89,853, 93,598, and 94,074 of SEQ ID NO: 1, from a nucleic acid sample to be assessed, or the corresponding nucleotide at this position if only a fragment of the sequence provided as SEQ ID NO: 1 is assessed. The presence of the reference allele(s) at said position(s) indicates that the individual from whom said nucleic acid sample or fragment is more likely to have a favorable response to a therapeutically-effective and pharmaceutically acceptable amount of a DPP-IV inhibitor or a DPP-IV inhibitor in combination with other oral antidiabetic therapy or DPP-IV inhibitor in combination with other oral anti-diabetic therapy compared with an individual having the variant allele at said position.

Importantly, the presence of the reference allele(s) at said position(s) in a nucleic acid sample provided by an individual indicates that said individual may be more likely to achieve a favorable response to the administration of a correspondingly lower amount of a DPP-IV inhibitor or a DPP-IV inhibitor in combination with other oral antidiabetic therapy or DPP-IV inhibitor in combination with other oral anti-diabetic therapy relative to another individual having the variant allele(s) at said position. Therefore, such individuals may be candidates to have the level of administered DPP-IV inhibitor "titrated-down".

Representative disorders that can be detected, diagnosed, identified, treated, prevented, and/or ameliorated by analysis of these SNPs (e.g., SEQ ID NOs: 69-77 and fragments and complements thereof) using the methods of this invention include, but are not limited to, the following diseases and disorders: DPP-IV abnormalities, susceptibility to developing DPP-IV abnormalities, diabetes, disorders associated with aberrant GRINL1A expression, disorders associated with aberrant GRINL1A regulation, disorders associated with aberrant GRINL1A activity, disorders associated with aberrant HbAlc levels, disorders associated with elevated HbAlc plasma/serum levels, diabetes, type II diabetes, complications of diabetes, including retinopathy, neuropathy, nephropathy and delayed wound healing, diseases related to diabetes including insulin resistance, impaired glucose homeostatis, hyperglycaemia, hyperinsulinemia, elevated blood levels of fatty acids or glycerol, obesity, hyperlipidemia including hypertriglyceridemia, Syndrome X, atherosclerosis, and hypertension, among others.

Features of **GRINL1A** SNP- Variant **Allele**

The invention provides isolated nucleic acid molecules comprising all or a portion of one or more alleles of the

human GRINL1A gene, as provided in SEQ ID NO: 2, and 69-75 (GenBank Accession No.: gi|NC_000015.8). The alleles described in SEQ ID NOs: 2 and 69-75 represent the variant alleles for this SNP and are exemplified by an "G" at nucleotide position 87,712 of SEQ ID NO: 73, "C" at nucleotide position 87,992 of SEQ ID NO: 2, "G" at nucleotide position 89,441 of SEQ ID NO: 69, "A" at nucleotide position 89,662 of SEQ ID NO: 70, "A" at nucleotide position 89,853 of SEQ ID NO: 71, "A" at nucleotide position 93,598 of SEQ ID NO: 74, and "C" at nucleotide position 94,074 of SEQ ID NO: 72. The variant allele(s) described in SEQ ID NOs: 2, and 69-75 can also be exemplified by the complementary nucleotide at each site, for example, "C" at nucleotide position 87,712 of SEQ ID NO: 74, "G" at nucleotide position 87,992 of SEQ ID NO: 2, "C" at nucleotide position 89,441 of SEQ ID NO: 69, "T" at nucleotide position 89,662 of SEQ ID NO: 70, "T" at nucleotide position 89,853 of SEQ ID NO: 71,"T" at nucleotide position 93,598 of SEQ ID NO: 74, and "G" at nucleotide position 94,074 of SEQ ID NO: 72. Fragments of this polynucleotide are at least about 10 nucleotides, at least about 20 nucleotides, at least about 40 nucleotides, or at least about 100 contiguous nucleotides and comprise the variant allele at the nucleotide position 87,712 in SEQ ID NO: 73, position 87,992 in SEQ ID NO: 2, position 89,441 in SEQ ID NO: 69, position 89,662 in SEQ ID NO: 70, position 89,853 in SEQ ID NO: 71, position 93,598 in SEQ ID NO: 74, and position 94,074 in SEQ ID NO: 72, or at positions 87,712, 87,992, 89,441, 89,662, 89,853, 93,598, and/or 94,074 in SEQ ID NO: 75.

The invention also provides methods for predicting the likelihood that an individual will have favorable response to the administration of a therapeutically-effective and pharmaceutically acceptable amount of a DPP-IV inhibitor or a DPP-IV inhibitor in combination with other oral antidiabetic therapy or DPP-IV inhibitor in combination with other oral anti-diabetic therapy. In these embodiments, the methods comprise the step of identifying the nucleotide present at nucleotide position 87,992 of SEQ ID NO: 2, from a nucleic acid sample to be assessed, or the corresponding nucleotide at this position if only a fragment of the sequence provided as SEQ ID NO: 2 is assessed. The presence of the variant allele ("C" allele) at said position indicates that the individual from whom said nucleic acid sample or fragment was obtained is less likely to have a favorable response to a therapeutically-effective and pharmaceutically acceptable amount of a DPP-IV inhibitor or a DPP-IV inhibitor in combination with other oral antidiabetic therapy or DPP-IV inhibitor in combination with other oral anti-diabetic therapy, compared to an individual having the reference allele at said position.

Importantly, the presence of the variant allele at said position in a nucleic acid sample provided by an individual indicates that said individual is less likely to have a favorable response to a DPP-IV inhibitor or DPP-IV inhibitor in combination with other oral anti-diabetic therapy and that the typical dose may not be sufficient relative to another individual having the reference allele at said position. In addition, the presence of the variant allele at said position in a DNA sample may indicate that a higher dose of DPP-IV inhibitor or a DPP-IV inhibitor in combination with other oral antidiabetic therapy may be required to enable the individual to achieve a favorable response to the administration of a therapeutically-effective and pharmaceutically acceptable amount of a DPP-IV inhibitor or a DPP-IV inhibitor in combination with other oral antidiabetic therapy.

Features of **HSD11B1** SNP- Reference Allele

The invention provides isolated nucleic acid molecules comprising all or a portion of one or more alleles of the human HSD11B1 gene, as provided in SEQ ID NO:3 (GenBank Accession No.: gi|NC_000001.9). The allele described in SEQ ID NO: 3 represents the reference allele for this SNP and is exemplified by a "C" at nucleotide position 34,403 of SEQ ID NO: 3. The reference allele described in SEQ ID NO: 3 can also be exemplified by the complementary nucleotide "G" at position 34,403. Fragments of this polynucleotide are at least about 10 nucleotides, at least about 20 nucleotides, at least about 40 nucleotides, or at least about 100 contiguous nucleotides and comprise the reference allele at the nucleotide position 34,403 in SEQ ID NO: 3.

The invention provides methods for predicting whether an individual will have an increased likelihood of achieving a favorable response to a therapeutically-effective and pharmaceutically acceptable amount of a DPP-IV inhibitor or a DPP-IV inhibitor in combination with other oral antidiabetic therapy. In these embodiments, the methods comprise the step of identifying the nucleotide present at nucleotide position 34,403 of SEQ ID NO: 3, from a nucleic acid sample to be assessed, or the corresponding nucleotide at this position if only a fragment of the sequence provided as SEQ ID NO: 3 is assessed. The presence of the reference allele ("C" allele) at said position indicates that the individual from whom the nucleic acid sample or fragment was obtained is more likely to have a favorable response to a therapeutically-effective and pharmaceutically acceptable amount of a DPP-IV inhibitor or a DPP-IV inhibitor in combination with other oral antidiabetic therapy compared with an individual having the variant allele at said position.

Importantly, the presence of the reference allele at said position in a nucleic acid sample provided by an individual indicates that said individual is more likely to have a favorable response to the administration of a correspondingly lower amount of a DPP-IV inhibitor or a DPP-IV inhibitor in combination with other oral antidiabetic therapy relative to another individual having the variant allele(s) at said position. Therefore, such individuals may be candidates to have the level of administered DPP-IV inhibitor "titrated-down".

Representative disorders that can be detected, diagnosed, identified, treated, prevented, and/or ameliorated

by analysis of these SNPs (e.g., SEQ ID NOs: 3 and 4 and fragments and complements thereof) using the methods of this invention include, but are not limited to, the following diseases and disorders: DPP-IV abnormalities, susceptibility to developing DPP-IV abnormalities, diabetes, disorders associated with aberrant **HSD11B1** expression, disorders associated with aberrant HSD11B1 regulation, disorders associated with aberrant **HSD11B1** activity, disorders associated with aberrant HbA1c levels, disorders associated with elevated HbAlc plasma/serum levels, diabetes, type II diabetes, complications of diabetes, including retinopathy, neuropathy, nephropathy and delayed wound healing, diseases related to diabetes including insulin resistance, impaired glucose homeostatis, hyperglycaemia, hyperinsulinemia, elevated blood levels of fatty acids or glycerol, obesity, hyperlipidemia including hypertriglyceridemia, Syndrome X, atherosclerosis, and hypertension, among others.

**Features of HSD11B1 SNP- Variant Allele**

The invention provides isolated nucleic acid molecules comprising all or a portion of one or more alleles of the human **HSD11B1** gene, as provided in SEQ ID NO:4 (GenBank Accession No.: gi|NC_000001.9). The allele described in SEQ ID NO: 4 represents the variant allele for this SNP and is exemplified by a "T" at nucleotide position 34,403 of SEQ ID NO: 4. The variant allele described in SEQ ID NO: 4 can also be exemplified by the complementary nucleotide "A" at position 34,403. Fragments of this polynucleotide are at least about 10 nucleotides, at least about 20 nucleotides, at least about 40 nucleotides, or at least about 100 contiguous nucleotides and comprise the variant allele at the nucleotide position 34,403 in SEQ ID NO: 4.

The invention also provides methods for predicting the likelihood that an individual will have a favorable response to the administration of a therapeutically-effective amount of a DPP-IV inhibitor or a DPP-IV inhibitor in combination with other oral antidiabetic therapy. In these embodiments, the methods comprise the step of identifying the nucleotide present at nucleotide position 34,403 of SEQ ID NO: 4, from a nucleic acid sample to be assessed, or the corresponding nucleotide at this position if only a fragment of the sequence provided as SEQ ID NO: 4 is assessed. The presence of the variant allele ("T" allele) at said position indicates that the individual from whom said nucleic acid sample or fragment was obtained is less likely to have a favorable response to a therapeutically-effective amount of a DPP-IV inhibitor or a DPP-IV inhibitor in combination with other oral antidiabetic therapy, compared to an individual having the reference allele at said position.

Importantly, the presence of the variant allele at said position in a nucleic acid sample provided by an individual indicates that said individual is less likely to have a favorable response to a DPP-IV inhibitor or a DPP-IV inhibitor in combination with other oral antidiabetic therapy and that the typical dose may not be sufficient relative to another individual having the reference allele at said position. In addition, the presence of the variant allele at said position in a nucleic acid sample may indicate that a higher dose of DPP-IV inhibitor may be required to enable the individual to achieve a favorable response to the administration of a therapeutically-effective amount of a DPP-IV inhibitor or a DPP-IV inhibitor in combination with other oral antidiabetic therapy.

**Features of CHST10 SNPs - Reference Allele**

The invention provides isolated nucleic acid molecules comprising all or a portion of one or more alleles of the human CHST10 gene, as provided in SEQ ID NO:5 (GenBank Accession No.: gi|NC_000002.10). The allele(s) described in SEQ ID NO: 5 represents the reference allele(s) for this SNP and is exemplified by a "G" at nucleotide position 24,707, "C" at nucleotide position 34,078, "G" at nucleotide position 35,799, "A" at nucleotide position 38,709, "C" at nucleotide position 38,947 and "T" at nucleotide position 41,180 of SEQ ID NO: 5. The reference allele(s) described in SEQ ID NO: 5 can also be exemplified by the complementary nucleotide at each site, for example, "C" at nucleotide position 24,707, "G" at nucleotide position 34,078, "C" at nucleotide position 35,799, "T" at nucleotide position 38,709, "G" at nucleotide position 38,947 and "A" at nucleotide position 41,180 of SEQ ID NO: 5. Fragments of this polynucleotide are at least about 10 nucleotides, at least about 20 nucleotides, at least about 40 nucleotides, or at least about 100 contiguous nucleotides and comprise the reference allele at the nucleotide position(s) 24,707, 34,078, 35,799, 38,709, 38,947, and 41,180 in SEQ ID NO: 5.

The invention provides methods for predicting whether an individual will have an increased likelihood of achieving a favorable response to a therapeutically-effective and pharmaceutically acceptable amount of a DPP-IV inhibitor or a DPP-IV inhibitor in combination with other oral antidiabetic therapy. In these embodiments, the methods comprise the step of identifying the nucleotide present at nucleotide position(s) 24,707, 34,078, 35,799, 38,709, 38,947, and 41,180 of SEQ ID NO: 5, from a nucleic acid sample to be assessed, or the corresponding nucleotide at this position if only a fragment of the sequence provided as SEQ ID NO: 5 is assessed. The presence of the reference allele(s) at said position(s) indicates that the individual from whom said nucleic acid sample or fragment was obtained is more likely to have a favorable response to a therapeutically-effective and pharmaceutically acceptable amount of a DPP-IV inhibitor or a DPP-IV inhibitor in combination with other oral antidiabetic therapy compared with an individual having the variant

allele at said position.

Importantly, the presence of the reference allele(s) at said position(s) in a nucleic acid sample provided by an individual indicates that said individual is more likely to have a favorable response to the administration of a correspondingly lower amount of a DPP-IV inhibitor or a DPP-IV inhibitor in combination with other oral antidiabetic therapy relative to another individual having the variant allele(s) at said position. Therefore, such individuals may be candidates to have the level of administered DPP-IV inhibitor "titrated-down".

Representative disorders that can be detected, diagnosed, identified, treated, prevented, and/or ameliorated by analysis of these SNPs (e.g., SEQ ID NOs: 5-12 and fragments and complements thereof) using the methods of this invention include, but are not limited to, the following diseases and disorders: DPP-IV abnormalities, susceptibility to developing DPP-IV abnormalities, diabetes, disorders associated with aberrant CHST10 expression, disorders associated with aberrant CHST10 regulation, disorders associated with aberrant CHST10 activity, disorders associated with aberrant HbA1c levels, disorders associated with elevated HbAlc plasma/serum levels, diabetes, type II diabetes, complications of diabetes, including retinopathy, neuropathy, nephropathy and delayed wound healing, diseases related to diabetes including insulin resistance, impaired glucose homeostatis, hyperglycaemia, hyperinsulinemia, elevated blood levels of fatty acids or glycerol, obesity, hyperlipidemia including hypertriglyceridemia, Syndrome X, atherosclerosis, and hypertension, among others.

**Features of CHST10 SNPs -Variant Alleles**

The invention provides isolated nucleic acid molecules comprising all or a portion of one or more alleles of the human CHST10 gene, as provided in SEQ ID NO: 6-11 (GenBank Accession No.: gi|NC_000002.10). The alleles described in SEQ ID NOs: 6-11 represent the variant alleles for this SNP and are exemplified by an "A" at nucleotide position 24,707 of SEQ ID NO: 6, "T" at nucleotide position 34,078 of SEQ ID NO: 7, "C" at nucleotide position 35,799 of SEQ ID NO: 8, "G" at nucleotide position 38,709 of SEQ ID NO: 9, "A" at nucleotide position 38,947 of SEQ ID NO: 10, and "C" at nucleotide position 41,180 of SEQ ID NO: 11. The variant allele(s) described in SEQ ID NOs: 6-11 can also be exemplified by the complementary nucleotide at each site, for example, "T" at nucleotide position 24,707 of SEQ ID NO: 6, "A" at nucleotide position 34,078 of SEQ ID NO: 7, "G" at nucleotide position 35,799 of SEQ ID NO: 8, "C" at nucleotide position 38,709 of SEQ ID NO: 9, "T" at nucleotide position 38,947 of SEQ ID NO: 10, and "G" at nucleotide position 41,180 of SEQ ID NO: 11. Fragments of this polynucleotide are at least about 10 nucleotides, at least about 20 nucleotides, at least about 40 nucleotides, or at least about 100 contiguous nucleotides and comprise the variant allele at the nucleotide position 24,707 in SEQ ID NO: 6, position 34,078 in SEQ ID NO: 7, position 35,799 in SEQ ID NO: 8, position 38,709 in SEQ ID NO: 9, position 38,947 in SEQ ID NO: 10 and position 41,180 in SEQ ID NO: 11, or at positions 24,707, 34,078, 35,799, 38,709, 38,947, 41,180 in SEQ ID NO: 12.

The invention also provides methods for predicting the likelihood that an individual will have favorable response to the administration of a therapeutically-effective and pharmaceutically acceptable amount of a DPP-IV inhibitor or a DPP-IV inhibitor in combination with other oral antidiabetic therapy. In these embodiments, the methods comprise the step of identifying the nucleotide present at nucleotide position 24707 of SEQ ID NO: 6, from a nucleic acid sample to be assessed, or the corresponding nucleotide at this position if only a fragment of the sequence provided as SEQ ID NO: 6 is assessed. The presence of the variant allele ("A" allele) at said position indicates that the individual from whom said nucleic acid sample or fragment was obtained has a decreased is less likely to have a favorable response to a therapeutically-effective and pharmaceutically acceptable amount of a DPP-IV inhibitor or a DPP-IV inhibitor in combination with other oral antidiabetic therapy, compared to an individual having the reference allele at said position.

Importantly, the presence of the variant allele at said position in a nucleic acid sample provided by an individual indicates that said individual is less likely to have a favorable response to a DPP-IV inhibitor or a DPP-IV inhibitor in combination with other oral antidiabetic therapy and that the typical dose may not be sufficient relative to another individual having the reference allele at said position. In addition, the presence of the variant allele at said position in a DNA sample may indicate that a higher dose of DPP-IV inhibitor or a DPP-IV inhibitor in combination with other oral antidiabetic therapy may be required to enable the individual to achieve a favorable response to the administration of a therapeutically-effective and pharmaceutically acceptable amount of a DPP-IV inhibitor or a DPP-IV inhibitor in combination with other oral antidiabetic therapy.

**Polynucleotides**

The invention provides a polynucleotide comprising the sequence identified as SEQ ID NO: 63 and/or 64 for the TEKT5 gene, SEQ ID NOs: 1, 2 and 69-75 for the GRINL1A gene, SEQ ID NO: 3 and/or 4 for the HSD11B1 gene, and SEQ ID NOs: 5-12 for the CHST10 gene; or a fragment containing the polymorphic allele, wherein said fragment comprises at least 10 contiguous nucleotides of SEQ ID NOs: 1, 2 and 69-75 for the GRINL1A gene, at least 10 contiguous nucleotides of SEQ ID NO: 3 and/or 4 for the **HSD11B1** gene, and at least 10 contiguous nucleotides of SEQ ID NOs:

5-12 for the CHST10 gene.

**TABLE I**

| Gene | Number of Polymorphic Loci | SNP | Nucleotide Position of Polymorphic Locus | Allele | Nucleotide at Polymorphic Locus | SEQ ID NO: |
|---|---|---|---|---|---|---|
| TEKT5 | 1 | SNP_A-2222802 (rs2541508) | 26472 | Reference | T | 63 |
| | | | | Variant | C | 64 |
| GRINL1A | 7 | SNP_A2276140 (rs11853270) | 87992 | Reference | T | 1 |
| | | | | Variant | C | 2 |
| | | SNP_A2276140 (rs4774281) | 89322 | Reference | A | 1 |
| | | | | Variant | G | 69 |
| | | SNP_A2276140 (rs8037000) | 89543 | Reference | G | 1 |
| | | | | Variant | A | 70 |
| | | SNP_A2276140 (rs6493943) | 89734 | Reference | G | 1 |
| | | | | Variant | A | 71 |
| | | SNP_A2276140 (rs4774282) | 93995 | Reference | G | 1 |
| | | | | Variant | C | 72 |
| | | SNP2 | 87593 | Reference | A | 1 |
| | | | | Variant | G | 73 |
| | | SNP12 | 93479 | Reference | G | 1 |
| | | | | Variant | A | 74 |
| HSD11B1 | 1 | SNP_A-4283459 (rs17015076) | 34403 | Reference | C | 3 |
| | | | | Variant | T | 4 |
| CHST10 | 6 | SNP_A-2295278 (rs1370627) | 24707 | Reference | G | 5 |
| | | | | Variant | A | 6 |
| | | SNP_A-1908426 (rs6716367) | 34078 | Reference | C | 5 |
| | | | | Variant | T | 7 |
| | | SNP_A-1872327 (rs7585898) | 35799 | Reference | G | 5 |
| | | | | Variant | C | 8 |
| | | SNP_A-1810168 (rs17024136) | 38709 | Reference | A | 5 |
| | | | | Variant | G | 9 |
| | | SNP_A-1861179 (rs17024129) | 38947 | Reference | C | 5 |
| | | | | Variant | A | 10 |
| | | SNP_A-4205974 (rs7574684) | 41180 | Reference | T | 5 |
| | | | | Variant | C | 11 |

In certain embodiments, the invention is directed to a polynucleotide comprising the sequence identified as SEQ ID NO: 63 and/or 64 for the TEKT5 gene, SEQ ID NOs: 1 and/or 2, 69-75 for the GRINL1A gene, SEQ ID NO: 3 and/or 4 for the **HSD11B1** gene, or SEQ ID NO: 5 and/or 6 - 12 for the CHST10 gene which is less than, or equal to, a polynucleotide sequence that is 5 mega basepairs, 1 mega basepairs, 0.5 mega basepairs, 0.1 mega basepairs, 50,000 basepairs, 20,000 basepairs, or 10,000 basepairs in length.

The invention further provides polynucleotides with sequences complementary to those of the polynucleotides of the invention as disclosed herein. Such sequences can be complementary to the sequence disclosed as SEQ ID NO: 63 and/or 64 for the TEKT5 gene, SEQ ID NOs: 1 and/or 2, 69-75 for the GRINL1A gene, SEQ ID NO: 3 and/or 4 for the HSD11B1 gene, and SEQ ID NO: 5 and/or 6 - 12 for the CHST10 gene.

The invention further provides fragments of polynucleotides comprising the sequence identified as SEQ ID NO: 63 and/or 64 for the TEKT5 gene, SEQ ID NOs: 1 and/or 2, 69-75 for the GRINL1A gene, SEQ ID NO: 3 and/or 4 for the HSD11B1 gene, and/or SEQ ID NO: 5 and/or 6 - 12 for the CHST10 gene, as well as the complementary sequences thereof.

The invention encompasses the application of Polymerase Chain Reaction (PCR) methodology to the polynucleotide sequences of the invention, and/or cDNA encoding the polypeptides of the invention. PCR techniques for the amplification of nucleic acids are described *inter alia* in US Patent No. 4,683,195 and Saiki et al., 1988, Science, 239:487-491. PCR, for example, can include the following steps: denaturation of template nucleic acid (if double-stranded), annealing of primer to target, and polymerization. The nucleic acid template in the amplification reaction can be genomic DNA, cDNA, RNA, or a PNA. PCR can be used to amplify specific sequences from genomic DNA, specific RNA sequences (converted to cDNA), and/or cDNA transcribed from mRNA. References for the general use of PCR techniques, including specific method parameters, include Mullis et al., 1987, Cold Spring Harbor Symp. Quant. Biol., 51:263,, Ehrlich (ed), 1989, PCR Technology, Stockton Press, NY; Ehrlich et al., 1991, Science, 252:1643-1650; and "PCR Protocols, A Guide to Methods and Applications", Eds., Innis et al., Academic Press, New York, (1990).

## Polynucleotide Variants

The invention also encompasses other sequence variants of the polynucleotide sequences described herein, e.g., SEQ ID NO: 63 and/or 64 for the TEKT5 gene, SEQ ID NOs: 1 and/or 2, 69-75 for the GRINL1A gene, SEQ ID NO: 3 and/or 4 for the **HSD11B1** gene, and/or SEQ ID NOs: 5-12 for the CHST10 gene, as well as the complementary sequences thereto and fragments thereof. The term "polynucleotide variant" (as distinguished from the term "variant allele") refers to a polynucleotide having a nucleotide sequence that shares substantial sequence homology with but is not identical with the sequence of the polynucleotide of the invention. For example, the polynucleotide variant has at least 80% sequence homology with, preferably at least 90% sequence homology, and more preferably 95% sequence homology with the sequence of the polynucleotide of the invention and retains the essential properties and characteristics thereof. Generally, the sequences of polynucleotide variants are overall closely similar, and, in many regions, identical to the sequence of the polynucleotide of the invention.

In other embodiments, the invention encompasses nucleic acid molecules that comprise a polynucleotide that hybridizes under stringent conditions, or alternatively, under lower stringency conditions, to a polynucleotide sequence of SEQ ID NOs: 1-12, 63-64, and 69-75. Polynucleotides that hybridize to the complement of these nucleic acid molecules under stringent hybridization conditions or alternatively, under lower stringency conditions, are also encompassed by the invention.

As is known in the art, typically, hybridization is said to be carried out under a) high stringency conditions if the incubation temperature of the hybridization reaction is lower than Melting Temperature (Tm) by less than or equal to 15 degree, b) moderate stringency conditions if the incubation temperature of the hybridization reaction is lower than Tm by 15 - 25 degree, and c) low stringency conditions if the incubation temperature of the hybridization reaction is lower than Tm by 25 - 35 degree. (Handout of Adv. Mol. Biol.. Sep 13, 2004, Florida State University). For washing, a higher stringency wash is typically done at 65°C in 0.1 x SSC, 0.1% SDS and a lower stringency wash is typically done at 60°C in 2 x SSC, 0.1% SDS. (Nucleic Acid Hybridization, A Practical Approach, Hames & Higgins, IPL Press, p. 97).

## Polynucleotide Fragments

The invention is directed to polynucleotide fragments of the polynucleotides of the invention, and polynucleotide sequences that hybridize thereto.

According to the invention, a "polynucleotide fragment" refers to a polynucleotide having a nucleic acid sequence that is a portion of the nucleic acid having a nucleotide sequence identified by SEQ ID NO: 63 and/or 64 for the TEKT5 gene, SEQ ID NOs: 1 and/or 2, 69-75 for the GRINL1A gene, SEQ ID NO: 3 and/or 4 for the **HSD11B1** gene and/or SEQ ID NO: 5 and/or 6 - 12 for the CHST10 gene, or the complementary strand thereto. The nucleotide fragments of the invention are at least about 15 nucleotides, at least about 20 nucleotides, at least about 30 nucleotides, at least about 40 nucleotides, at least about 50 nucleotides, at least about 75 nucleotides, or at least about 150 nucleotides in length, and comprise at least one polymorphic locus of the gene. Larger fragments (e.g., 50, 150, 500, 600, 2000 nucleotides) as described herein are also included.

A fragment "at least 20 nucleotide in length," for example, is intended to include 20 or more contiguous nucleotides from the nucleic acid sequence shown in SEQ ID NO: 63 and/or 64 for the TEKT5 gene, SEQ ID NOs: 1 and/or

2, 69-75 for the GRINL1A gene, SEQ ID NO: 3 and/or 4 for the HSD11B1 gene, and SEQ ID NO: 5 and/or 6 - 12 for the CHST10 gene, and the complementary sequences thereof. As used herein, the term "about" includes the particularly recited value, a value larger or smaller by several (5, 4, 3, 2, or 1) nucleotides, at either terminus, or at both termini. The nucleotide fragments have uses that include, but are not limited to, diagnostic probes and primers as discussed herein.

Moreover, representative examples of polynucleotide fragments of the invention, include, for example, isolated fragments comprising a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO: 63 and/or 64 for the TEKT5 gene, to the end of SEQ ID NOs: 1 and/or 2, 69-75 for the GRINL1A gene, to the end of SEQ ID NO: 3 and/or 4 for the **HSD11B1** gene, and to the end of SEQ ID NO: 5 and/or 6 - 12 for the CHST10 gene, or the complementary strand thereto. The fragments can encode a polypeptide that has biological activity. The polynucleotide fragments can be used as probes or primers as discussed herein.

Also encompassed by the invention are variant polynucleotides that hybridize to polynucleotide fragments described here under stringent hybridization conditions or lower stringency conditions, which conditions have been previously provide herein.

**Kits**

The invention provides kits that can be used in the methods described herein. For example, the invention provides kits comprising at least one reagent for identifying which allelic form (i.e., reference or variant allele) of the SNPs identified herein is present in a biological sample. In certain embodiments, the kit can be used to determine whether the biological sample contains a reference or variant nucleotide (allele) at one or more polymorphic loci, including a polymorphic locus of a TEKT5, GRINL1A, **HSD11B1** or CHST10 gene (for example, a polymorphic locus found in SEQ ID NOs: 1-12, 63-64, and 69-75). In specific embodiments, the kit can be used to determine whether the sample contains a reference or variant nucleotide (allele) at one or more of the following: nucleotide position 26,472 of SEQ ID NOs: 63 and 64 for the TEKT5 gene, nucleotide position 87,712 of SEQ ID NOs: 1, 73 and/or 75, nucleotide position 87,992 of SEQ ID NOs: 1, 2 and/or 75, nucleotide position 89,441 of SEQ ID NOs: 1, 69 and/or 77, nucleotide position 89,662 of SEQ ID NOs: 1, 70 and/or 75, nucleotide position 89,853 of SEQ ID NOs: 1, 71 and/or 75, nucleotide position 93,598 of SEQ ID NOs: 1, 74 and/or 75, and nucleotide position 94,074 of SEQ ID NOs: 1, 72 and/or 75 for the GRINL1A gene, nucleotide position 34,403 of SEQ ID NOs: 3 and 4 for the **HSD11B1** gene, and nucleotide position 24,707 of SEQ ID NOs: 5, 6 and/or 12, nucleotide position 34,078 of SEQ ID NOs: 5, 7 and/or 12, nucleotide position 35,799 of SEQ ID NOs: 5, 8 and/or 12, nucleotide position 38,709 of SEQ ID NOs: 5, 9 and/or 12, nucleotide position 38,947 of SEQ ID NOs: 5, 10 and/or 12, and nucleotide position 41,180 of SEQ ID NOs: 5, 11 and/or 12 for the CHST10 gene.

For example, kits of the invention can comprise at least one antibody specific for a particular protein or peptide encoded by one allelic form of the gene, including antibodies specific for a protein or peptide encoded by any of the polynucleotides of SEQ ID NOs: 1-12, 63-64, and 69-75.

In certain embodiments, the oligonucleotides provided in the kits hybridize to a nucleic acid sequence that spans a polymorphic locus (i.e., SNP-containing fragment) found in a human TEKT5, GRINL1A, HSD11B1, or CHST10 gene. In particular embodiments, oligonucleotides are provided that hybridize to a nucleic acid sequence that spans a polymorphic locus found in SEQ ID NOs: 1, 3, 5, or 63 including, for example, a nucleic acid sequence that spans the polymorphic locus containing a reference allele at nucleotide position 26,472 of SEQ ID NO: 63, a nucleic acid sequence(s) that spans the polymorphic locus containing a reference allele at nucleotide position(s) 87,712, 87,992, 89,441, 89,662, 89,853, 93,598, and/or 94,074 of SEQ ID NO: 1, a nucleic acid sequence that spans the polymorphic locus containing a reference allele at nucleotide position 34,403 of SEQ ID NO: 3, and a nucleic acid sequence(s) that spans the polymorphic locus containing a reference allele at nucleotide position(s) 24,707, 34,078, 35,799, 38,709, 38,947, and/or 41,180 of SEQ ID NO: 5. In certain other embodiments, oligonucleotides are provided that hybridize to a nucleic acid sequence that spans a polymorphic locus found in SEQ ID NOs: 2, 4, 6 -12, 64, or 69-75 including, for example, a nucleic acid sequence that spans the polymorphic locus containing a variant allele at nucleotide position 26,472 of SEQ ID NO: 64, a nucleic acid sequence(s) that spans the polymorphic locus containing a variant allele at nucleotide position 87,712, 87,992, 89,441, 89,662, 89,853, 93,598, 94,074 of SEQ ID NOs: 73, 2, 69, 70, 71, 74, and 72, respectively (or at one or more positions 87,712, 87,992, 89,441, 89,662, 89,853, 93,598, 94,074 of SEQ ID NO: 75), a nucleic acid sequence that spans the polymorphic locus containing a variant allele at nucleotide position 34,403 of SEQ ID NO: 4, and a nucleic acid sequence that spans the polymorphic locus containing a variant allele at nucleotide position 24,707, 34,078, 35,799, 38,709, 38,947, 41,180 of SEQ ID NOs: 6, 7, 8, 9, 10 and 11, respectively (or at one or more positions 24,707, 34,078, 35,799, 38,709, 38,947, 41,180 of SEQ ID NO: 12). In other particular embodiments, the oligonucleotides provided in the kits comprise any of SEQ ID NOs: 13-44 and 65-99..

The invention also provides kits comprising at least one reagent for amplifying an allelic form of one or more

of the SNPs identified herein. In further embodiments, the invention provides kits comprising at least one reagent for sequencing an allelic form of one or more of the SNPs identified herein. Thus, the kits can comprise one or more pairs of allele-specific oligonucleotides capable of hybridizing to different forms of a polymorphism as described herein, including, for example, oligonucleotides that hybridize to a nucleic acid sequence comprising at least one polymorphic locus, in addition to the complement of said nucleic acid sequence. These oligonucleotides can be probes or primers, for example, oligonucleotide primers used to amplify a nucleic acid sequence across a polymorphic locus and oligonucleotide primers used to sequence the amplified nucleic acid sequences.

In certain embodiments, the kits of the invention comprise a single primer or probe of the invention, providing means to detect at least one polymorphic locus, said means preferably comprising a purified primer or probe, in one or more containers. Such a primer or probe can further comprise a detectable label such as a fluorescent compound, an enzymatic substrate, a luminescent compound, a fluorophore, and/or a fluorophore linked to a terminator contained therein. Such a kit can further comprise reagents required to enable adequate hybridization of said single primer or probe to a nucleic acid test sample, such that under suitable conditions, the primer or probe is capable of binding to said nucleic acid in the test sample and signaling whether the variant or reference allele at the polymorphic locus is present in said test sample.

In other embodiments, the allele-specific oligonucleotides provided in the kits are immobilized to a substrate. Optional additional components of the kit include, for example, reagents, buffers, restriction enzymes, reverse-transcriptase or polymerase, nucleoside triphosphates, means used to label (for example, an avidin-enzyme conjugate and enzyme substrate and chromogen if the label is biotin; fluorophores, chromophores or other labels as described herein), and the appropriate buffers for reverse transcription, PCR, or hybridization reactions.

In addition, the kits of the invention can provide an assay system for carrying out the method of identifying which allelic form (i.e., reference or variant allele) of the SNPs identified herein is present in a sample. Such kits generally include a support with surface-bound oligonucleotides, and a reporter for detecting hybridization of said oligonucleotide to a test polynucleotide.

In certain embodiments, the kits comprise a solid support to which are affixed oligonucleotides comprising at least 10 contiguous nucleotides of SEQ ID NO: 63 or 64 for the TEKT5 gene, SEQ ID NO: 1, 2 or 69-75 for the GRINL1A gene, SEQ ID NO: 3 or 4 for the **HSD11B1** gene, and/or SEQ ID NO: 5 or 6 - 12 for the CHST10 gene wherein said oligonucleotide further comprises at least one polymorphic locus of SEQ ID NO: 63 or 64 for the TEKT5 gene, SEQ ID NO: 1, 2 or 69-75 for the GRINL1A gene, SEQ ID NO: 3 or 4 for the **HSD11B1** gene, and/or SEQ ID NO: 5 or 6 - 12 for the CHST10 gene. In such embodiments, a polynucleotide within a sample comprising the same or similar sequence to said oligonucleotide can be detected by hybridization.

The solid surface reagent is prepared by known techniques for attaching protein material to solid support material, such as polymeric beads, dip sticks, 96-well plate or filter material. These attachment methods generally include non-specific adsorption of the oligonucleotide to the support or covalent attachment of the oligonucleotide to a chemically reactive group on the solid support. Alternatively, streptavidin coated plates can be used in conjunction with biotinylated oligonucleotide(s).

In certain embodiments, the kits of the invention comprise means for detecting the presence of a polymorphic locus that is a specific allele of at least one polynucleotide in a nucleic acid test sample that serves as a template nucleic acid, for use in methods of the invention comprising: (a) forming an oligonucleotide bound to the polymorphic locus wherein the oligonucleotide comprises a fluorophore linked to a terminator contained therein; and (b) detecting fluorescence polarization of the fluorophore of the fluorescently-labeled oligonucleotide, wherein the oligonucleotide is formed from a primer bound to said nucleic acid sample immediately 3' to the polymorphic locus and a terminator covalently linked to a fluorophore, and wherein said terminator-linked fluorophore binds to the polymorphic locus and reacts with the primer to produce an extended primer that is said fluorescently labeled oligonucleotide, wherein an increase in fluorescence polarization indicates the presence of the specific allele at the polymorphic locus, thereby detecting the presence of the specific allele at the polymorphic locus by said increase in fluorescence polarization.

The kits of the invention can comprise the following non-limiting examples of flurophores linked to a primer or probe of the invention: 5-carboxyfluorescein (FAM-ddNTPs); 6-carboxy-X-rhodamine (ROX-ddNTPs); N,N,N',N'-tetramethyl-6-carboxyrhodamine (TMR-ddNTPs); and BODIPY-Texas Red (BTR-ddNTPs).

In any of the above-described embodiments, the kit also contains instructions for carrying out the methods.

**Methods for Using Allelic Polynucleotides of the Invention**

The determination of the polymorphic form(s) present in an individual at one or more polymorphic sites defined herein can be used in a number of methods.

Employing the various methods described herein, the polynucleotides of the invention, including allelic and variant forms thereof, can be used to determine whether an individual has a reference allele or a variant allele at one or more polymeric loci found in a TEKT5, GRINL1A, **HSD11B1** or CHST10 gene. The determination of the polymorphic

form(s) present in an individual at one or more polymorphic sites defined herein can be used in a number of diagnostic and treatment methods as discussed herein.

Further, employing the various methods described herein, the polynucleotides of the invention, including allelic and variant forms thereof, can be used to test patient populations to determine whether a certain population, such as a particular ethnic population, demonstrates an increased frequency of having either a reference allele or a variant allele at one or more polymeric loci found in a TEKT5, GRINL1A, **HSD11B1** or CHST10 gene. The determination of the polymorphic form(s) present in a certain population of individuals, such a particular ethnic population, at one or more polymorphic sites defined herein can be used in diagnostic and treatment methods adapted for that population.

The polynucleotides of the invention, including allelic and variant forms thereof, have uses that include, but are not limited to, diagnosing individuals to identify whether a given individual is more likely to have a favorable response to the administration of a therapeutically-effective amount of a DPP-IV inhibitor or a DPP-IV inhibitor in combination with other oral antidiabetic therapy. Such diagnosis is particularly useful in designing a specific treatment and dosage regimen customized and optimized for that particular individual. An optimized treatment regimen can not only improve therapeutic outcome, but can also minimize side effects.

Using the methods and polynucleotides provided herein, such diagnosis is made by determining whether the individual harbors either the reference allele or variant allele at one or more polymorphic loci of a TEKT5, GRINL1A, HSD11B1 or CHST10 gene, wherein an individual harboring the reference allele of a TEKT5, GRINL1A, HSD11B1 or CHST10 gene is more likely to have a favorable response to an administered DPP-IV inhibitor or a DPP-IV inhibitor in combination with other oral antidiabetic therapy compared to an individual harboring the variant allele of a TEKT5, GRINL1A, HSD11B1 or CHST10 gene. For example, in certain embodiments of the invention, the method for determining whether an individual is more likely to have a favorable response to the administration of a therapeutically-effective amount of a DPP-IV inhibitor or a DPP-IV inhibitor in combination with other oral antidiabetic therapy comprises the step of determining whether the individual harbors the reference thymidine allele at position 26,472 of SEQ ID NO: 63 of the TEKT5 gene. In further embodiments of the invention, said method comprises the step of determining whether the individual harbors the reference thymidine allele at position 87,992 of SEQ ID NO: 1, reference adenosine at position 89,441 of SEQ ID NO: 1, reference guanosine at position 89,662 of SEQ ID NO: 1, reference guanosine at position 89,853 of SEQ ID NO: 1, reference guanosine at position 94,074 of SEQ ID NO: 1, reference adenosine at position 87,712 of SEQ ID NO: 1, and reference guanosine at position 93,598 of SEQ ID NO: 1 of the GRINL1A gene. In further embodiments of the invention, said method comprises the step of determining whether the individual harbors the reference cytidine allele at position 34,403 of SEQ ID NO: 3 of the HSD11B1 gene. In yet further embodiments, said method comprises determining whether the individual harbors the reference guanosine allele at position 24,707 of SEQ ID NO: 5, reference cytidine at position 34,078 of SEQ ID NO: 5, reference guanosine at position 35,799 of SEQ ID NO: 5, reference adenosine at position 38,709 of SEQ ID NO: 5, reference cytidine at nucleotide position 38,947 of SEQ ID NO: 5 and reference thymidine at position 41,180 of SEQ ID NO: 5 of the CHST10 gene,

For those individuals predicted to have a lower likelihood of achieving a favorable response (i.e., those individuals having a variant allele at one or more polymorphic loci of a TEKT5, GRINL1A, HSD11B1 or CHST10 gene), an increased dosage of a DPP-IV inhibitor or a DPP-IV inhibitor in combination with other oral antidiabetic therapy may be warranted. Such a higher level of a therapeutically-effective dose of a DPP-IV inhibitor or a DPP-IV inhibitor in combination with other oral antidiabetic therapy for an individual identified as being less likely to have a favorable response can be, for example, about 5%, 10%, 15%, 20%, 25%, 30%, 35%, 40%, 45%, 50%, 55%, 60%, 65%, 75%, 80%, 85%, 90%, or 95% higher, or 1.5-, 2-, 2.5-, 3-, 3.5-, 4-, 4,5-, or even 5-fold higher than the prescribed or typical dose, as may be the case.

For those individuals predicted to be more likely to have a favorable response (i.e., those individuals having a reference allele at one or more polymorphic loci of a TEKT5, GRINL1A, HSD11B1 or CHST10 gene), the administration of a correspondingly lower amount of a DPP-IV inhibitor or a DPP-IV inhibitor in combination with other oral antidiabetic therapy relative to another individual having the variant allele(s) at said position may be warranted. Therefore, such individuals may require the level of administered DPP-IV inhibitor or a DPP-IV inhibitor in combination with other oral antidiabetic therapy to be "titrated-down" to achieve a favorable response. Such a lower level of a therapeutically-effective dose of a DPP-IV inhibitor or a DPP-IV inhibitor in combination with other oral antidiabetic therapy for an individual identified as more likely to have a favorable response can be, for example, about 5%, 10%, 15%, 20%, 25%, 30%, 35%, 40%, 45%, 50%, 55%, 60%, 65%, 75%, 80%, 85%, 90%, or 95% lower, or 1.5-, 2-, 2.5-, 3-, 3.5-, 4-, 4,5-, or even 5-fold lower than the prescribed or typical dose, as may be the case.

The same uses applied to individuals can be applied to patient populations, including particular ethnic populations.

In other embodiments, the polynucleotides and polypeptides of the invention, including allelic and variant forms thereof, either alone, or in combination with other polymorphic polynucleotides (haplotypes) are useful as genetic markers for predicting whether an individual is more likely to have a favorable response to the administration of a therapeutically-effective amount of a DPP-IV inhibitor or a DPP-IV inhibitor in combination with other oral antidiabetic therapy.

Additionally, the polynucleotides and polypeptides of the invention, including allelic and/or variant forms thereof,

are useful for creating additional antagonists directed against these polynucleotides and polypeptides, which include, but are not limited to the design of antisense RNA, ribozymes, siRNA and miRNA, PNAs, antibodies, recombinant zinc finger proteins (Wolfe et al., 2000, Structure Fold Des. 8:739-50; Kang et al., 2000, J. Biol, Chem. 275:8742-8; Wang et al., 1999, Proc. Natl. Acad. Sci. U.S.A. 96:9568-73; McColl et al., 1999, Proc. Natl. Acad. Sci. U.S.A. 96:9521-6; Segal et al., 1999, Proc. Natl. Acad. Sci. U.S.A. 96:2758-63; Wolfe et al., 1998, J. Molec. Biol. 285:1917-34; Pomerantz et al., 1998, Biochemistry 37:965-70; Leon et al., 2000, Biol. Res. 33:21-30; Berg et al., 1997, Ann. Rev. Biophys. Biomol. Struct. 26:357-71), in addition to other types of antagonists that are either described elsewhere herein, or known in the art.

The polynucleotides and polypeptides of the invention, including allelic and/or variant forms thereof, are useful for identifying small molecule antagonists directed against the variant forms of these polynucleotides and polypeptides, preferably wherein such small molecules are useful as therapeutic and/or pharmaceutical compounds for treating, detecting, prognosing, and/or preventing the following, nonlimiting diseases and/or disorders: DPP-IV abnormalities, susceptibility to developing DPP-IV abnormalities, diabetes, disorders associated with aberrant TEKT5 expression, disorders associated with aberrant TEKT5 regulation, disorders associated with aberrant TEKT5 activity, disorders associated with aberrant GRINL1A expression, disorders associated with aberrant GRINL1A regulation, disorders associated with aberrant GRINL1A activity, disorders associated with aberrant HSD11B1 expression, disorders associated with aberrant HSD11B1 regulation, disorders associated with aberrant HSD11B1 activity, disorders associated with aberrant CHST10 expression, disorders associated with aberrant CHST10 regulation, disorders associated with aberrant CHST10 activity, disorders associated with aberrant HbA1c levels, disorders associated with elevated HbAlc plasma/serum levels, diabetes, type II diabetes, complications of diabetes, including retinopathy, neuropathy, nephropathy and delayed wound healing, diseases related to diabetes including insulin resistance, impaired glucose homeostatis, hyperglycaemia, hyperinsulinemia, elevated blood levels of fatty acids or glycerol, obesity, hyperlipidemia including hypertriglyceridemia, Syndrome X, atherosclerosis, and hypertension.

Additional disorders that can be detected, diagnosed, identified, treated, prevented, and/or ameliorated by the SNPs and methods of the invention include, but are not limited to, the following diseases and disorders: diabetic related diseases such as insulin resistance, hyperglycemia, obesity, inflammation, dysmetabolic syndrome, and related diseases. Additional uses of the polynucleotides and polypeptides of the invention are provided herein.

**Haplotype Based Genetic Analysis**

The invention further provides methods for applying the polynucleotides of the invention to the elucidating haplotypes, particularly haplotypes associated with any one or more of the disease conditions referenced elsewhere herein. A "haplotype" is defined as a pattern of a set of alleles of single nucleotide polymorphisms, often along a chromosome. A non-limiting example is three single nucleotide polymorphisms (SNP1, SNP2, and SNP3) in one chromosome region, of which SNP1 is an A/G polymorphism, SNP2 is a G/C polymorphism, and SNP3 is an A/C polymorphism. A and G are the alleles for the first, G and C for the second, and A and C for the third SNP. Given two alleles for each SNP, there are three possible genotypes for individuals at each SNP. For example, for the first SNP, A/A, A/G and G/G are the possible genotypes for individuals. When an individual has a genotype for a SNP in which the alleles are not the same, for example A/G for the first SNP, then the individual is a heterozygote. When an individual has an A/G genotype at SNP1, G/C genotype at SNP2, and A/C genotype at SNP3, there are four possible combinations of haplotypes (A, B, C, and D) for this individual. The set of SNP genotypes of this individual alone would not provide sufficient information to resolve which combination of haplotypes this individual possesses. However, when this individual's parents' genotypes are available, haplotypes could then be assigned unambiguously. For example, if one parent had an A/A genotype at SNP1, a G/C genotype at SNP2, and an A/A genotype at SNP3, and the other parent had an A/G genotype at SNP1, C/C genotype at SNP2, and C/C genotype at SNP3, while the child was a heterozygote at all three SNPs, there is only one possible haplotype combination, assuming there was no crossing over in this region during meiosis.

When the genotype information of relatives is not available, haplotype assignment can be done using the long range-PCR method (Clark, 1990, Molec. Biol. Evol. 7: 111-22; Clark et al., 1998, Am J Hum Genet 63: 595-612; Fullerton et al., 2000, Am J Hum. Genet 67: 881-900; Templeton et al., 2000, Am J Hum Genet 66: 69-83). When the genotyping result of the SNPs of interest are available from general population samples, the most likely haplotypes can also be assigned using statistical methods (Excoffier & Slatkin, 1995, Mol Biol Evol 12: 921-7; Fallin & Schork, 2000, Am J Hum Genet 67: 947-59; Long et al., 1995, Am J Hum Genet 56: 799-810; Scheet, P., & Stephens, M. American Journal of Human Genetics, 78 (4), 629-644 (2006); Stephens, M., & Donnelly, P. American Journal of Human Genetics, 73 (5), 1162-1169 (2003); Zhang, S., Pakstis, A. J., Kidd, K. K., Zhao, H., Stephens, M., Smith, N. J., et al. American Journal of Human Genetics, 69(4), 906-914 (2001)).

Once an individual's haplotype in a certain chromosome region (i.e., locus) has been determined, it can be used as a tool for genetic association studies using different methods, which include, for example, haplotype relative risk analysis (Knapp et al., 1993, Am J Hum Genet 52: 1085-93; Li et al., 1998, Schizophr Res 32: 87-92; Matise, 1995, Genet Epidemiol 12: 641-5; Ott, J., 1989, Genet Epidemiol 6: 127-30; Terwilliger & Ott, 1992, Hum Hered 42: 337-46).

Haplotype based genetic analysis, using a combination of SNPs, provides increased detection sensitivity, and hence statistical significance, for genetic associations of diseases, as compared to analyses using individual SNPs as markers. Multiple SNPs present in a single gene or a continuous chromosomal region are useful for such haplotype-based analyses.

## Uses of the Polynucleotides

Each of the polynucleotides identified herein can be used in numerous ways as reagents utilizing known techniques.

Increased or decreased expression of the gene in affected organisms as compared to unaffected organisms can be assessed using polynucleotides of the invention. Any of these alterations, including altered expression, or the presence of at least one SNP of the invention within the gene, can be used as a diagnostic or prognostic marker.

The method(s) provided herein can preferably be applied in a diagnostic method and/or kits in which polynucleotides and/or polypeptides are attached to a solid support. In one exemplary method, the support may be a "gene chip" or a "biological chip" as described in U.S. Patents Nos. 5,837,832, 5,874,219, and 5,856,174. Further, such a gene chip with polynucleotides of the invention attached can be used to identify polymorphisms between the polynucleotide sequences, with polynucleotides isolated from a test subject. The knowledge of such polymorphisms (i.e. their location, as well as, their existence) would be beneficial in identifying disease loci for many disorders, including proliferative diseases and conditions. Such a method is described in U.S. Patents Nos. 5,858,659 and 5,856,104. The U.S. patents referenced supra are hereby incorporated by reference in their entirety herein.

The invention encompasses polynucleotides of the invention that are chemically synthesized or reproduced as peptide nucleic acids (PNA), or according to other methods known in the art. The use of PNAs would serve as the preferred form if the polynucleotides are incorporated onto a solid support, or gene chip. For the purposes of the invention, a peptide nucleic acid (PNA) is a polyamide type of DNA analog and the monomeric units for adenine, guanine, thymine and cytosine are available commercially (e.g., Perceptive Biosystems). Certain components of DNA, such as phosphorus, phosphorus oxides, or deoxyribose derivatives, are not present in PNAs (as disclosed by Nielsen et al., 1991, Science 254: 1497 and Egholm et al., 1993, Nature 365: 666). PNAs bind specifically and tightly to complementary DNA strands and are not degraded by nucleases. In fact, PNA binds more strongly to DNA than DNA itself does. Smaller probes can be used than with DNA due to the stronger binding characteristics of PNA: DNA hybrids. In addition, it is more likely that single base mismatches can be determined with PNA/DNA hybridization because a single mismatch in a PNA/DNA 15-mer lowers the melting point ($T_m$) by 8°-20° C, vs. 4°-16° C for the DNA/DNA 15-mer duplex. Also, the absence of charge groups in PNA means that hybridization can be done at low ionic strengths and reduce possible interference by salt during the analysis.

Such methods of using duplex oligonucleotides are known in the art and are encompassed by the invention (see EP1007712, which is hereby incorporated by reference herein in its entirety).

## EXAMPLES

<u>EXAMPLE 1 - METHODS OF GENOTYPING THE SNPs OF THE INVENTION</u>

Genomic DNA samples from patients enrolled in Bristol Myers Squibb Company clinical trials (designated as "Trial A" and "Trial B") for the DPP-IV inhibitor, Saxagliptin, were genotyped for SNPs in the human TEKT5, GRINL1A, **HSD11B1** and CHST10 genes and evaluated relative to each patients response.

Genotyping was performed using Affymetrix gene chips (Affymetrix Inc., Mountain View, CA) or the TaqMan® method (Applied Biosystems, Foster City, CA) according to the manufacturers' protocols.

TABLE II

| SNP | Forward Taqman® Primer | Reverse Taqman® Primer | Reference Probe | Variant Probe |
|---|---|---|---|---|
| **TEKT5** | AGGCTGCCAGCT<br>CCACTTC<br>(SEQ ID NO: 65) | TGCAGTGGGA<br>AATGCTTTTG<br>(SEQ ID NO: 66 | CCAGCAC**T**GGAGC<br>CTGCAGC<br>(SEQ ID NO: 67) | CCAGCAC**C**GGAG<br>CCTGCA<br>(SEQ ID NO: 68) |
| **GRINL1A (rs11853270)** | TCTGGGCACACTCC<br>CAGCT<br>(SEQ ID NO: 13) | ACTCAGGACCCA<br>AACCCAGG<br>(SEQ ID NO: 14) | CCGAGTGCGCCATC<br>TC**T**TTCTTCA<br>(SEQ ID NO: 15) | CCGAGTGCGCCATC<br>TC**C**TTCTTC<br>(SEQ ID NO: 16) |
| **GRINL1A (rs4774281)** | CAGGTCCCATGGAT<br>TGCTCT<br>(SEQ ID NO: 76) | CACAGAAGCCA<br>GCCACAGAG<br>(SEQ ID NO: 77) | CCCCACATA**T**GAAT<br>TACAAAGGAAACCA<br>A<br>(SEQ ID NO: 78) | CCCCACATA**C**GAAT<br>TACAAAGGAAACC<br>A<br>(SEQ ID NO: 79) |
| **GRINL1A (rs8037000)** | CACTTTGGGAGGCC<br>GAGTT (SEQ ID NO:<br>80) | ATGACCACCTGG<br>TTAATTTTTGTA<br>TTT (SEQ ID NO:<br>81) | TCTTGAAC**C**CCTGGC<br>CT-MGBNFQ (SEQ ID<br>NO: 82) | GTCTTGAAC**T**CCTG<br>GCCT-MGBNFQ<br>(SEQ ID NO: 83) |
| **GRINL1A (rs6493943)** | CAGAGGTTGCATTG<br>AGCTGAGAT (SEQ<br>ID NO: 84) | TATTATACAGGC<br>TTCTGACACCTC<br>CATA (SEQ ID<br>NO: 85) | CAAATCTCTCT**C**TCT<br>TGCCCA-MGBNFQ<br>(SEQ ID NO: 86) | CAAATCTCTCT**T**TC<br>TTGCCCA-MGBNFQ<br>(SEQ ID NO: 87) |
| **GRINL1A (rs4774282)** | CATAGGGAGTGGG<br>CACTGCT<br>(SEQ ID NO: 88) | GTGCTCCTTCTT<br>GGTGGCTG<br>(SEQ ID NO: 89) | CTGCTGATTGCT**G**GT<br>TGAGTCTCACC<br>(SEQ ID NO: 90) | TGCTGATTGCT**C**GT<br>TGAGTCTCACC<br>(SEQ ID NO: 91) |
| **GRINL1A (SNP2)** | TCCTACCCTTGCAG<br>GACTTTTCT (SEQ<br>ID NO: 92) | TGGAGGAGGAA<br>AAGAAGCCAT<br>(SEQ ID NO: 93) | TTCTCCACCT**A**TCTC<br>CCT-MGBNFQ (SEQ<br>ID NO: 94) | TTCTCCACCT**G**TCT<br>CCCT-MGBNFQ<br>(SEQ ID NO: 95) |

(continued)

| SNP | Forward Taqman® Primer | Reverse Taqman® Primer | Reference Probe | Variant Probe |
|---|---|---|---|---|
| **GRINL1A (SNP12)** | ACAATTCATACAAG CATCTGTAAACCA (SEQ ID NO: 96) | AAAGACAGATA GGCCCCCCA (SEQ ID NO: 97) | CTCCCCCGACACAC A-mgbnfq (SEQ ID NO: 98) | CCTCCCCTGACACA CA-mgbnfq (SEQ ID NO: 99) |
| **HSD11B1** | GCCACAAAGAGA AGCAAGGC (SEQ ID NO: 17) | TCAGAATGAAT GGGATAATTAT GGG (SEQ ID NO:18) | CACATTCCTTGAC **C**CAGAATTTTACT TCCA (SEQ ID NO:19) | CACATTCCTTGAC **T**CAGAATTTTACT TCCAGG (SEQ ID NO:20) |
| **CHST10** (rs1370627) | CATCCCCTTCCTT AACACCTGA (SEQ ID NO: 21) | TGACAGCCCAG AGGGA (SEQ ID NO: 22) | ACGGCTC**C**TAACG C (SEQ ID NO: 23) | ACGGCTC**T**TAAC GC (SEQ ID NO: 24) |
| **CHST10** (rs6716367) | AGTCCAAGGTGG CAGAAAGC (SEQ ID NO: 25) | CTCAGCCTCCA TTCCTGGTC (SEQ ID NO: 26) | CTGGCCTTA**G**CTG TCTTTCCTGGG (SEQ ID NO: 27) | CTGGCCTTA**A**CTG TCTTTCCTGGGC (SEQ ID NO: 28) |
| **CHST10** (rs7585898) | GCATGGCCACAC CCTTCTAG (SEQ ID NO: 29) | GACTCCGTCTC AAAGAAAAAA ATATATATG (SEQ ID NO: 30) | CTCTGGTCTA**G**AA GTC (SEQ ID NO: 31) | CTGGTCTA**C**AAGT CTAC (SEQ ID NO: 32) |
| **CHST10** (rs17024136) | AGTCCCTGTGGG ATGGTGCT (SEQ ID NO: 33) | CTTGTCTGCCA AGGGCCAGT (SEQ ID NO: 34) | CGGGCCACCACA**A** TTAACATCACG (SEQ ID NO: 35) | CGGGCCACCACA **G**TTAACATCACG (SEQ ID NO: 36) |
| **CHST10** (rs17024129) | CCAGGATGGTAG CTGGCATT (SEQ ID NO: 37) | CAGGGGTTTCT ACACCTAAAA CATG (SEQ ID NO: 38) | TGCCAAGACAG**C**A AAAGAACCGC (SEQ ID NO: 39) | TGCCAAGACAG**A** AAAAGAACCGCA (SEQ ID NO: 40) |

(continued)

| SNP | Forward Taqman® Primer | Reverse Taqman® Primer | Reference Probe | Variant Probe |
|---|---|---|---|---|
| **CHST10** (rs7574684) | TGATGCTTCCTCC TGTAGGTTCC (SEQ ID NO: 41) | GGAGCAGGGA ACCTCAAACC (SEQ ID NO: 42) | CCCCTGTTTA**T**GG TGCTGTTTCCCC (SEQ ID NO: 43) | CCCCTGTTTA**C**GG TGCTGTTTCCC (SEQ ID NO: 44) |
| ** The allelic nucleotide in each probe sequence is shown in bold and underlined. | | | | |

The probe sequences for each polymorphism disclosed herein were selected to provide optimal melting temperature criteria. Occasionally, a probe directed to the antisense strand was selected because the melting temperature of the antisense probe was more beneficial for the assay conditions used. Nonetheless, the present invention contemplates probe sequences directed to the opposite strand of each allele for each polymorphism. Aside from the probe sequences disclosed herein, one skilled in the art would be able to design appropriate sense and antisense probe sequences to detect which allele is present at the polymorphic locus for each polymorphism of the present invention.

EXAMPLE 2 - STATISTICAL ANALYSIS OF THE ASSOCIATION BETWEEN SAXAGLIPTIN RESPONSE AND THE SNPs OF THE INVENTION

The association between favorable DPP-IV inhibitor therapy response and the single nucleotide polymorphisms of the invention were investigated by applying statistical analysis to the results of the genotyping assays described herein. The analysis identified one or more of specific genomic factors in genomic DNA samples from index cases and control subjects who were exposed to DPP-IV inhibitor treatment in a clinical study.

There were two clinical trials, designated "Trial A" and Trial B", in which the association between the TEKT5, GRINL1A, HSD11B1 and CHST10 SNPs and the response to saxagliptin alone or in combination with other oral antidiabetic therapy was evaluated. The association between the SNPs and the response to saxagliptin was evaluated in two ways. SNPs from the genome-wide SNP panel were tested for association with response to DPP-IV inhibitors by two analytical methodologies. In one method, SNPs were identified with significant differences in mean HBA1C response among individuals with different genotypes on treatment, taking into account covariates such as demographic differences with an analysis of covariance (ANCOVA) statistical model. SNPs with consistent in their effect on HBA1C in both trials were considered for further evaluation. In a second method, SNPs were examined for association with response cluster. SNPs consistently associated with response cluster in both trials were further evaluated for influence on fasting glycosylated hemoglobin or glucose levels using analysis of covariance.

Sample: Subjects in BMS clinical trials, (Trial A and Trial B) receiving DPP-IV inhibitor (saxigliptin) therapy. All subjects have diabetes and the study group was mixed with respect to age, race and duration of diabetes.

Measures: Single nucleotide polymorphisms (SNPs) in a genome-wide panel were genotyped on all subjects essentially as described in Example 1. SNPs in human TEKT5, GRINL1A, HSD11B1 and CHST10 were found to be consistently associated with HBA1C response as described above. The SNPs that were genotyped likely represent a sample of the polymorphic variation in each gene and are not exhaustive with regard to coverage of the total genetic variation that may be present in each gene. The SNP for which a statistical association to DPP-IV inhibitor-dependent metabolic abnormalities was confirmed is provided in Table III.

Statistical Analyses: Initial analysis to determine SNP association with cluster membership was performed using the genotypic test (2-degrees of freedom) implemented in the program PLINK (Purcell et al., 2007 Amer. Journ. Hum Genet. 81: 559-75. PLINK: a tool set for whole-genome association and population-based linkage analyses). SNPs that were associated with cluster membership in the same direction (i.e. the same allele was associated with super-response) at P<= 0.001 in two independent trials of saxagliptin were selected for follow-up analysis. Follow-up analysis was performed employing a statistical package, SPSS v.14 (SPSS Inc. Chicago, I11). Some SNP associations were also discovered or confirmed using an ANCOVA modeling approach as described below. SNPs that were associated with differential genotype response were identified by a significant difference in HBA1C response (P <= 0.05) in two independent trials. Analysis was performed employing a statistical package, SAS v. 8.2 (SAS Institute Inc., Cary, NC).

Clustering: Cluster analysis was employed to identify homogeneous sub-groups that exhibited markedly different efficacy responses to saxagliptin therapy. Baseline glycosylated hemoglobin (HbA1c) and change in HbA1C after twelve weeks (study A) or twenty-four weeks (study B) of DPP-IV inhibitor therapy for each individual were used in this analysis. Individuals with similar responses to saxagliptin therapy, as measured by HbAlc levels, were grouped together. This process was iteratively repeated until all individuals were clustered into groups, either non-responders (poor response to saxagliptin therapy) or super responders (response to saxagliptin therapy). The two-step clustering routine implemented in SPSS version 12 (Chicago, Illinois, US) was used. Differences in means between clusters for HbAlc were evaluated using Kruskal-Wallis test.

ANCOVA: An analysis of covariance (ANCOVA) was employed to identify SNPs with genotypes exhibiting different efficacy responses to saxagliptin therapy in two different trials. Baseline glycosylated hemoglobin (HbA1c) and change in HbA1C after twelve or twenty-four weeks (trial-dependent) of DPP-IV inhibitor (saxagliptin) therapy for each individual were used in this analysis. The ANCOVA model provides an estimate of mean final HbA1C after treatment for different genotypes, taking into account clinical and demographic information. The general form of this model is given by the equation:

$$\text{Final HbA1c} = \beta_0 + \beta_1\ \text{baselineHbA1c} + \beta_2\ \text{baselinePlasmaGlucose} + \beta_3\ \text{Drug(0/1)} + \beta_4\ \text{Genotype} + \beta_5\ \text{Genotype*Drug},$$

where the model coefficients were estimated for each SNP and study population, and additional covariates may be included for different study populations. This may be used as an estimate of the expected final HbA1C for an individual of a particular genotype receiving DPP-IV inhibitor after 12 to 24 weeks on treatment.

The response to saxagliptin therapy for each SNP was modeled by taking into account baseline HbA1C and other baseline covariates, such as demographic variables. The SNP genotype was included in the model and the difference in HbA1C was estimated between wildtype homozygous and heterozygous or rare homozygous individuals. SNPs that were associated with differential genotype response were identified by a significant ($P <= 0.05$) difference (in the same direction) in HBA1C response between genotypes in two independent trials. Analysis was performed using contrast estimates with the GLM procedure in SAS v. 8.2 (SAS Institute Inc., Cary, NC).

The genotypes of the individuals in both groups (non-responders and super responders) were determined with respect to the polymorphic loci located at nucleotide position 26,472 of SEQ ID NO: 63 or 64 (TEKT5), nucleotide position 87,992 of SEQ ID NO: 1 or 2 (GRINL1A), nucleotide position 34,403 of SEQ ID NO: 3 or 4 (HSD11B1), nucleotide position 24,707 of SEQ ID NO: 5 or 6, nucleotide position 34,078 of SEQ ID NO: 5 or 7, nucleotide position 35,799 of SEQ ID NO: 5 or 8, nucleotide position 38,709 of SEQ ID NO: 5 or 9, nucleotide position 38,947 of SEQ ID NO: 5 or 10, and nucleotide position 41,180 of SEQ ID NO: 5 or 11 (CHST10). Specifically, for each individual in both groups, it was determined whether the individual had a reference allele or a variant allele at each SNP of the three gene sequences, for example, a reference ("T") allele or a variant ("C") allele at position 26,472 of SEQ ID NO: 63 or 642; a reference ("T") allele or a variant ("C") allele at position 87,992 of SEQ ID NO: 1 or 2; a reference ("C") allele or a variant ("T") allele at position 34,403 of SEQ ID NO: 3 or 4; a reference ("G") allele or a variant ("A") allele at position 24,707 of SEQ ID NO: 5 or 6; a reference ("C") allele or a variant ("T") allele at position 34,078 of SEQ ID NO: 5 or 7; a reference ("G") allele or a variant ("C") allele at position 35,799 of SEQ ID NO: 5 or 8; a reference ("A") allele or a variant ("G") allele at position 38,709 of SEQ ID NO: 5 or 9; a reference ("C") allele or a variant ("A") allele at position 38,947 of SEQ ID NO: 5 or 10; and a reference ("T") allele or a variant ("C") allele at position 41,180 of SEQ ID NO: 5 or 11. Genetic association between the SNPs and the non-responder and super responder clusters was assessed using Fisher's exact test.

Table III shows the results of the frequency of the variant allele in each of the three SNPs found in the non-responder and super responder cluster group populations.

Results: Two distinct subgroups of patients were observed in this trial as shown in Table 3 (the first and second rows for each SNP represent studies A and B, respectively), where the allelic frequency of the variant allele for GRINL1A, HSD11B1 and CHST10 genes is higher in the non-responder group than in the super-responder group. That is, a higher percentage of individuals in the non-responder group have the variant allele for each of the three genes than the percentage of individuals in the super-responder group. P-values for the allelic association test (1 degree of freedom) are provided for each SNP in studies A and B. These results indicate that the variant allele for GRINL1A, HSD11B1 and CHST10 is associated with a decreased likelihood of having a favorable response to the administration of a therapeutically-effective amount of a DPP-IV inhibitor or a DPP-IV inhibitor in combination with other oral antidiabetic therapy. The results from the ANCOVA approach indicate that for TEKT5, the wild-type homozygous individuals differ significantly from heterozygous in HBA1C response to administration of a therapeutically-effective amount of a DPP-IV inhibitor or a DPP-IV inhibitor in combination with other oral antidiabetic therapy.

**TABLE III**

| *Gene* | *SNP* | *Allele Frequency* | | *P value* |
|---|---|---|---|---|
| | | *Non-responders* | *Superresponders* | |
| **GRINL1A** | rs11853270 | **0.39** | **0.11** | **0.0004** |
| | | **0.35** | **0.13** | **0.001** |
| **HSD11B1** | rs17015076 | **0.25** | **0.04** | **0.0002** |
| | | **0.25** | **0.05** | **0.001** |
| **CHST10** | rs1370627 | **0.17** | **0.03** | **0.0008** |
| | | **0.20** | **0.02** | **0.001** |

Figures 1-8 show the results of SNP association with saxagliptin response when the number of super-responder and non-responder individuals having the various genotypes was determined: (1) homozygous for reference allele at

position 87,992 of SEQ ID NO: 1 or 2 (T/T); (2) heterozygous for allele at position 87,992 of SEQ ID NO: 1 or 2 (T/C); (3) homozygous for variant allele at position 87,992 of SEQ ID NO: 1 or 2 (C/C); (4) homozygous for reference allele at position 34,403 of SEQ ID NO: 3 or 4 (C/C); (5) heterozygous for allele at position 34,403 of SEQ ID NO: 3 or 4 (C/T); (6) homozygous for variant allele at position 34,403 of SEQ ID NO: 3 or 4 (T/T); (7) homozygous for reference allele at position 24,707 of SEQ ID NO: 5 or 6 (G/G); (8) heterozygous for allele at position 24,707 of SEQ ID NO: 5 or 6 (G/A); (9) homozygous for variant allele at position 24,707 of SEQ ID NO: 5 or 6 (A/A); (10) homozygous for reference allele at position 34,078 of SEQ ID NO: 5 or 7 (C/C); (11) heterozygous for allele at position 34,078 of SEQ ID NO: 5 or 7 (C/T); (12) homozygous for variant allele at position 34,078 of SEQ ID NO: 5 or 7 (T/T); (13) homozygous for reference allele at position 35,799 of SEQ ID NO: 5 or 8 (G/G); (14) heterozygous for allele at position 35,799 of SEQ ID NO: 5 or 8 (G/C); (15) homozygous for variant allele at position 35,799 of SEQ ID NO: 5 or 8 (C/C); (16) homozygous for reference allele at position 38,709 of SEQ ID NO: 5 or 9 (A/A); (17) heterozygous for allele at position 38,709 of SEQ ID NO: 5 or 9 (A/G); (18) homozygous for variant allele at position 38,709 of SEQ ID NO: 5 or 9 (G/G); (19) homozygous for reference allele at position 38,947 of SEQ ID NO: 5 or 10 (C/C); (20) heterozygous for allele at position 38,947 of SEQ ID NO: 5 or 10 (C/A); (21) homozygous for variant allele at position 38,947 of SEQ ID NO: 5 or 10 (A/A); (22) homozygous for reference allele at position 41,180 of SEQ ID NO: 5 or 11 (T/T); (23) heterozygous for allele at position 41,180 of SEQ ID NO: 5 or 11 (T/C); (24) homozygous for variant allele at position 41,180 of SEQ ID NO: 5 or 11 (C/C); (25) homozygous for reference allele at position 26,472 of SEQ ID NO: 63 or 64 (T/T); (26) heterozygous for allele at position 26,472 of SEQ ID NO: 63 or 64 (T/C); (27) homozygous for variant allele at position 26,472 of SEQ ID NO: 63 or 64 (C/C); (28) (1) homozygous for reference allele at position 87,992 of SEQ ID NO: 1 or 2 (T/T); (29) heterozygous for allele at position 87,992 of SEQ ID NO: 1 or 2 (T/C); (30) homozygous for variant allele at position 87,992 of SEQ ID NO: 1 or 2 (C/C).

Figure 1 depicts the saxagliptin response results for the GRINL1A SNP found at position 87,992 of SEQ ID NO: 1 or 2. As shown, a high percentage of individuals in the super responder group are homozygous for the reference allele (T/T) whereas a much lower percentage of individuals in the super responder group are heterozygous (T/C) and none of the individuals in the super responder group are homozygous for the variant allele (C/C). These results show a strong association between the reference allele and a favorable saxagliptin response.

Figure 2 depicts the saxagliptin response results for the HSD11B1 SNP found at position 34,403 of SEQ ID NO: 3 or 4. As shown, a high percentage of individuals in the super responder group are homozygous for the reference allele (C/C), whereas a much lower percentage of individuals in the super responder group are heterozygous (C/T) and an even lower percentage of the individuals in the super responder group are homozygous for the variant allele (T/T). These results show a strong association between the reference allele and a favorable saxagliptin response.

Figure 3 depicts the saxagliptin response results for the CHST10 SNP found at position 24,707 of SEQ ID NO: 5 or 6. As shown, a high percentage of individuals in the super responder group are homozygous for the reference allele (G/G), whereas a much lower percentage of individuals in the super responder group are heterozygous (G/A) and none of the individuals in the super responder group are homozygous for the variant allele (A/A). These results show a strong association between the reference allele and a favorable saxagliptin response.

Figure 4 depicts the saxagliptin response results for the CHST10 SNP found at position 34,078 of SEQ ID NO: 5 or 7. As shown, a high percentage of individuals in the super responder group are homozygous for the reference allele (C/C), whereas a much lower percentage of individuals in the super responder group are heterozygous (C/T) and none of the individuals in the super responder group are homozygous for the variant allele (T/T). These results show a strong association between the reference allele and a favorable saxagliptin response.

Figure 5 depicts the saxagliptin response results for the CHST10 SNP found at position 35,799 of SEQ ID NO: 5 or 8. As shown, a high percentage of individuals in the super responder group are homozygous for the reference allele (G/G), whereas a much lower percentage of individuals in the super responder group are heterozygous (G/C) and none of the individuals in the super responder group are homozygous for the variant allele (C/C). These results show a strong association between the reference allele and a favorable saxagliptin response.

Figure 6 depicts the saxagliptin response results for the CHST10 SNP found at position 38,709 of SEQ ID NO: 5 or 9. As shown, a high percentage of individuals in the super responder group are homozygous for the reference allele (A/A), whereas a much lower percentage of individuals in the super responder group are heterozygous (A/G) and none of the individuals in the super responder group are homozygous for the variant allele (G/G). These results show a strong association between the reference allele and a favorable saxagliptin response. Figure 7 depicts the saxagliptin response results for the CHST10 SNP found at position 38,947 of SEQ ID NO: 5 or 10. As shown, a high percentage of individuals in the super responder group are homozygous for the reference allele (C/C), whereas a much lower percentage of individuals in the super responder group are heterozygous (C/A) and none of the individuals in the super responder group are homozygous for the variant allele (A/A). These results show a strong association between the reference allele and a favorable saxagliptin response.

Figure 8 depicts the saxagliptin response results for the CHST10 SNP found at position 41,180 of SEQ ID NO: 5 or 11. These results show a strong association between the reference allele and a favorable saxagliptin response.

Figure 10 shows a statistical association between human GRINL1A SNP (nucleotide position 87,992 of SEQ ID NOs: 1 and 2) alleles "T" (WT, reference) and "C" (SNP carriers, variant) with a likelihood of a favorable response to the administration of a therapeutically-effective amount of a DPP-IV inhibitor. The response to DPP-IV inhibitor is assessed by measuring fasting blood glucose levels in blood. Results are shown in terms of mean change in fasting glucose levels for each genotype patient. As shown, reference carriers have a higher reduction in mean fasting glucose levels than SNP carriers in response to saxagliptin treatment.

Figure 19 depicts the saxagliptin response results for the TEKT5 SNP found at position 26,472 of SEQ ID NO: 63 or 64. The homozygous reference allele (T/T) individuals have lower mean HbA1C in response to therapy than heterozygous individuals. The difference in mean HbA1C response between T/T and T/C individuals on therapy was found to be statistically significant in both trials. These results show a strong association between individuals being homozygous for the reference allele and having a favorable saxagliptin response. Thus, individuals having the reference allele at the described SNP in the TEKT5 gene are more likely to have a favorable response to the administration of a therapeutically-effective amount of a DPP-IV inhibitor or a DPP-IV inhibitor in combination with other oral antidiabetic therapy. The ANCOVA model also provides an estimate of the mean final HbA1C for different genotypes, and may be used as an estimate of the expected final HbA1C for an individual of a particular TEKT5 genotype receiving DPP-IV inhibitor.

Figure 20 depicts the saxagliptin/metformin response results for the GRINL1A SNP found at position 87,992 of SEQ ID NO: 1 or 2. The homozygous reference allele (T/T) individuals have greater mean HbA1C glyburide-corrected change from baseline in response to therapy than heterozygous individuals. These results show a strong association between the reference allele and a favorable saxagliptin/metformin response.

Figure 21 depicts the saxagliptin/glyburide response results for the GRINL1A SNP found at position 87,992 of SEQ ID NO: 1 or 2. The homozygous reference allele (T/T) individuals have greater mean HbA1C metformin-corrected change from baseline in response to therapy than heterozygous individuals. These results show a strong association between the reference allele and a favorable saxagliptin/metformin response.

These results demonstrate that polymorphisms in the TEKT5, GRINL1A, **HSD11B1** and CHST10 genes contribute to differences in the favorability of response to saxagliptin therapy independent of other significant predictors such as age and duration of diabetes.

Further saxagliptin studies performed with individuals having either the homozygous reference allele (T/T) as compared with those carrying the variant allele (C/T or C/C)at position 87,992 of SEQ ID NO: 1 or 2 showed that differences in genotype produced significant differences in the response to saxagliptin administration as measured by the mean change in HbAlc levels following saxagliptin treatment. The results of these studies are shown in Figure 11. Specifically, the variant SNP carriers experienced little change in HbAlc levels following saxagliptin treatment as compared with individuals homozygous for the reference allele. In contrast, those subjects homozygous for the reference allele experienced a significant reduction in mean HbAlc levels following saxagliptin treatment. These results indicate that individuals with the GRINL1A reference allele show an increased therapeutic response to saxagliptin treatment compared with individuals having the GRINL1A variant allele. The significance of the differences between means of reference allele homozygotes and variant allele carriers was determined using analysis of covariance.

Other studies performed with individuals having the homozygous reference allele (T/T) as compared with those carrying the variant allele (C/T or C/C) at position 87,992 of SEQ ID NO: 1 (GRINL1A gene) showed similar results. Figure 12 shows that differences in genotype produced significant differences in the response to saxagliptin administration as measured by the mean change in fasting glucose levels following saxagliptin treatment. Specifically, the variant SNP carriers experienced little change in the fasting glucose levels following saxagliptin treatment as compared with matched placebo controls. In contrast, those subjects homozygous for the reference allele experienced a significant reduction in the mean fasting glucose levels following saxagliptin treatment as compared to carriers of the variant allele (C/T and C/C).. These results indicate that individuals homozygous for the GRINL1A reference allele show an increased therapeutic response to saxagliptin treatment compared with individuals having the GRINL1A variant allele.

Similarly, Figure 13 shows that differences in genotype produced significant differences in the response to saxagliptin administration as measured by the mean change in AUC glucose levels following saxagliptin treatment. Specifically, the variant SNP carriers experienced little change in the AUC glucose levels following saxagliptin treatment as compared with those homozygous for the reference allele.. In contrast, those homozygous for the reference allele experienced a significant reduction in the AUC glucose levels following saxagliptin treatment. These results indicate that individuals with the GRINL1A reference genotype show an increased therapeutic response to saxagliptin treatment compared with individuals having the GRINL1A variant.

The results discussed above were further confirmed by measuring DPP-IV activity in the two GRINL1A allelic subgroups in the presence or absence of DPP-IV inhibitor. The DPP-IV activity experiments showed no significant difference in plasma DPP-IV activity between the subpopulation having the homozygous reference allele and the subpopulation carrying the variant allele, suggesting that variant allele carriers do not respond to saxagliptin treatment despite inhibition in DPP-IV activity. Differences in within-group group means were significant. All pairwise between-group differences in means were significant. Other pairwise comparisons were significant.

Similarly, further saxagliptin studies performed with individuals having the homozygous reference allele as compared with those carrying the variant at position 34,403 of SEQ ID NO: 3 or 4 (HSD11B1 gene) showed that differences in genotype produced significant differences in the response to saxagliptin administration as measured by the mean change in HbAlc levels following saxagliptin treatment. The results of these studies are shown in Figure 14. Specifically, the variant SNP carriers experienced little change in HbAlc levels following saxagliptin treatment as compared those subjects homozygous for the reference allele. In contrast, the reference allele homozygotes experienced a greaterreduction in mean HbAlc levels following saxagliptin treatment as compared to carriers of the variant allele. These results indicate that individuals with the HSD11B1 reference allele show an increased therapeutic response to saxagliptin treatment compared with individuals having the HSD11B1 variant allele.

Similarly, further saxagliptin studies were performed with individuals having either the homozygous reference allele or the homozygous variant allele of the CHST10 gene. Specifically, saxagliptin studies performed in Trial A and Trial B with individuals the homozygous reference allele as compared with those carrying the variant allele at position 24,707 of SEQ ID NO: 5 or 6, position 34,078 of SEQ ID NO: 5 or 7, position 35,799 of SEQ ID NO: 5 or 8, position 38,709 of SEQ ID NO: 5 or 9, position 38,947 of SEQ ID NO: 5 or 10, or position 41,180 of SEQ ID NO: 5 or 11 (HSD11B1 gene) showed that differences in genotype produced differences in the response to saxagliptin administration as measured by the mean change in HbAlc levels following saxagliptin treatment. The results of these studies are shown in Figure 15 - 20, respectively. The variant SNP carriers experienced little change in HbAlc levels following saxagliptin treatment as compared with the reference allele homozygotes. These results indicate that individuals with the CHST10 reference allele show an increased therapeutic response to saxagliptin treatment compared with individuals having the CHST10 variant allele.

## EXAMPLE 3 - METHODS OF ISOLATING THE NATIVE FORMS OF THE GENES OF THE INVENTION (HUMAN TEKT5, GRINL1A, HSD11B1, AND CHST10)

A number of methods have been described in the art that can be utilized in isolating the native forms of the human TEKT5, GRINL1A, HSD11B1, and CHST10 genes. Rather than describe known methods here, several specific methods are referenced below and are hereby incorporated by reference herein in their entireties. The artisan, skilled in the molecular biology arts, would be able to isolate the native form of human TEKT5, GRINL1A, HSD11B1, and CHST10 genes based upon the methods and information contained, and/or referenced, therein.

For example, BAC clone RP11-100A21 is believed to contain the entire genomic sequence of GRINL1A; BAC clone RP11-117D13 is believed to contain the entire genomic sequence of HSD11B1, and BAC clone RP11-292K15 is known to contain the entire genomic sequence of CHST10, and BAC clone RP11-916G12 is known to contain the entire genomic sequence of TEKT5.

Additional methods for isolating the human TEKT5, GRINL1A,HSD11B1, and CHST10 genes can also be found in the references cited in the Genbank accession numbers for each gene provided herein that are publicly available and are also hereby incorporated by reference herein.

For example, additional methods for isolating the human GRINL1A gene can be found in the Genbank database under the accession number gi|NC_000015.8, including but not limited to, Roginski et al., Genomics 84 (2), 265-276 (2004).

Additional methods for isolating the human HSD11B1 gene can be found in the Genbank data base under the accession number gi|NC_000001.9, including but not limited to, Tannin et al., J. Biol. Chem. 266 (25), 16653-16658 (1991).

Additional methods for isolating the human CHST10 gene can be found in the Genbank data base under the accession number gi|NC_000002.10, including but not limited to, Ong et al., J. Biol. Chem. 273 (9), 5190-5195 (1998); Ong et al., J. Biol. Chem. 274 (36), 25608-25612 (1999); and/or Kang et al., J. Biol. Chem. 277 (38), 34766-34772 (2002).

## EXAMPLE 4 - METHODS OF ISOLATING POLYMORPHIC FORMS OF THE GENES OF THE INVENTION (HUMAN TEKT5, GRINL1A,HSD11B1, AND CHST10)

Since the allelic genes of the invention represent genes present within at least a subset of the human population, these genes can be isolated using the methods provided in Example 3 above. For example, the source DNA used to isolate the allelic gene can be obtained through a random sampling of the human population and repeated until the allelic form of the gene is obtained. Preferably, samples of source DNA from the human population are screened using the SNPs and methods of the invention to identify those sources that comprise the allelic form of the gene. Once identified, such a source can be used to isolate the allelic form of the gene(s). The invention encompasses the isolation of such allelic genes from both genomic and/or cDNA libraries created from such source(s).

In reference to the specific methods provided in Example 3 above, it is expected that isolating the polymorphic

alleles of the human TEKT5, GRINL1A,HSD11B1, and CHST10 genes would be within the skill of an artisan trained in the molecular biology arts. Nonetheless, a detailed exemplary method for isolating the polymorphic alleles of the human TEKT5, GRINL1A,HSD11B1, and CHST10 genes is provided. As an example, methods for isolating the variant allele ("C") at nucleotide position 87,992 of SEQ ID NO: 2 are provided. One skilled in the art would know how to adapt the methods described here to isolate other polymorphic alleles, including the variant allele ("C") at nucleotide position 26,472 of SEQ ID NO: 64 (TEKT5 gene), the variant allele ("C") at nucleotide position 87,992 of SEQ ID NO: 2, the variant allele ("G") at nucleotide position 89,441 of SEQ ID NO: 69, the variant allele ("A") at nucleotide position 89,662 of SEQ ID NO: 70, the variant allele ("A") at nucleotide position 89,853 of SEQ ID NO: 71, the variant allele ("C") at nucleotide position 94,074 of SEQ ID NO: 72, the variant allele ("G") at nucleotide position 87,712 of SEQ ID NO: 73, and the variant allele ("A") at nucleotide position 93,598 of SEQ ID NO: 74 (GRINL1A gene), the variant allele ("T") at nucleotide position 34,403 of SEQ ID NO: 4 (HSD11B1 gene) and the variant allele ("A") at nucleotide position 24,707 of SEQ ID NO: 6, the variant allele ("T") at nucleotide position 34, 078 of SEQ ID NO: 7, the variant allele ("C") at nucleotide position 35,799 of SEQ ID NO: 8, the variant allele ("G") at nucleotide position 38,709 of SEQ ID NO: 9, the variant allele ("A") at nucleotide position 38,947 of SEQ ID NO: 10, and the variant allele ("C") at nucleotide position 41,180 of SEQ ID NO: 11 (CHST10 gene).

First, the individuals with the "C" allele at the locus corresponding to nucleotide 87,992 of SEQ ID NO: 1 or 2 are identified by genotyping genomic DNA samples using the method outlined in Example 1 herein. Other methods of genotyping can be employed, such as the FP-SBE method (Chen et al., 1999, Genome Res. 9(5):492-498), or other methods described herein. DNA samples publicly available (e.g., from the Coriell Institute (Collingswood, NJ) or from the clinical samples described herein can be used. Oligonucleotide primers that are used for this genotyping assay are provided in Example 1.

By analyzing genomic DNA samples, individuals with the variant allele of the GRINL1A SNP can be identified. Once identified, clones comprising the genomic sequence can be obtained using methods well known in the art (see Sambrook et al., 1989, Molecular Cloning: A Laboratory Manual, Cold Spring Harbor Laboratory Press, Cold Spring Harbor, NY; and Current Protocols in Molecular Biology, 1995, Ausubel et al., eds, John Wiley and Sons, Inc., which are hereby incorporated by reference herein.).

If cDNA clones of the coding sequence of this allele of the gene are of interest, such clones can be obtained in accordance with the following steps. Lymphoblastoid cell lines can be obtained from the Coriell Institute. These cells can be grown in RPMI-1640 medium with L-glutamine plus 10% FCS at 37degrees. PolyA+ RNA is then isolated from these cells using Oligotex Direct Kit (Life Technologies).

First strand cDNA (complementary DNA) is produced using Superscript Preamplification System for First Strand cDNA Synthesis (Life Technologies, Cat No 18089-011) using these polyA+ RNA as templates, as specified in the users manual which is hereby incorporated herein by reference in its entirety. Specific cDNA encoding the human GRINL1A protein is amplified by polymerase chain reaction (PCR) using a forward primer that hybridizes to the 5'-UTR region, a reverse primer that hybridizes to the 3'-UTR region, and these first strand cDNA as templates (Sambrook et al., 1989, *Id*.). Alternatively, these primers can be designed using Primer3 program (Rozen, 2000, pp.365-386, Bioinformatics Methods and Protocols in Methods of Molecular Biology, S. Krawetz, S. Misener, Eds., Humana Press, Totowa, NJ). Restriction enzyme sites (example: *Sal*I for the forward primer, and *Not*I for reverse primer) are added to the 5'-end of these primer sequences to facilitate cloning into expression vectors after PCR amplification. PCR amplification can be performed essentially as described in the owner's manual of the Expand Long Template PCR System (Roche Molecular Biochemicals) following manufacturer's standard protocol, which is hereby incorporated herein by reference in its entirety.

PCR amplification products are digested with restriction enzymes (such as *Sal*I and *Not*I, for example) and ligated with expression vector DNA cut with the same set of restriction enzymes. pSPORT (Invitrogen) is one example of such an expression vector. After ligated DNA is introduced into *E. coli* cells (Sambrook et al., 1989, *Id*.), plasmid DNA is isolated from these bacterial cells. This plasmid DNA is sequenced to confirm the presence of an intact (full-length) coding region of the human GRINL1A protein with the variation, if the variation results in changes in the encoded amino acid sequence, using methods well known in the art and described elsewhere herein.

The skilled artisan would appreciate that the above method can be applied to isolating other novel human GRINL1A genes through the simple substitution of applicable PCR and sequencing primers. Such primers can be selected from any one of the applicable primers provided in herein, or can be designed using the Primer3 program (Rozen S 2000) as described. Such primers can preferably comprise at least a portion of any one of the polynucleotide sequences of the invention.

## EXAMPLE 5 - METHOD OF ENGINEERING THE ALLELIC FORMS OF THE GENES OF THE INVENTION (HUMAN TEKT5, GRINL1A,HSD11B1, AND CHST10)

Aside from isolating the allelic genes of the present invention from DNA samples obtained from the human population, as described in Example 4 above, the invention also encompasses methods of engineering the allelic genes

of the present invention through the application of site-directed mutagenesis to the isolated native forms of the genes. Such methodology can be applied to synthesize allelic forms of the genes comprising at least one, or more, of the encoding SNPs of the present invention (e.g., silent, missense) - preferably at least 1, 2, 3, or 4 encoding SNPs for each gene.

In reference to the specific methods provided in Example 4 above, it is expected that isolating the novel polymorphic TEKT5, GRINL1A, HSD11B1, and CHST10 genes of the present invention would be within the ordinary skill of an artisan trained in the molecular biology arts. Nonetheless, a detailed exemplary method of engineering the polymorphic alleles to comprise the encoding and/or non-coding polymorphic nucleic acid sequence is provided. As an example, methods for engineering the variant allele ("C") at nucleotide position 87,992 of SEQ ID NO: 2, is provided. One skilled in the art would know how to adapt the methods described here to engineer other alleles, including the variant allele ("C") at nucleotide position 26,472 of SEQ ID NO: 64 (TEKT5 gene), the variant allele ("C") at nucleotide position 87,992 of SEQ ID NO: 2 (GRINL1A gene), the variant allele ("G") at nucleotide position 89,441 of SEQ ID NO: 69 (GRINL1A gene), the variant allele ("A") at nucleotide position 89,662 of SEQ ID NO: 70 (GRINL1A gene), the variant allele ("A") at nucleotide position 89,853 of SEQ ID NO: 71 (GRINL1A gene), the variant allele ("C") at nucleotide position 94,074 of SEQ ID NO: 72 (GRINL1A gene), the variant allele ("G") at nucleotide position 87,712 of SEQ ID NO: 73 (GRINL1A gene), and the variant allele ("A") at nucleotide position 93,598 of SEQ ID NO: 74 (GRINL1A gene),the variant allele ("T") at nucleotide position 34,403 of SEQ ID NO: 4 (HSD11B1 gene), the variant allele ("A") at nucleotide position 24,707 of SEQ ID NO:6 (CHST10 gene), the variant allele ("T") at nucleotide position 34,078 of SEQ ID NO:7 (CHST10 gene), the variant allele ("C") at nucleotide position 35,799 of SEQ ID NO:8 (CHST10 gene), the variant allele ("G") at nucleotide position 38,709 of SEQ ID NO:9 (CHST10 gene), the variant allele ("A") at nucleotide position 38,947 of SEQ ID NO:10 (CHST10 gene), and the variant allele ("C") at nucleotide position 41,180 of SEQ ID NO:11 (CHST10 gene).

Briefly, genomic clones containing the human GRINL1A gene can be identified by homology searches with the BLASTN program (Altschul, SF et al., 1990, J. Mol. Biol. 215: 403-410) against the Genbank non-redundant nucleotide sequence database using the published human GRINL cDNA sequence (GenBank Accession No.: gi|NC_000015.8), for example, BAC RP11-100A21. Alternatively, the genomic sequence of the human GRINL1A gene can be obtained as described herein. After obtaining these clones, they are sequenced to confirm the validity of the DNA sequences.

Similarly, genomic clones containing the human TEKT5 gene can be identified by homology searches with the BLASTN program (Altschul, SF et al., 1990, J. Mol. Biol. 215: 403-410) against the Genbank non-redundant nucleotide sequence database using the published human TEKT5 cDNA sequence (GenBank Accession No.: gi|NM_144674.1), for example, BAC RP11-916G12. Alternatively, the genomic sequence of the human TEKT5 gene can be obtained as described herein. After obtaining these clones, they are sequenced to confirm the validity of the DNA sequences.

Similarly, genomic clones containing the human HSD11B1 gene can be identified by homology searches with the BLASTN program (Altschul, SF et al., 1990, J. Mol. Biol. 215: 403-410) against the Genbank non-redundant nucleotide sequence database using the published human HSD11B1 cDNA sequence (GenBank Accession No.: gi|NC_000001.9), for example, BAC RP11-117D13. Alternatively, the genomic sequence of the human HSD11B1 gene can be obtained as described herein. After obtaining these clones, they are sequenced to confirm the validity of the DNA sequences.

Similarly, genomic clones containing the human CHST10 gene can be identified by homology searches with the BLASTN program (Altschul, SF et al., 1990, J. Mol. Biol. 215: 403-410) against the Genbank non-redundant nucleotide sequence database using the published human CHST10 cDNA sequence (GenBank Accession No.: gi|NC_000002.10), for example, BAC RP11-292K15. Alternatively, the genomic sequence of the human CHST10 gene can be obtained as described herein. After obtaining these clones, they are sequenced to confirm the validity of the DNA sequences.

However, in the case of the variant alleles, genomic clones would need to be obtained and can be identified by homology searches with the BLASTN program (Altschul SF, 1990, *Id*.) against the Genbank non-redundant nucleotide sequence database using the published human GRINL1A genomic sequence (GenBank Accession No.: NC_000015.8), using the published human TEKT5 genomic sequence (GenBank Accession No.: NM_144674), using the published human GRINL1A genomic sequence (GenBank Accession No.: NM_001018090), using the published human HSD11B1 genomic sequence (GenBank Accession No.: NC_000001.9), or using the published human CHST10 genomic sequence (GenBank Accession No.: NC_000002.10). Alternatively, the genomic sequence of the human HSD11B1, or CHST10 gene can be obtained as described herein. After obtaining these clones, they are sequenced to confirm the validity of the DNA sequences.

Once these clones are confirmed to contain the intact wild type cDNA or genomic sequence of the human TEKT5, GRINL1A, HSD11B1, or CHST10 coding and/or non-coding region, the variant polymorphism (mutation) can be introduced into the native sequence using PCR directed *in vitro* mutagenesis (Cormack, B., Directed Mutagenesis Using the Polymerase Chain Reaction. Current Protocols in Molecular Biology, John Wiley & Sons, Inc. Supplement 37: 8.5.1-8.5.10, (2000)). In this method, synthetic oligonucleotides are designed to incorporate a point mutation at one end of an amplified fragment. Following polymerase chain reaction (PCR), the amplified fragments are made blunt-ended by treatment with Klenow Fragment. These fragments are then ligated and subcloned into a vector to facilitate sequence analysis. This method consists of the following steps.

1. Subcloning of cDNA or genomic insert into a plasmid vector, or BAC sequence if the clone is a genomic sequence, containing multiple cloning sites and M13 flanking sequences, such as pUC19 (Sambrook et al., 1989, *Id.*), in the forward orientation. The skilled artisan would appreciate that other plasmids could be equally substituted, and may be desirable in certain circumstances.
2. Introduction of a mutation by PCR amplification of the genomic region downstream of the mutation site using a primer including the mutation. (Figure 8.5.2 in Cormack, 2000, *Id.*)). In the case of introducing the variant mutation(s) into the human TEKT5, GRINL1A, HSD11B1, or CHST10 genomic sequence, the following M13 reverse primer in addition to one of the below gene mutation primers can be used.
M13 reverse sequencing primer:

5'-AGCGGATAACAATTTCACACAGGA-3' (SEQ ID NO: 45).

GRINL1A rs11853270 Mutation primer:

5'- CATGGCCGAGTGCGCCATCTC**C**TTCTTCAAAGTCTCTCCTCTTAGG -3' (SEQ ID NO: 46)

HSD11B1 rs17015076 Mutation primer:

5'- GGGATACACATTCCTTGACTCAGAATTTTACTTCCAGGAATCCTG-3' (SEQ ID NO: 47)

CHST10 (rs1370627) Mutation primer:

5'- GGAAGCATCACACAGCGTTAAGAGCCGTTTCCTTCAGGTGTTAAGG-3' (SEQ ID NO: 48)

CHST10 (rs6716367) Mutation primer:

5'- GATAATGTAGAACTGGCCTTAACTGTCTTTCCTGGGCTCTGG-3' (SEQ ID NO: 49)

CHST10 (rs7585898) Mutation primer:

5'- GATCAAAAGCTGTAGACTT**G**TAGACCAGAGTCTAGAAGGG-3' (SEQ ID NO: 50)

CHST10 (rs17024136) Mutation primer:

5'- GACAGTGATCGTGATGTTAACTGTGGTGGCCCGTGTTCATAGC-3' (SEQ ID NO: 51)

CHST10 (rs17024129) Mutation primer:

5'- CATGTCATTGCGGTTCTTTTTCTGTCTTGGCATATGATTAATTAC-3' (SEQ ID NO: 52)

CHST10 (rs7574684) Mutation primer:

5'- CTTGAGGGGAAACAGCACC**G**TAAACAGGGGCAGCAAGGCC-3' (SEQ ID NO: 53)

TEKT5 Mutation primer:

5'- CAGTAGCTTGGCTGCAGGCTCC**A**GTGCTGGAAGGAATCGGCTG -3' (SEQ ID NO: 100)

GRINL1A (SNP2) Mutation primer:

5'-CTTTTCTCTCCTGGTTCTCCACCT**A**TCTCCCTGGTCTGTCCTTCTCC-3' (SEQ ID NO: 101)

GRINL1A (rs4774281) Mutation primer:

5'-CCTTTGGTTTCCTTTGTAATTC**G**TATGTGGGGTAATAGCTCTGTGG-3' (SEQ ID NO: 102)

GRINL1A (rs8037000) Mutation primer:

5'-GCGGATCATTTGAGGCCAGGGGTTCAAGACCAGACTGGCCC-3' (SEQ ID NO:103)

GRINL1A (rs6493943) Mutation primer:

5'-GCACTCCAGCCTGGGCAAGA**<u>G</u>**AGAGAGATTTGGTCTCG -3' (SEQ ID NO: 104)

GRINL1A (SNP12) Mutation primer:

5'-CCATAAGATTGACTGTGTGTGTC**<u>G</u>**GGGGAGGGTGGGGGGCCTATC-3' (SEQ ID NO: 105)

GRINL1A (rs4774282) Mutation primer:

5'-GGTTTTAGAGCACTGCTGATTGCT**<u>G</u>**GTTGAGTCTCACCACTGGAGG-3' (SEQ ID NO: 106)

Mutation primer contains the mutation variant nucleotide at the 5' end (in bold and underlined) and a portion of its flanking sequence. M13 reverse sequencing primer hybridizes to the pUC19 vector. Subcloned cDNA or genomic clone comprising the human GRINL1A cDNA or genomic sequence is used as a template (described in Step 1). A 100 ul PCR reaction mixture is prepared using 10ng of the template DNA, 200 uM 4dNTPs, 1uM primers, 0.25U Taq DNA polymerase (PE), and standard Taq DNA polymerase buffer. Typical PCR cycling conditions are as follows:

| | |
|---|---|
| 20-25 cycles: | 45 sec, 93 degrees |
| | 2 min, 50 degrees |
| | 2 min, 72 degrees |
| 1 cycle: | 10 min, 72 degrees |

After the final extension step of PCR, 5U Klenow Fragment is added and incubated for 15 minutes at 30 degrees. The PCR product is then digested with the restriction enzyme, EcoRI.

3. PCR amplification of the upstream region is then performed, using subcloned cDNA or genomic clone as a template (the product of Step 1). This PCR is done using the M13 forward primer in conjunction with one of the following gene flanking primers:

M13 forward sequencing primer:

5'-CGCCAGGGTTTTCCCAGTCACGAC-3' (SEQ ID NO: 54).

GRINL1A rs11853270 Flanking primer:

5'- CCTAAGAGGAGAGACTTTGAAGAA**<u>G</u>**GAGATGGCGCACTCGGCCATG - 3' (SEQ ID NO: 55).

HSD11B1 rs17015076 Flanking primer:

5'- CAGGATTCCTGGAAGTAAAATTCTG**<u>A</u>**GTCAAGGAATGTGTATCCC -3' (SEQ ID NO: 56).

CHST10 (rs1370627) Flanking primer:

5'- CCTTAACACCTGAAGGAAACGGCTC**<u>T</u>**TAACGCTGTGTGATGCTTCC -3' (SEQ ID NO: 57).

CHST10 (rs6716367) Flanking primer:

5'- CCAGAGCCCAGGAAAGACAG**<u>T</u>**TAAGGCCAGTTCTACATTATC -3' (SEQ ID NO: 58).

CHST10 (rs7585898) Flanking primer:

5'- CCCTTCTAGACTCTGGTCTA**<u>C</u>**AAGTCTACAGCTTTTGATC -3' (SEQ ID NO: 59).

CHST10 (rs17024136) Flanking primer:

5'- GCTATGAACACGGGCCACCACA**<u>G</u>**TTAACATCACGATCACTGTC -3' (SEQ ID NO: 60).

CHST10 (rs17024129) Flanking primer:

5'- GTAATTAATCATATGCCAAGACAGA**<u>A</u>**AAAGAACCGCAATGACATG -3' (SEQ ID NO: 61).

CHST10 (rs7574684) Flanking primer:

5'- GGCCTTGCTGCCCCTGTTTA**<u>C</u>**GGTGCTGTTTCCCCTCAAG -3' (SEQ ID NO: 62).

TEKT5 Flanking Primer:

5' CAGTAGCTTGGCTGCAGGCTCC**<u>A</u>**GTGCTGGAAGGAATCGGCTG-3' (SEQ ID NO: 107).

GRINL1A (SNP2) Flanking primer:

5'-GGAGAAGGACAGACCAGGGAGA**<u>T</u>**TAGGTGGAGAACCAGGAGAGAAAAG -3' (SEQ ID NO: 108).

GRINL1A (rs4774281) Flanking primer:

5'-CCACAGAGCTATTACCCCACATA**<u>C</u>**GAATTACAAAGGAAACCAAAGG-3'(SEQ ID NO: 109).

GRINL1A (rs8037000) Flanking primer:

5'- GGGCCAGTCTGGTCTTGAAC**<u>C</u>**CCTGGCCTCAAATGATCCGC-3' (SEQ ID NO: 110).

GRINL1A (rs6493943) Flanking primer:

5'-CTTTTTTTTTTTGAGACCAAATCTCTCT**<u>C</u>**TCTTGCCCAGGCTGGAGTGC - 3' (SEQ ID NO: 111).

GRINL1A (SNP12) Flanking primer:

5'- GATAGGCCCCCCACCCTCCCC**<u>C</u>**GACACACACAGTCAATCTTATGG-3' (SEQ ID NO: 112).

GRINL1A (rs4774282) Flanking primer:

5'- CCTCCAGTGGTGAGACTCAAC**<u>C</u>**AGCAATCAGCAGTGCTCTAAAACC-3' (SEQ ID NO: 113).

Flanking primer is complementary to the upstream flanking sequence and mutation locus of variable mutation (in bold and underlined). M13 forward sequencing primer hybridizes to the pUC19 vector. PCR conditions and Klenow treatments follow the same procedures as provided in Step 2, above. The PCR product is then digested with the restriction enzyme, HindIII.

4. Prepare the pUC19 vector for cloning the cDNA or genomic clone comprising the polymorphic locus. Digest pUC19 plasmid DNA with EcoRI and HindII. The resulting digested vector fragment can then be purified using techniques well known in the art, such as gel purification, for example.

5. Combine the products from Step 2 (PCR product containing mutation), Step 3 (PCR product containing the upstream region), and Step 4 (digested vector), and ligate them together using standard blunt-end ligation conditions (Sambrook, et al., 1989).

6. Transform the resulting recombinant plasmid from Step 5 into E.coli competent cells using methods known in the art, such as, for example, the transformation methods described in Sambrook, et al., 1989, *Id.*.

7. Analyze the amplified fragment portion of the plasmid DNA by DNA sequencing to confirm the point mutation, and absence of any other mutations introduced during PCR. The method of sequencing the insert DNA, including the primers utilized, are described herein or are otherwise known in the art.

EXAMPLE 6- ALTERNATIVE METHODS OF GENOTYPING POLMORPHISMS ENCOMPASSED BY THE INVENTION

Preparation of Samples

Polymorphisms are detected in a target nucleic acid from an individual being analyzed. For assay of genomic DNA, virtually any biological sample (other than pure red blood cells) is suitable. For example, convenient tissue samples include whole blood, semen, saliva, tears, urine, fecal material, sweat, buccal, skin and hair. For assay of cDNA or mRNA, the tissue sample must be obtained from an organ in which the target nucleic acid is expressed.

Many of the methods described below require amplification of DNA from target samples. This can be accomplished, for example, by polymerase chain reaction (PCR). See generally PCR Technology: Principles and Applications for DNA Amplification (ed. H.A. Erlich, Freeman Press, NY, NY, 1992); PCR Protocols: A Guide to Methods and Applications (eds. Innis, et al., Academic Press, San Diego, CA, 1990); Mattila et al., Nucleic Acids Res. 19, 4967 (1991); Eckert et al., PCR Methods and Applications 1, (1991); PCR (eds. McPherson et al., IRL Press, Oxford); and U.S. Patent 4,683,202.

Other suitable amplification methods include the ligase chain reaction (LCR) (see Wu and Wallace, Genomics 4:560 (1989), Landegren et al., Science 241:1077 (1988), transcription amplification (Kwoh et al., Proc. Natl. Acad. Sci. USA 86, 1173 (1989), self-sustained sequence replication (Guatelli et al., Proc. Nat. Acad. Sci. USA, 87:1874 (1990)) and nucleic acid based sequence amplification (NASBA). The latter two amplification methods involve isothermal reactions based on isothermal transcription, which produce both single stranded RNA (ssRNA) and double stranded DNA (dsDNA) as the amplification products in a ratio of about 30 or 100 to 1, respectively. Additional methods of amplification are known in the art or are described elsewhere herein.

Detection of Polymorphisms in Target DNA

There are two distinct types of analysis of target DNA for detecting polymorphisms. The first type of analysis, sometimes referred to as de novo characterization, is carried out to identify polymorphic sites not previously characterized (i.e., to identify new polymorphisms). This analysis compares target sequences in different individuals to identify points of variation, i.e., polymorphic sites. By analyzing groups of individuals representing the greatest ethnic diversity among humans and greatest breed and species variety in plants and animals, patterns characteristic of the most common alleles/haplotypes of the locus can be identified, and the frequencies of such alleles/haplotypes in the population can be determined. Additional allelic frequencies can be determined for subpopulations characterized by criteria such as geography, race, or gender. The de novo identification of polymorphisms of the invention is described in the Examples section.

The second type of analysis determines which form(s) of a characterized (known) polymorphism are present in individuals under test. Additional methods of analysis are known in the art or are described elsewhere herein.

Allele-Specific Probes

The design and use of allele-specific probes for analyzing polymorphisms is described, for example, by Saiki et al., Nature 324,163-166 (1986); Dattagupta, EP 235,726, and Saiki, WO 89/11548. Allele-specific probes can be designed that hybridize to a segment of target DNA from one individual but do not hybridize to the corresponding segment from another individual due to the presence of different polymorphic forms in the respective segments from the two individuals. Hybridization conditions should be sufficiently stringent so that there is a significant difference in hybridization intensity between alleles, and preferably an essentially binary response, whereby a probe hybridizes to only one of the alleles. Some probes are designed to hybridize to a segment of target DNA such that the polymorphic locus aligns with a central position (e.g., in a 15-mer at the 7 position; in a 16-mer, at either the 8 or 9 position) of the probe. This design of probe achieves good discrimination in hybridization between different allelic forms.

Allele-specific probes are often used in pairs, one member of a pair showing a perfect match to a reference form of a target sequence and the other member showing a perfect match to a variant form. Several pairs of probes can then be immobilized on the same support for simultaneous analysis of multiple polymorphisms within the same target sequence.

Tiling Arrays

The polymorphisms can also be identified by hybridization to nucleic acid arrays, some examples of which are described in WO 95/11995. The same arrays or different arrays can be used for analysis of characterized polymorphisms. WO 95/11995 also describes sub arrays that are optimized for detection of a variant form of a precharacterized polymorphism. Such a sub array contains probes designed to be complementary to a second reference sequence, which is

an allelic variant of the first reference sequence. The second group of probes is designed by the same principles as described, except that the probes exhibit complementarity to the second reference sequence. The inclusion of a second group (or additional groups) can be particularly useful for analyzing short subsequences of the primary reference sequence in which multiple mutations are expected to occur within a short distance commensurate with the length of the probes (e.g., two or more mutations within 9 to 21 bases).

Allele-Specific Primers

An allele-specific primer hybridizes to a site on target DNA overlapping a polymorphism and only primes amplification of an allelic form to which the primer exhibits perfect complementarity. See Gibbs, Nucleic Acid Res. 17,2427-2448 (1989). This primer is used in conjunction with a second primer that hybridizes at a distal site. Amplification proceeds from the two primers, resulting in a detectable product that indicates the particular allelic form is present. A control is usually performed with a second pair of primers, one of which shows a single base mismatch at the polymorphic locus and the other of which exhibits perfect complementarity to a distal site. The single-base mismatch prevents amplification and no detectable product is formed. The method works best when the mismatch is included in the 3'-most position of the oligonucleotide aligned with the polymorphism because this position is the most destabilizing elongation from the primer (see, e.g., WO 93/22456).

Direct-Sequencing

The direct analysis of the sequence of polymorphisms of the invention can be accomplished using either the dideoxy chain termination method or the Maxam - Gilbert method (see Sambrook et al., Molecular Cloning, A Laboratory Manual (2nd Ed., CSHP, New York 1989); Zyskind et al., Recombinant DNA Laboratory Manual, (Acad. Press, 1988)).

Denaturing Gradient Gel Electrophoresis

Amplification products generated using the polymerase chain reaction can be analyzed by the use of denaturing gradient gel electrophoresis. Different alleles can be identified based on the different sequence-dependent melting properties and electrophoretic migration of DNA in solution. Erlich, ed., PCR Technology. Principles and Applications for DNA Amplification, (W .H. Freeman and Co, New York, 1992), Chapter 7.

Single-Strand Conformation Polymorphism Analysis

Alleles of target sequences can be differentiated using single-strand conformation polymorphism analysis, which identifies base differences by alteration in electrophoretic migration of single stranded PCR products, as described in Orita et al., Proc. Nat. Acad. Sci. 86,2766-2770 (1989). Amplified PCR products can be generated as described above, and heated or otherwise denatured, to form single stranded amplification products. Single-stranded nucleic acids may refold or form secondary structures that are partially dependent on the base sequence. The different electrophoretic mobilities of single-stranded amplification products can be related to base-sequence differences between alleles of target sequences.

Single Base Extension

An alternative method for identifying and analyzing polymorphisms is based on single-base extension (SBE) of a fluorescently-labeled primer coupled with fluorescence resonance energy transfer (FRET) between the label of the added base and the label of the primer. Typically, the method, such as that described by Chen et al., (PNAS 94:10756-61 (1997), uses a locus-specific oligonucleotide primer labeled on the 5' terminus with 5-carboxyfluorescein (FAM). This labeled primer is designed so that the 3' end is immediately adjacent to the polymorphic locus of interest. The labeled primer is hybridized to the locus, and single base extension of the labeled primer is performed with fluorescently-labeled dideoxyribonucleotides (ddNTPs) in dye-terminator sequencing fashion. An increase in fluorescence of the added ddNTP in response to excitation at the wavelength of the labeled primer is used to infer the identity of the added nucleotide.

EXAMPLE 7 - ADDITIONAL METHODS OF GENOTYPING THE SNPs OF THE INVENTION

There are a number of methods that can be employed for genotyping a SNP of the invention in addition to the methods described herein. The invention encompasses the following non-limiting types of genotype assays: PCR-free genotyping methods, Single-step homogeneous methods, Homogeneous detection with fluorescence polarization, Pyrosequencing, "Tag" based DNA chip system, Bead-based methods, fluorescent dye chemistry, Mass spectrometry

based genotyping assays, TaqMan® genotype assays, Invader genotype assays, and microfluidic genotype assays, among others.

Specifically encompassed by the invention are the following, non-limiting genotyping methods: Landegren, U., Nilsson, M. & Kwok, P. Genome Res 8, 769-776 (1998); Kwok, P., Pharmacogenomics 1, 95-100 (2000); Gut, I., Hum Mutat 17, 475-492 (2001); Whitcombe, D., Newton, C. & Little, S., Curr Opin Biotechnol 9, 602-608 (1998); Tillib, S. & Mirzabekov, A., Curr Opin Biotechnol 12, 53-58 (2001); Winzeler, E. et al., Science 281, 1194-1197 (1998); Lyamichev, V. et al., Nat Biotechnol 17, 292-296 (1999); Hall, J. et al., Proc Natl Acad Sci U S A 97, 8272-8277 (2000); Mein, C. et al., Genome Res 10, 333-343 (2000); Ohnishi, Y. et al., J Hum Genet 46, 471-477 (2001); Nilsson, M. et al., Science 265, 2085-2088 (1994); Baner, J., Nilsson, M., Mendel-Hartvig, M. & Landegren, U., Nucleic Acids Res 26, 5073-5078 (1998); Baner, J. et al., Curr Opin Biotechnol 12, 11-15 (2001); Hatch, A., Sano, T., Misasi, J. & Smith, C., Genet Anal 15, 35-40 (1999); Lizardi, P. et al., Nat Genet 19, 225-232 (1998); Zhong, X., Lizardi, P., Huang, X., Bray-Ward, P. & Ward, D., Proc Natl Acad Sci U S A 98, 3940-3945 (2001); Faruqi, F. et al. BMC Genomics 2, 4 (2001); Livak, K., Gnet Anal 14, 143-149 (1999); Marras, S., Kramer, F. & Tyagi, S., Genet Anal 14, 151-156 (1999); Ranade, K. et al., Genome Res 11, 1262-1268 (2001); Myakishev, M., Khripin, Y., Hu, S. & Hamer, D., Genome Re 11, 163-169 (2001); Beaudet, L., Bedard, J., Breton, B., Mercuri, R. & Budarf, M., Genome Res 11, 600-608 (2001); Chen, X., Levine, L. & PY, K., Genome Res 9, 492-498 (1999); Gibson, N. et al., Clin Chem 43, 1336-1341 (1997); Latif, S., Bauer-Sardina, I., Ranade, K., Livak, K. & PY, K., Genome Res 11, 436-440 (2001); Hsu, T., Law, S., Duan, S., Neri, B. & Kwok, P., Clin Chem 47, 1373-1377 (2001); Alderborn, A., Kristofferson, A. & Hammerling, U., Genome Res 10, 1249-1258 (2000); Ronaghi, M., Uhlen, M. & Nyren, P., Science 281, 363, 365 (1998); Ronaghi, M., Genome Res 11, 3-11 (2001); Pease, A. et al., Proc Natl Acad Sci U S A 91, 5022-5026 (1994); Southern, E., Maskos, U. & Elder, J., Genomics 13, 1008-1017 (1993); Wang, D. et al., Science 280, 1077-1082 (1998); Brown, P. & Botstein, D., Nat Genet 21, 33-37 (1999); Cargill, M. et al. Nat Genet 22, 231-238 (1999); Dong, S. et al., Genome Res 11, 1418-1424 (2001); Halushka, M. et al., Nat Genet 22, 239-247 (1999); Hacia, J., Nat Genet 21, 42-47 (1999); Lipshutz, R., Fodor, S., Gingeras, T. & Lockhart, D., Nat Genet 21, 20-24 (1999); Sapolsky, R. et al., Genet Anal 14, 187-192 (1999); Tsuchihashi, Z. & Brown, P., J Virol 68, 5863 (1994); Herschlag, D., J Biol Chem 270, 20871-20874 (1995); Head, S. et al., Nucleic Acids Res 25, 5065-5071 (1997); Nikiforov, T. et al., Nucleic Acids Res 22, 4167-4175 (1994); Syvanen, A. et al., Genomics 12, 590-595 (1992); Shumaker, J., Metspalu, A. & Caskey, C., Hum Mutat 7, 346-354 (1996); Lindroos, K., Liljedahl, U., Raitio, M. & Syvanen, A., Nucleic Acids Res 29, E69-9 (2001); Lindblad-Toh, K. et al., Nat Genet 24, 381-386 (2000); Pastinen, T. et al., Genome Res 10, 1031-1042 (2000); Fan, J. et al., Genome Res 10, 853-860 (2000); Hirschhorn, J. et al., Proc Natl Acad Sci U S A 97, 12164-12169 (2000); Bouchie, A., Nat Biotechnol 19, 704 (2001); Hensel, M. et al., Science 269, 400-403 (1995); Shoemaker, D., Lashkari, D., Morris, D., Mittmann, M. & Davis, R. Nat Genet 14, 450-456 (1996); Gerry, N. et al., J Mol Biol 292, 251-262 (1999); Ladner, D. et al., Lab Invest 81, 1079-1086 (2001); Iannone, M. et al. ,Cytometry 39, 131-140 (2000); Fulton, R., McDade, R., Smith, P., Kienker, L. & Kettman, J. J., Clin Chem 43, 1749-1756 (1997); Armstrong, B., Stewart, M. & Mazumder, A., Cytometry 40, 102-108 (2000); Cai, H. et al., Genomics 69, 395 (2000); Chen, J. et al., Genome Res 10, 549-557 (2000); Ye, F. et al. Hum Mutat 17, 305-316 (2001); Michael, K., Taylor, L., Schultz, S. & Walt, D., Anal Chem 70, 1242-1248 (1998); Steemers, F., Ferguson, J. & Walt, D., Nat Biotechnol 18, 91-94 (2000); Chan, W. & Nie, S., Science 281, 2016-2018 (1998); Han, M., Gao, X., Su, J. & Nie, S., Nat Biotechnol 19, 631-635 (2001); Griffin, T. & Smith, L., Trends Biotechnol 18, 77-84 (2000); Jackson, P., Scholl, P. & Groopman, J., Mol Med Today 6, 271-276 (2000); Haff, L. & Smirnov, I., Genome Res 7, 378-388 (1997); Ross, P., Hall, L., Smirnov, I. & Haff, L., Nat Biotechnol 16, 1347-1351 (1998); Bray, M., Boerwinkle, E. & Doris, P. Hum Mutat 17, 296-304 (2001); Sauer, S. et al., Nucleic Acids Res 28, E13 (2000); Sauer, S. et al., Nucleic Acids Res 28, E100 (2000); Sun, X., Ding, H., Hung, K. & Guo, B., Nucleic Acids Res 28, E68 (2000); Tang, K. et al., Proc Natl Acad Sci U S A 91, 10016-10020 (1999); Li, J. et al., Electrophoresis 20, 1258-1265 (1999); Little, D., Braun, A., O'Donnell, M. & Koster, H., Nat Med 3, 1413-1416 (1997); Little, D. et al. Anal Chem 69, 4540-4546 (1997); Griffin, T., Tang, W. & Smith, L., Nat Biotechnol 15, 1368-1372 (1997); Ross, P., Lee, K. & Belgrader, P., Anal Chem 69, 4197-4202 (1997); Jiang-Baucom, P., Girard, J., Butler, J. & Belgrader, P., Anal Chem 69, 4894-4898 (1997); Griffin, T., Hall, J., Prudent, J. & Smith, L., Proc Natl Acad Sci U S A 96, 6301-6306 (1999); Kokoris, M. et al., Mol Diagn 5, 329-340 (2000); Jurinke, C., van den Boom, D., Cantor, C. & Koster, H. (2001); and/or Taranenko, N. et al., Genet Anal 13, 87-94 (1996); Aomori, T., Yamamoto, K., Oguchi-Katayama, A., Kawai, Y., Ishidao, T., Mitani, Y., et al. Clinical Chemistry, 55(4), 804-812 (2009); Lin, C. H., Yeakley, J. M., McDaniel, T. K., & Shen, R. (2009); Konstantou, J. K., Ioannou, P. C., & Christopoulos, T. K. European Journal of Human Genetics, 17 (1) (2009); Li, X., Luo, J., Xiao, P., Shi, X., Tang, C., & Lu, Z. Clinica Chimica Acta, 399 (1-2), 40-44 (2009); Hui, L., DelMonte, T., & Ranade, K. Current Protocols in Human Genetics (SUPPL. 56), 2.10.1-2.10.8 (2008); Maresso, K., & Broeckel, U. (2008); Browning, S. R., & Browning, B. L. American Journal of Human Genetics 81(5), 1084-1097 (2007). In addition, the genotyping methods described and/or claimed in U.S. Patent Serial No. 6,458,540 and the methods described and/or claimed in U.S. Patent Serial No. 6,440,707 are also encompassed by the invention. All of the references and patents are hereby incorporated herein in their entireties.

The entire disclosure of each document cited (including patents, patent applications, journal articles, abstracts, laboratory manuals, books, or other disclosures) is hereby incorporated herein by reference in its entirety. Further, the

Sequence Listing submitted herewith is incorporated herein by reference in its entirety.

While this invention has been particularly shown and described with references to preferred embodiments thereof, it will be understood by those skilled in the art that various changes in form and details may be made therein without departing from the scope of the invention encompassed by the appended claims.

It will be clear that the invention may be practiced otherwise than as particularly described in the foregoing description and examples. Numerous modifications and variations of the invention are possible in light of the above teachings and, therefore, are within the scope of the appended claims.

The invention further discloses the following items:

1. A method comprising the steps of

   (a) assaying a sample from an individual with Type 2 diabetes and
   (b) detecting a reference or a variant allele at one or more polymorphic loci of SEQ ID NOs: 1-12, 63, and/or 69-75.

2. The method according to item 1, wherein said polymorphic loci are at nucleotide position 87,712 of SEQ ID NOs: 1, 73 and/or 75, nucleotide position 87,992 of SEQ ID NOs: 1, 2 and/or 75, nucleotide position 89,441 of SEQ ID NOs: 1, 69 and/or 75, nucleotide position 89,662 of SEQ ID NOs: 1, 70 and/or 75, nucleotide position 89,853 of SEQ ID NOs: 1, 71 and/or 75, nucleotide position 93,598 of SEQ ID NOs: 1, 74 and/or 75, and nucleotide position 94,074 of SEQ ID NOs: 1, 72 and/or 75.

3. The method according to item 1, wherein said reference allele at the polymorphic locus at nucleotide position 87,712 is "A" and said variant allele at the polymorphic locus is "G", wherein said reference allele at the polymorphic locus at nucleotide position 87,992 is "T" and said variant allele at the polymorphic locus is "C", wherein said reference allele at the polymorphic locus at nucleotide position 89,441 is "A" and said variant allele at the polymorphic locus is "G", wherein said reference allele at the polymorphic locus at nucleotide position 89,662 is "G" and said variant allele at the polymorphic locus is "A", wherein said reference allele at the polymorphic locus at nucleotide position 89,853 is "G" and said variant allele at the polymorphic locus is "A", wherein said reference allele at the polymorphic locus at nucleotide position 93,598 is "G" and said variant allele at the polymorphic locus is "A", and wherein said reference allele at the polymorphic locus at nucleotide position 94,074 is "G" and said variant allele at the polymorphic locus is "C".

4. The method of item 1, wherein the step of assaying a sample from an individual with Type 2 diabetes consists of:

   (a) obtaining a nucleic acid sample from said individual; and
   (b) subjecting the sample to genetic sequencing, microarray, or PCR analysis to identify the reference or variant allele.

5. The method of item 1, wherein the step of detecting a reference or a variant allele consists of:

   (a) subjecting the sample to automated genetic sequencing;
   (b) displaying sequencing data on a data screen for analysis; and
   (c) recording the identity of said reference or variant allele at one or more polymorphic loci of SEQ ID NOs: 1-12, 63, and/or 69-75.

6. The method of item 1, wherein the step of detecting a reference or a variant allele consists of:

   (a) obtaining sequence data from the assaying step; and
   (b) recording the identity of said reference or variant allele at one or more polymorphic loci of SEQ ID NOs: 1-12, 63, and/or 69-75.

7. The method of item 5 or 6, further comprising the steps of:

   (a) incorporating the identified reference or variant allele data at one or more polymorphic loci of SEQ ID NOs: 1-12, 63, and/or 69-75 into an ANCOVA analysis;
   (b) conducting said ANCOVA analysis; and
   (c) determining the probability of a favorable response or the magnitude of a favorable change in HbA1C of said individual resulting from treating said individual with a DPP-IV inhibitor or a DPP-IV inhibitor in combination with other oral antidiabetic therapy.

8. The method of item 5 or 6, further comprising the step of determining the probability of a favorable response or the magnitude of a favorable change in HbA1C of said individual resulting from treating said individual with a DPP-IV inhibitor or a DPP-IV inhibitor in combination with other oral antidiabetic therapy.

9. The method of item 7 or 8, further comprising the step of administering a DPP-IV inhibitor or a DPP-IV inhibitor in combination with other oral antidiabetic therapy to said individual where said individual is determined to have a probability of a favorable response or a significantly higher magnitude of favorable change in HbA1C resulting from treating said individual with a DPP-IV inhibitor or a DPP-IV inhibitor in combination with other oral antidiabetic therapy.

10. A method of treating an individual with Type 2 diabetes comprising the steps of:

(a) obtaining a nucleic acid sample from said individual;
(b) subjecting the sample to genetic sequencing, microarray, or PCR analysis;
(c) displaying sequencing data on a data screen for analysis;
(d) recording the identity of said reference or variant allele at one or more polymorphic loci of SEQ ID NOs: 1-12, 63, and/or 69-75;
(e) incorporating the identified reference or variant allele data at one or more polymorphic loci of SEQ ID NOs: 1-12, 63, and/or 69-75 into an ANCOVA analysis;
(f) conducting an ANCOVA analysis of said reference or variant allele data;
(g) determining a probability of the likelihood of a favorable response or the magnitude of a favorable change in HbA1C of said individual resulting from treating said individual with a DPP-IV inhibitor or a DPP-IV inhibitor in combination with other oral antidiabetic therapy; and
(h) administering a DPP-IV inhibitor or a DPP-IV inhibitor in combination with other oral antidiabetic therapy to said individual where said individual is determined to have a high probability of likelihood of a favorable response or a significantly high magnitude of a favorable change in HbA1C resulting from treatment of said individual with a DPP-IV inhibitor or a DPP-IV inhibitor in combination with other oral antidiabetic therapy.

11. A method of treating an individual with Type 2 diabetes comprising the steps of:

(a) obtaining a nucleic acid sample from said individual;
(b) subjecting the sample to sequence analysis;
(d) recording the identity of said reference or variant allele at one or more polymorphic loci of SEQ ID NOs: 1-12, 63, and/or 69-75;
(e) determining a probability of the likelihood of a favorable response or the magnitude of a favorable change in HbA1C of said individual resulting from treating said individual with a DPP-IV inhibitor or a DPP-IV inhibitor in combination with other oral antidiabetic therapy; and
(f) administering a DPP-IV inhibitor or a DPP-IV inhibitor in combination with other oral antidiabetic therapy to said individual where said individual is determined to have a high probability of likelihood of a favorable response or a significantly high magnitude of a favorable change in HbA1C resulting from treatment of said individual with a DPP-IV inhibitor or a DPP-IV inhibitor in combination with other oral antidiabetic therapy.

12. The method of item 9, 10, or 11, wherein said DPP-IV inhibitor is saxagliptin.

13. The method of item 9, 10, or 11, wherein said other oral antidiabetic therapy is metformin, TZD, or glyburide.

14. The method of item 7 or 8, wherein said probability of likelihood of a favorable or said significantly high magnitude of a favorable change in HbA1C response to administration of a DPP-IV inhibitor or a DPP-IV inhibitor in combination with other oral antidiabetic therapy is:

greater in individuals homozygous for reference alleles at one or more polymorphic loci of SEQ ID NOs: 1-12, 63, and/or 69-75;
lower in individuals heterozygous for variant alleles at one or more polymorphic loci of SEQ ID NOs: 1-12, 63, and/or 69-75; and
lower in individuals homozygous for variant alleles at one or more polymorphic loci of SEQ ID NOs: 1-12, 63, and/or 69-75.

15. The method of item 10 or 11, wherein said probability of likelihood of a favorable or said significantly high magnitude of a favorable change in HbA1C response to administration of a DPP-IV inhibitor or a DPP-IV inhibitor

in combination with other oral antidiabetic therapy is:

greater in individuals homozygous for reference alleles at one or more polymorphic loci of SEQ ID NOs: 1-12, 63, and/or 69-75;
lower in individuals heterozygous for variant alleles at one or more polymorphic loci of SEQ ID NOs: 1-12, 63, and/or 69-75; and
lower in individuals homozygous for variant alleles at one or more polymorphic loci of SEQ ID NOs: 1-12, 63, and/or 69-75.

16. A diagnostic kit for identifying a subject or patient more likely to have a favorable response to the administration of a therapeutically-effective amount of a DPP-IV inhibitor or a DPP-IV inhibitor in combination with other oral antidiabetic therapy, wherein said kit comprises:

oligonucleotides having SEQ ID NOs: 13-44, 65-66, and 76-103.

## Claims

**1.** A method comprising the steps of

(a) assaying a sample from an individual with Type 2 diabetes;
(b) detecting a reference or a variant allele at the polymorphic loci at position 26,472 of SEQ ID NOs: 63 or 64; and
(c) identifying an individual likely to have a high probability of likelihood of a favorable response or a significantly high magnitude of a favorable change in HbAIC response to administration of a DPP-IV inhibitor or a DPP-IV inhibitor in combination with other oral antidiabetic therapy, wherein a reference allele is present.

**2.** The method of claim 1, wherein the step of assaying a sample from an individual with Type 2 diabetes comprises:

(a) obtaining a nucleic acid sample from said individual; and
(b) subjecting the sample to genetic sequencing, microarray, or PCR analysis to identify the reference or variant allele.

**3.** The method of claim 1 or 2, wherein the step of detecting a reference or a variant allele comprises:

(a) subjecting the sample to automated genetic sequencing;
(b) displaying sequencing data on a data screen for analysis; and
(c) recording the identity of said reference or variant allele at the polymorphic loci at position 26,472 of SEQ ID NOs: 63 or 64;

or consists of

(a) obtaining sequence data from the assaying step; and
(b) recording the identity of said reference or variant allele at the polymorphic loci at position 26,472 of SEQ ID NOs: 63 or 64.

**4.** The method of claim 3, further comprising the steps of:

(a) incorporating the identified reference or variant allele data at the polymorphic loci at position 26,472 of SEQ ID NOs: 63 or 64 into an ANCOVA analysis;
(b) conducting said ANCOVA analysis; and
(c) determining the probability of a favorable response or the magnitude of a favorable change in HbAIC of said individual resulting from treating said individual with a DPP-IV inhibitor or a DPP-IV inhibitor in combination with other oral antidiabetic therapy,

or further comprising the step of:

determining the probability of a favorable response or the magnitude of a favorable change in HbAIC of said individual resulting from treating said individual with a DPP-IV inhibitor or a DPP-IV inhibitor in combination with

other oral antidiabetic therapy.

5. A DPP-IV inhibitor or a DPP-IV inhibitor in combination with other oral antidiabetic therapy for use in the treatment of Type 2 diabetes in an individual, wherein said individual is determined to have a high probability of likelihood of a favorable response or a significantly high magnitude of a favorable change in HbAIC resulting from said treatment wherein a reference allel is present, which determination comprises the steps of:

(a) subjecting a nucleic acid sample to be obtained from said individual to genetic sequencing, microarray, or PCR analysis;
(b) displaying sequencing data on a data screen for analysis;
(c) recording the identity of said reference or variant allele at the polymorphic loci at position 26,472 of SEQ ID NOs: 63 o 64;
(d) incorporating the identified reference or variant allele data at the polymorphic loci at position 26,472 of SEQ ID NOs: 63 or 64 into an ANCOVA analysis; and
(e) conducting an ANCOVA analysis of said reference or variant allele data;

or which determination comprises the steps of:

(a) subjecting a nucleic acid sample to be obtained from said individual to sequence analysis; and
(b) recording the identity of said reference or variant allele at the polymorphic loci at position 26,472 of SEQ ID NOs: 63 or 64.

6. Use of a DPP-IV inhibitor or a DPP-IV inhibitor in combination with other oral antidiabetic for the treatment of Type 2 diabetes in an individual, wherein said individual is determined to have a high probability of likelihood of a favorable response or a significantly high magnitude of a favorable change in HbAIC resulting from said treatment wherein a reference allel is present, which determination comprises the steps of:

(a) subjecting a nucleic acid sample to be obtained from said individual to genetic sequencing, microarray, or PCR analysis;
(b) displaying sequencing data on a data screen for analysis;
(c) recording the identity of said reference or variant allele at polymorphic loci at position 26,472 of SEQ ID NOs: 63 or 64;
(d) incorporating the identified reference or variant allele data at the polymorphic loci at position 26,472 of SEQ ID NOs: 63 or 64 into an ANCOVA analysis; and
(e) conducting an ANCOVA analysis of said reference or variant allele data;

or which determination comprised the steps of:

(a) subjecting a nucleic acid sample to be obtained from said individual to sequence analysis; and
(b) recording the identity of said reference or variant allele at the polymorphic loci at position 26,472 of SEQ ID NO:s 63 or 64.

7. The DPP-IV inhibitor or DPP-IV inhibitor combination of claim 5, or the use of claim 6, wherein said DPP-IV inhibitor is saxagliptin.

8. The DPP-IV inhibitor combination of claim 5, or the use of claim 6, wherein said other oral antidiabetic therapy is metformin, TZD, or glyburide.

9. The method of claim 4, the DPP-IV inhibitor or DPP-IV inhibitor combination of claim 5, or the use of claim 6, wherein said probability of likelihood of a favorable or said significantly high magnitude of a favorable change in HbAIC response to administration of a DPP-IV inhibitor or a DPP-IV inhibitor in combination with other oral antidiabetic therapy is:

greater in individuals homozygous for reference alleles at the polymorphic loci at position 26,472 of SEQ ID NOs: 63 or 64;
lower in individuals heterozygous for variant alleles at the polymorphic loci at position 26,472 of SEQ ID NOs: 63 or 64; and
lower in individuals homozygous for variant alleles at the polymorphic loci at position 26,472 of SEQ ID NOs: 63 or 64.

**FIG. 1**

Association of SNP rs11853270 in GRINL1A with Saxagliptin Response Clusters

Rs11853270 represents position 87,992 of SEQ ID NOs: 1 and 2

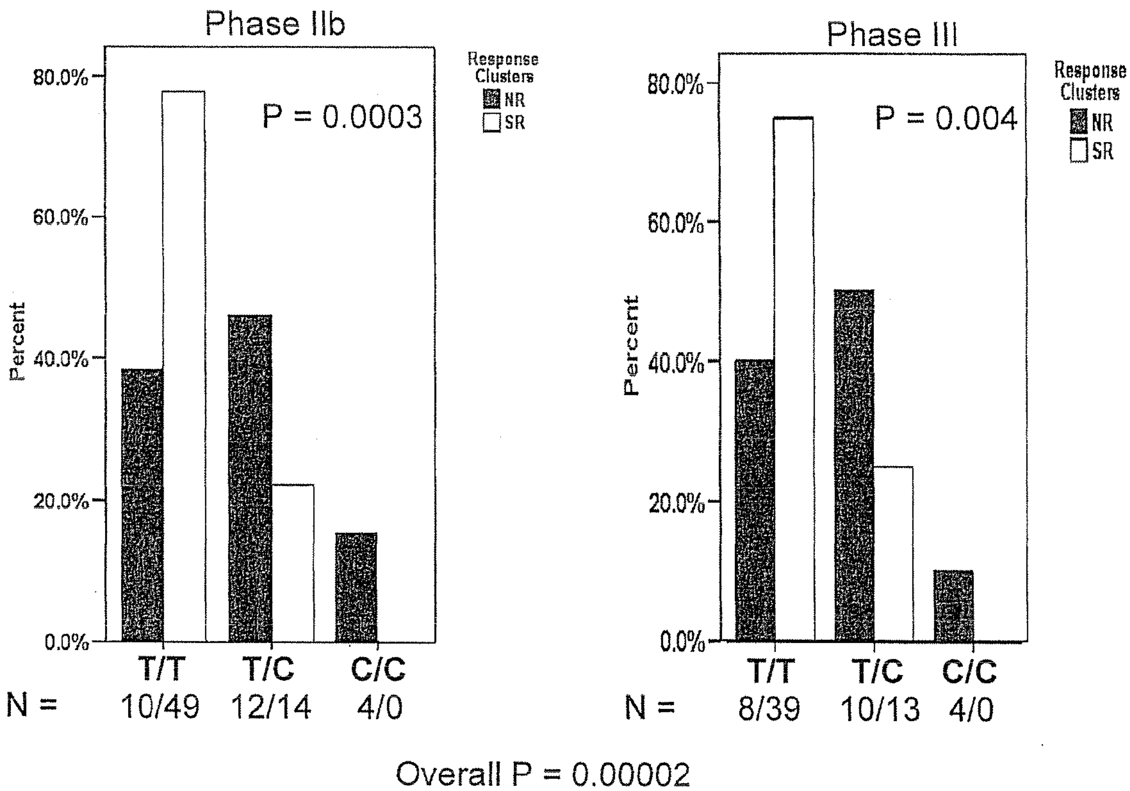

**FIG. 2**

Association of SNP rs17015076 in HSD11B1 with Saxagliptin Response Clusters

Rs17015076 represents position 34,403 of SEQ ID NOs: 3 and 4

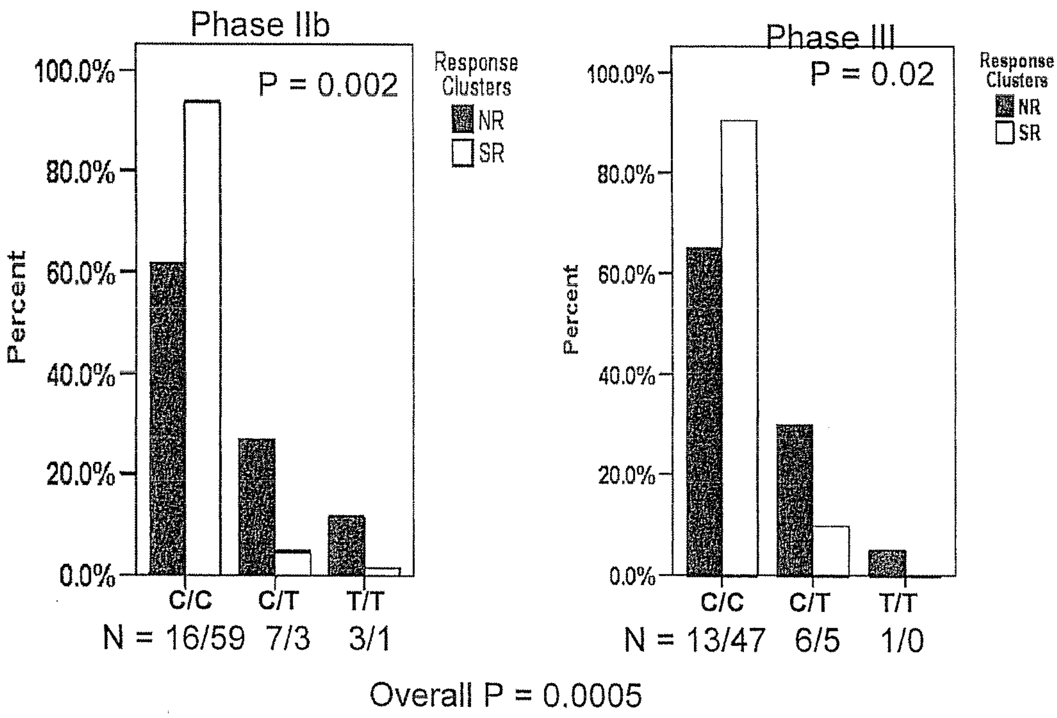

FIG. 3

Association of SNP rs1370627 in CHST10 with Saxagliptin Response Clusters

Rs1370627 represents position 24,707 of SEQ ID NOs: 5 and 6

Allele G in this figure corresponds with the reference allele on the reverse complement strand and Allele A in this figure corresponds with the variant allele on the reverse complement strand

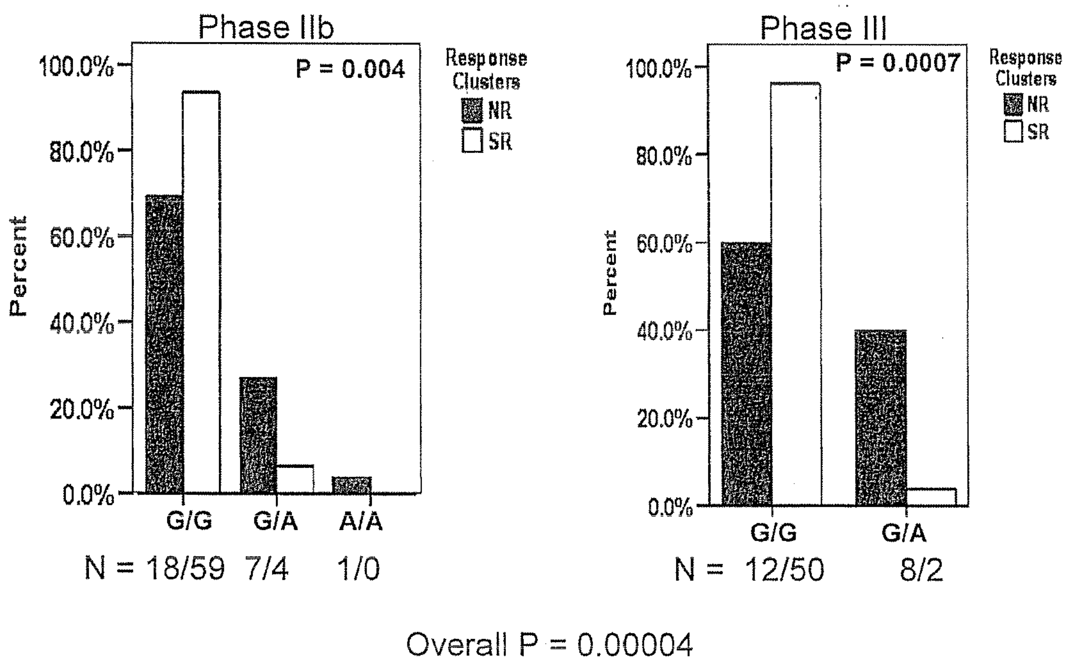

FIG. 4

Association of SNP rs6716367 in CHST10 with Saxagliptin Response Clusters

Rs6716367 represents position 34,078 of SEQ ID NOs: 5 and 7

Allele G in this figure corresponds with the reference allele on the reverse complement strand and Allele A in this figure corresponds with the variant allele on the reverse complement strand

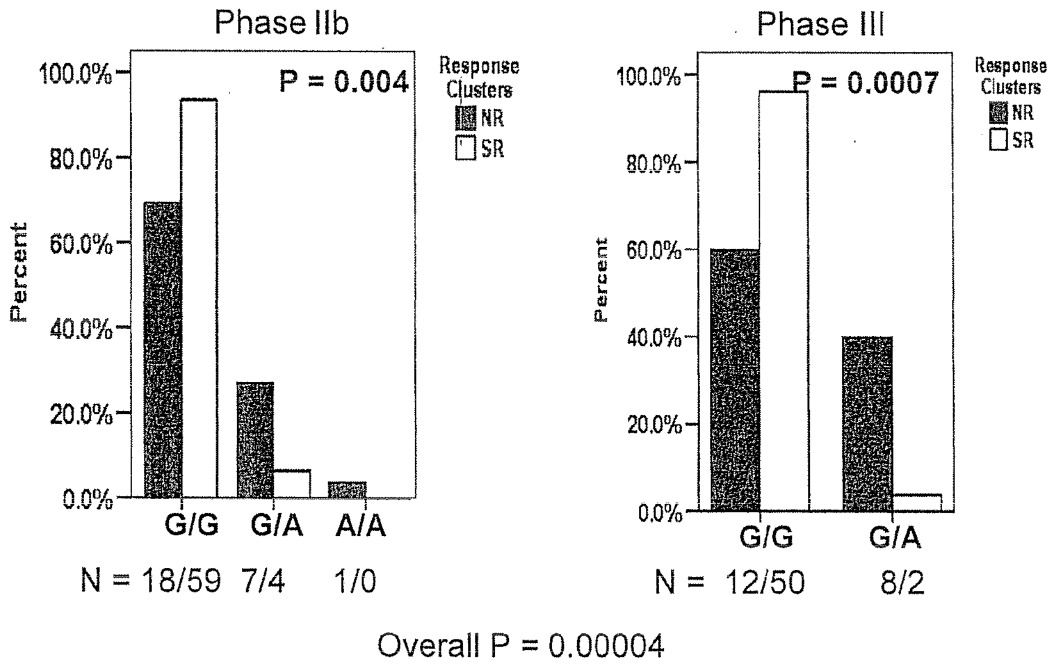

FIG. 5

Association of SNP rs7585898 in CHST10 with Saxagliptin Response Clusters

Rs7585898 represents position 35,799 of SEQ ID NOs: 5 and 8

Allele C in this figure corresponds with the reference allele on the reverse complement strand and Allele G in this figure corresponds with the variant allele on the reverse complement strand

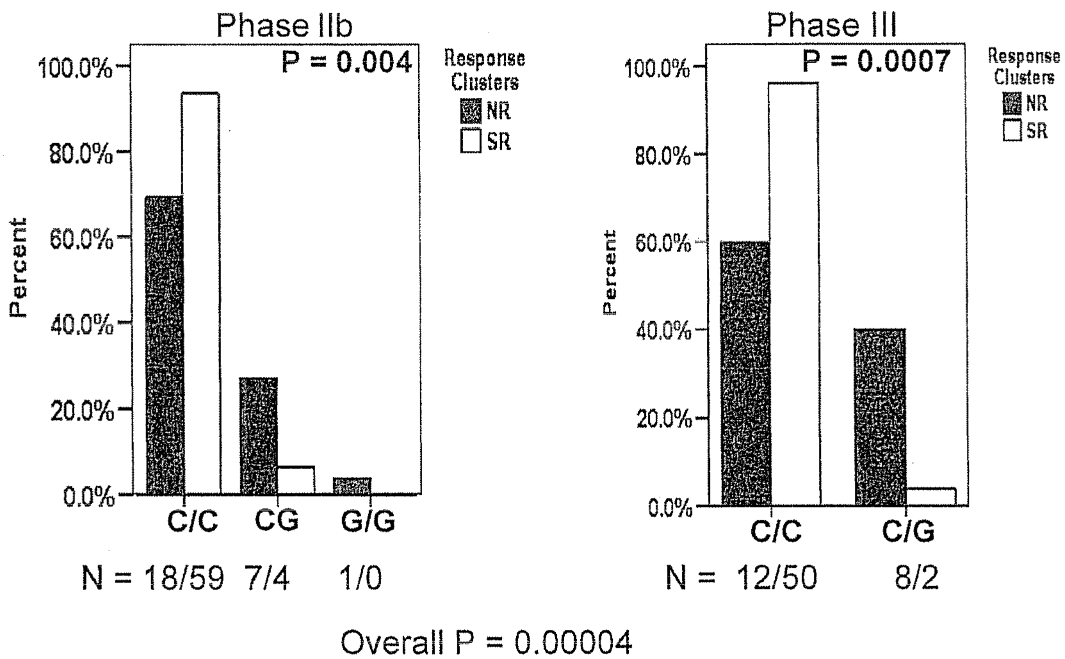

FIG. 6

Association of SNP rs17024136 in CHST10 with Saxagliptin Response Clusters

Rs17024136 represents position 38,709 of SEQ ID NOs: 5 and 9

Allele T in this figure corresponds with the reference allele on the reverse complement strand and Allele C in this figure corresponds with the variant allele on the reverse complement strand

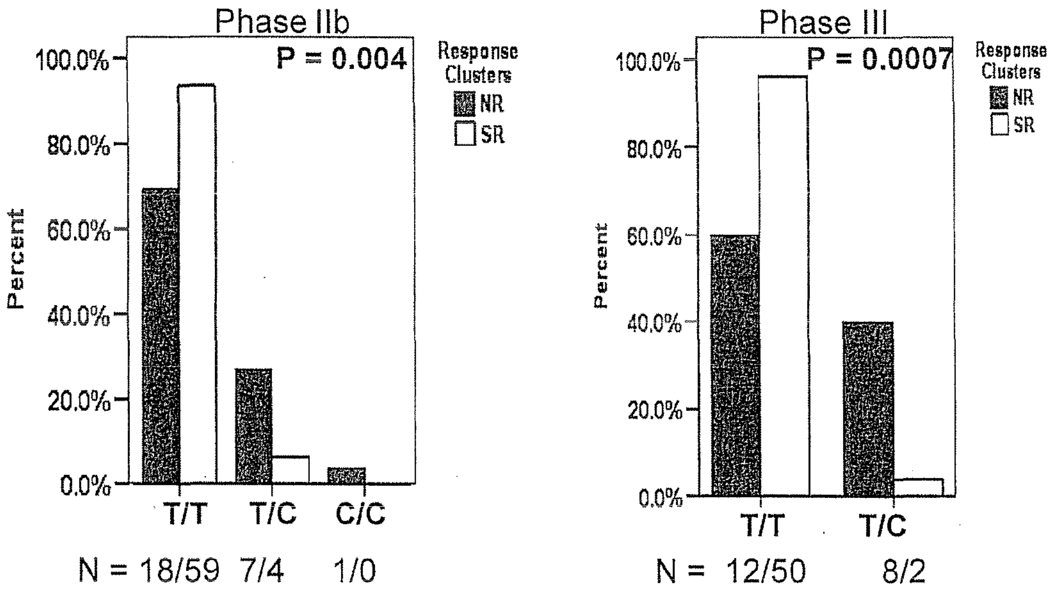


Overall P = 0.00004

FIG. 7

Association of SNP rs17024129 in CHST10 with Saxagliptin Response Clusters

Rs17024129 represents position 38,947 of SEQ ID NOs: 5 and 10

Allele G in this figure corresponds with the reference allele on the reverse complement strand and Allele T in this figure corresponds with the variant allele on the reverse complement strand

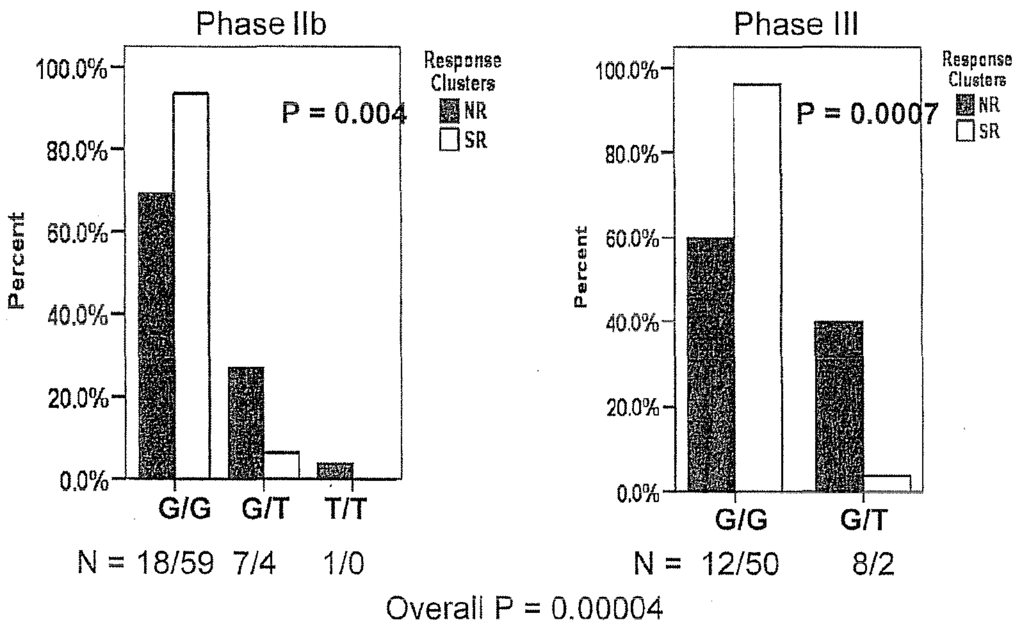

FIG. 8

Association of SNP rs7574684 in CHST10 with Saxagliptin Response Clusters

Rs7574684 represents position 41,180 of SEQ ID NOs: 5 and 11

Allele A in this figure corresponds with the reference allele on the reverse complement strand and Allele G in this figure corresponds with the variant allele on the reverse complement strand

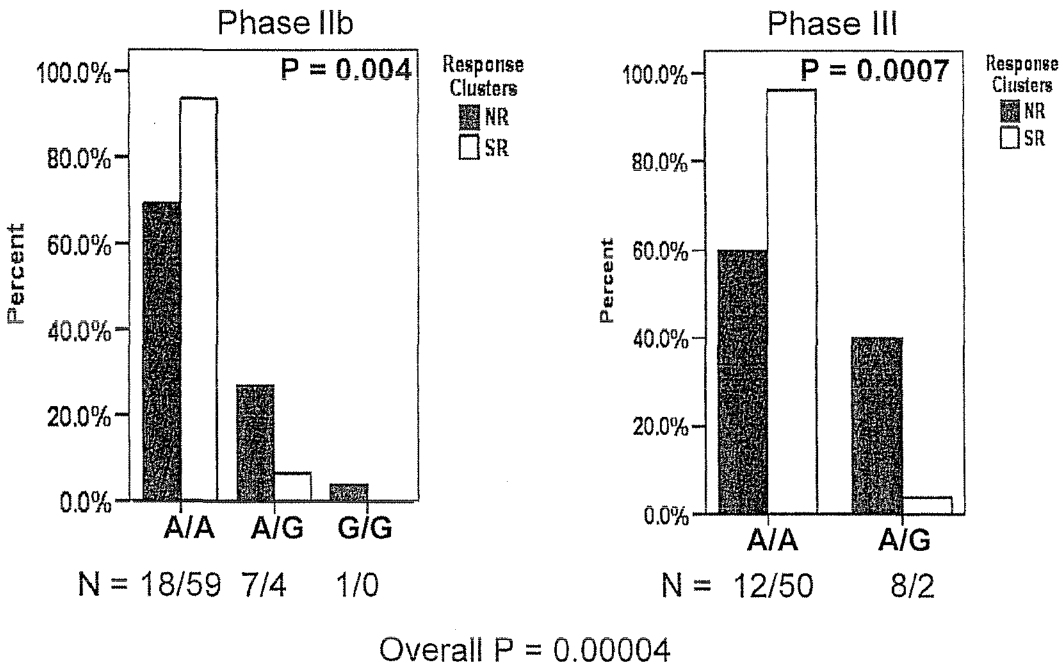

FIG. 9

Association of GRINL1A SNP rs11853270 with Mean Change in HbA1c levels in Response to Saxagliptin Treatment

Rs11853270 represents postion 87,992 of SEQ ID NOs: 1 and 2

| Trial/Treatment | Mean Change in HbA1c ± SD (N) | | |
|---|---|---|---|
| | Overall | WT | SNP Carriers |
| CV181-008 | | | |
| Placebo | -0.3 ± 0.9 (68) | -0.3 ± 0.7 (55) | -0.1 ± 1.3 (13) |
| Saxagliptin | -0.6 ± 0.9 (97) | -0.9 ± 0.7 (68) | -0.1 ± 1.0 (29) |
| CV181-011 | | | |
| Placebo | 0.2 ± 1.4 (58) | 0.4 ± 1.4 (37) | -0.3 ± 1.1 (21) |
| Saxagliptin | -0.5 ± 0.9 (182) | -0.6 ± 0.9 (129) | -0.3 ± 0.9 (53) |

CV181-008: 2.5, 5 & 10 mg doses, P = 0.0003 between wt and SNP carriers on-treatment
CV181-011: randomized cohort, P = 0.03 between wt and SNP carriers on-treatment with baseline HbA1c as covariate

SNP genotype frequencies: Wildtype (WT) ~ 70%; SNP carriers ~ 30%

FIG. 10

Association of GRINL1A SNP rs11853270 with Mean Change in Fasting Glucose levels in Response to Saxagliptin Treatment

Rs11853270 represents postion 87,992 of SEQ ID NOs: 1 and 2

| **Trial/Treatment** | **Mean Change in Fasting Glucose, mgdL ± SD (N)** | | |
|---|---|---|---|
| | **Overall** | **WT** | **SNP Carriers** |
| **CV181-008** | | | |
| **Placebo** | 0.9 ± 32 (70) | 2 ± 28 (57) | -4 ± 49 (13) |
| **Saxagliptin** | -12 ± 29 (92) | -17 ± 25 (65) | -1 ± 34 (27) |
| **CV181-011** | | | |
| **Placebo** | 8 ± 59 (58) | 12 ± 67 (37) | 2 ± 43 (21) |
| **Saxagliptin** | -15 ± 36 (185) | -17 ± 35 (131) | -10 ± 37 (54) |

CV181- 008: 2.5, 5 & 10 mg doses, P = 0.005 between wt and SNP carriers on-treatment
CV181-011: randomized cohort, P = 0.2 between wt and SNP carriers on-treatment with baseline fasting glucose as covariate

SNP genotype frequencies: Wildtype (WT) ~70%; SNP carriers ~30%

**FIG. 11**

Association of GRINL1A SNP rs11853270 with Mean Change in AUC Glucose levels in Response to Saxagliptin Treatment

Rs11853270 represents postion 87,992 of SEQ ID NOs: 1 and 2

| Trial/Treatment | Mean Change in AUC Glucose ± SD (N) | | |
|---|---|---|---|
| | Overall | WT | SNP Carriers |
| **CV181-008** | | | |
| **Placebo** | 137 ± 2313 (66) | 343 ± 2235 (57) | -1171 ± 2504 (9) |
| **Saxagliptin** | -575 ± 2447 (86) | -1017 ± 1977 (61) | 506 ± 3121 (25) |
| **CV181-011** | | | |
| **Placebo** | -1637 ± 14004 (41) | -122 ± 15367 (25) | -4003 ± 11632 (16) |
| **Saxagliptin** | -7621 ± 10914 (132) | -8389 ± 10430 (131) | -5719 ± 11964 (38) |

CV181-008: 2.5, 5 & 10 mg doses, P = 0.002 between wt and SNP carriers on-treatment
CV181-011: randomized cohort, P = 0.09 between wt and SNP carriers on-treatment with baseline AUC glucose as covariate

SNP genotype frequencies: Wildtype (WT) ~70%; SNP carriers ~30%

**FIG. 12**

Association of HSD11B1 SNP rs17015076 with Mean Change in HbA1c levels in Response to Saxagliptin Treatment

Rs17015076 represents postion 34.403 of SEQ ID NOs: 3 and 4

| Trial/Treatment | Mean Change in HbA1c ± SD (N) | | |
|---|---|---|---|
| | Overall | WT | SNP Carriers |
| **CV181-008** | | | |
| **Placebo** | -0.3 ± 0.9 (68) | -0.4 ± 0.9 (57) | 0.1 ± 0.7 (11) |
| **Saxagliptin** | -0.7 ± 0.8 (94) | -0.7 ± 0.8 (82) | -0.1 ± 1.2 (12) |
| **CV181-011** | | | |
| **Placebo** | 0.1 ± 1.4 (59) | 0.2 ± 1.4 (49) | -0.4 ± 1.4 (10) |
| **Saxagliptin** | -0.5 ± 0.9 (181) | -0.6 ± 0.8 (158) | -0.5 ± 1.4 (23) |

CV181-008: 2.5, 5 & 10 mg doses, P = 0.008 between wt and SNP carriers on-treatment
CV181-011: randomized cohort, P = 0.09 between wt and SNP carriers on-treatment with baseline HbA1c as covariate

**FIG. 13**

Association of CHST10 SNP rs1370627 with Mean Change in HbA1c levels in Response to Saxagliptin Treatment

Rs1370627 represents postion 24,707 of SEQ ID NOs: 5 and 6

| **Trial/Treatment** | **Mean Change in HbA1c ± SD (N)** | | |
|---|---|---|---|
| | **Overall** | **WT** | **SNP Carriers** |
| **CV181-008** | | | |
| **Placebo** | -0.3 ± 0.8 (72) | -0.3 ± 0.9 (64) | -0.3 ± 0.7 (8) |
| **Saxagliptin** | -0.7 ± 0.9 (102) | -0.7 ± 0.9 (92) | -0.3 ± 1.2 (10) |
| **CV181-011** | | | |
| **Placebo** | 0.1 ± 1.3 (62) | 0.05 ± 1.3 (56) | 0.6 ± 2.3 (6) |
| **Saxagliptin** | -0.6 ± 0.9 (195) | -0.6 ± 0.9 (175) | -0.1 ± 0.9 (20) |

CV181-008: 2.5, 5 & 10 mg doses, P = 0.08 between wt and SNP carriers on-treatment
CV181-011: randomized cohort, P = 0.001 between wt and SNP carriers on-treatment with baseline HbA1c as covariate

**FIG. 14**

Association of CHST10 SNP rs6716367 with Mean Change in HbA1c levels in Response to Saxagliptin Treatment

Rs6716367 represents postion 34,078 of SEQ ID NOs: 5 and 7

| **Trial/Treatment** | **Mean Change in HbA1c ± SD (N)** | | |
|---|---|---|---|
| | **Overall** | **WT** | **SNP Carriers** |
| **CV181-008** | | | |
| **Placebo** | -0.3 ± 0.8 (73) | -0.2 ± 0.9 (62) | -0.4 ± 0.7 (11) |
| **Saxagliptin** | -0.7 ± 0.9 (98) | -0.7 ± 0.8 (87) | -0.5 ± 1.3 (11) |
| **CV181-011** | | | |
| **Placebo** | 0.1 ± 1.3 (61) | 0.0 ± 1.2 (54) | 0.9 ± 2.2 (7) |
| **Saxagliptin** | -0.6 ± 0.9 (192) | -0.6 ± 0.9 (169) | -0.3 ± 1.4 (23) |

CV181-008: 2.5, 5 & 10 mg doses, P = 0.2 between wt and SNP carriers on-treatment
CV181-011: randomized cohort, P = 0.03 between wt and SNP carriers on-treatment with baseline HbA1c as covariate

**FIG. 15**

Association of CHST10 SNP rs7585898 with Mean Change in HbA1c levels in Response to Saxagliptin Treatment

Rs7585898 represents postion 35,799 of SEQ ID NOs: 5 and 8

| Trial/Treatment | Mean Change in HbA1c ± SD (N) | | |
|---|---|---|---|
| | **Overall** | **WT** | **SNP Carriers** |
| **CV181-008** | | | |
| **Placebo** | -0.3 ± 0.9 (69) | -0.2 ± 0.9 (59) | -0.5 ± 0.7 (10) |
| **Saxagliptin** | -0.7 ± 0.9 (97) | -0.7 ± 0.8 (86) | -0.3 ± 1.4 (11) |
| **CV181-011** | | | |
| **Placebo** | 0.1 ± 1.3 (61) | 0.0 ± 1.2 (54) | 0.9 ± 2.2 (7) |
| **Saxagliptin** | -0.6 ± 0.9 (190) | -0.6 ± 0.9 (168) | -0.3 ± 1.4 (22) |

CV181-008: 2.5, 5 & 10 mg doses, P = 0.1 between wt and SNP carriers on-treatment
CV181-011: randomized cohort, P = 0.02 between wt and SNP carriers on-treatment with baseline HbA1c as covariate

**FIG. 16**

Association of CHST10 SNP rs17024136 with Mean Change in HbA1c levels in Response to Saxagliptin Treatment

Rs17024136 represents postion 38,709 of SEQ ID NOs: 5 and 9

| **Trial/Treatment** | **Mean Change in HbA1c ± SD (N)** | | |
|---|---|---|---|
| | **Overall** | **WT** | **SNP Carriers** |
| **CV181-008** | | | |
| **Placebo** | -0.3 ± 0.8 (73) | -0.2 ± 0.9 (62) | -0.4 ± 0.7 (11) |
| **Saxagliptin** | -0.7 ± 0.9 (101) | -0.7 ± 0.8 (88) | -0.3 ± 1.3 (13) |
| **CV181-011** | | | |
| **Placebo** | 0.1 ± 1.3 (61) | 0.0 ± 1.2 (54) | 0.9 ± 2.2 (7) |
| **Saxagliptin** | -0.6 ± 0.9 (194) | -0.6 ± 0.9 (171) | -0.3 ± 1.4 (23) |

CV181-008: 2.5, 5 & 10 mg doses, P = 0.04 between wt and SNP carriers on-treatment
CV181-011: randomized cohort, P = 0.03 between wt and SNP carriers on-treatment with baseline HbA1c as covariate

FIG. 17

Association of CHST10 SNP rs17024129 with Mean Change in HbA1c levels in Response to Saxagliptin Treatment

Rs17024129 represents postion 38,947 of SEQ ID NOs: 5 and 10

| Trial/Treatment | Mean Change in HbA1c ± SD (N) | | |
|---|---|---|---|
| | Overall | WT | SNP Carriers |
| CV181-008 | | | |
| Placebo | -0.3 ± 0.8 (72) | -0.2 ± 0.9 (61) | -0.4 ± 0.7 (11) |
| Saxagliptin | -0.7 ± 0.9 (101) | -0.7 ± 0.8 (88) | -0.4 ± 1.2 (13) |
| CV181-011 | | | |
| Placebo | 0.1 ± 1.3 (61) | 0.0 ± 1.2 (54) | 0.9 ± 2.2 (7) |
| Saxagliptin | -0.6 ± 0.9 (190) | -0.6 ± 0.9 (167) | -0.3 ± 1.4 (23) |

CV181-008: 2.5, 5 & 10 mg doses, P = 0.07 between wt and SNP carriers on-treatment
CV181-011: randomized cohort, P = 0.02 between wt and SNP carriers on-treatment with baseline HbA1c as covariate

**FIG. 18**

Association of CHST10 SNP rs7574684 with Mean Change in HbA1c levels in Response to Saxagliptin Treatment

Rs7574684 represents postion 41,180 of SEQ ID NOs: 5 and 11

| **Trial/Treatment** | **Mean Change in HbA1c ± SD (N)** | | |
|---|---|---|---|
| | **Overall** | **WT** | **SNP Carriers** |
| **CV181-008** | | | |
| **Placebo** | -0.3 ± 0.8 (70) | -0.2 ± 0.9 (59) | -0.4 ± 0.7 (11) |
| **Saxagliptin** | -0.7 ± 0.9 (102) | -0.7 ± 0.8 (88) | -0.3 ± 1.2 (14) |
| **CV181-011** | | | |
| **Placebo** | 0.1 ± 1.3 (61) | 0.0 ± 1.2 (54) | 0.9 ± 2.2 (7) |
| **Saxagliptin** | -0.6 ± 0.9 (191) | -0.6 ± 0.9 (168) | -0.3 ± 1.4 (23) |

CV181-008: 2.5, 5 & 10 mg doses, P = 0.04 between wt and SNP carriers on-treatment
CV181-011: randomized cohort, P = 0.02 between wt and SNP carriers on-treatment with baseline HbA1c as covariate

FIG. 19

Association of SNP rs2541508 in TEKT5 with Saxagliptin Response Clusters

Rs2541508 represents position 26,472 of SEQ ID NOs: 63 and 64

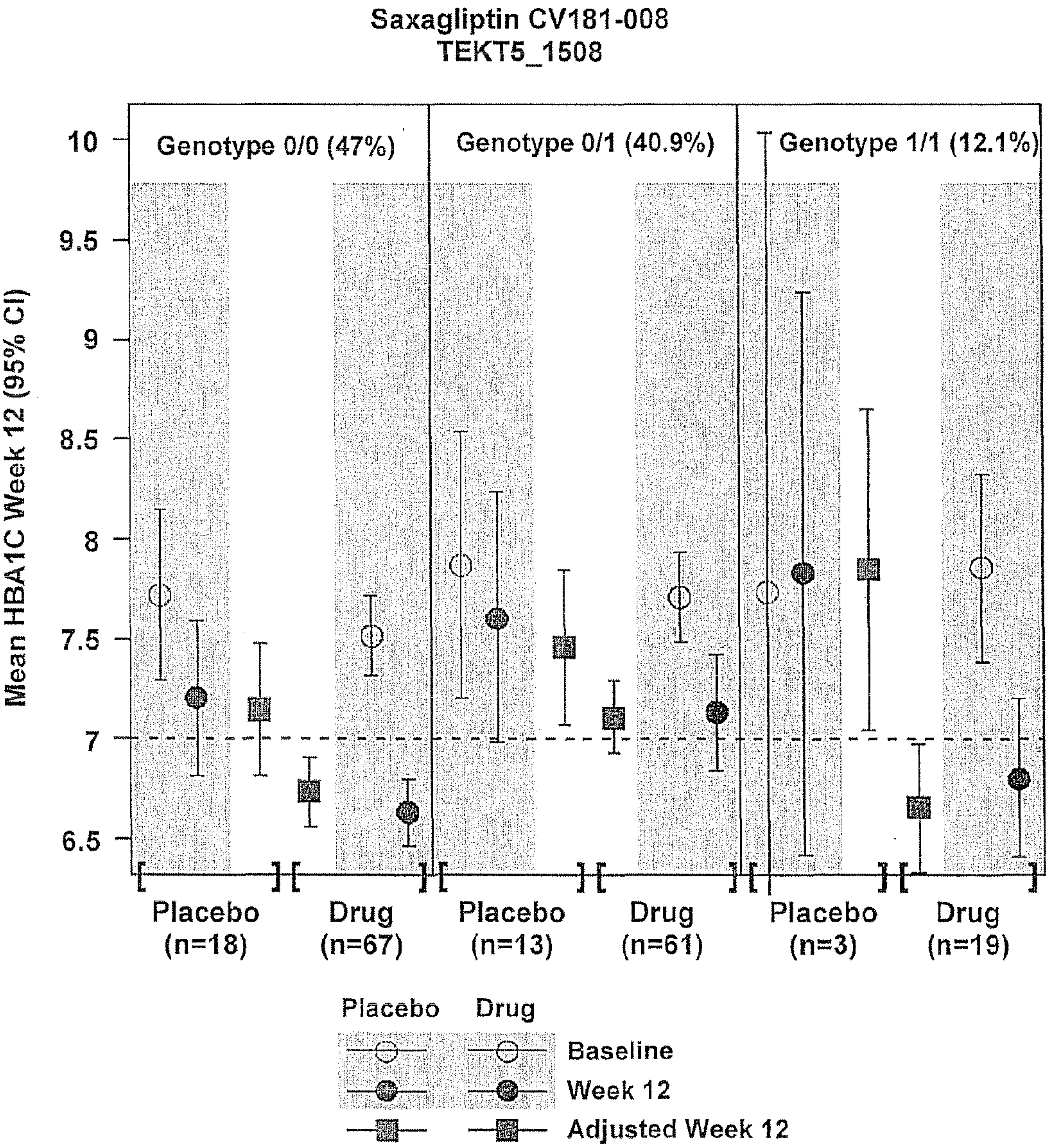


p = 0.0042

FIG. 20

Association of SNP rs11853270 in GRINL1A with Saxagliptin Response in Combination with Metformin

Rs 11853270 represents position 87,712 of SEQ ID NOs:1, 2, and 75

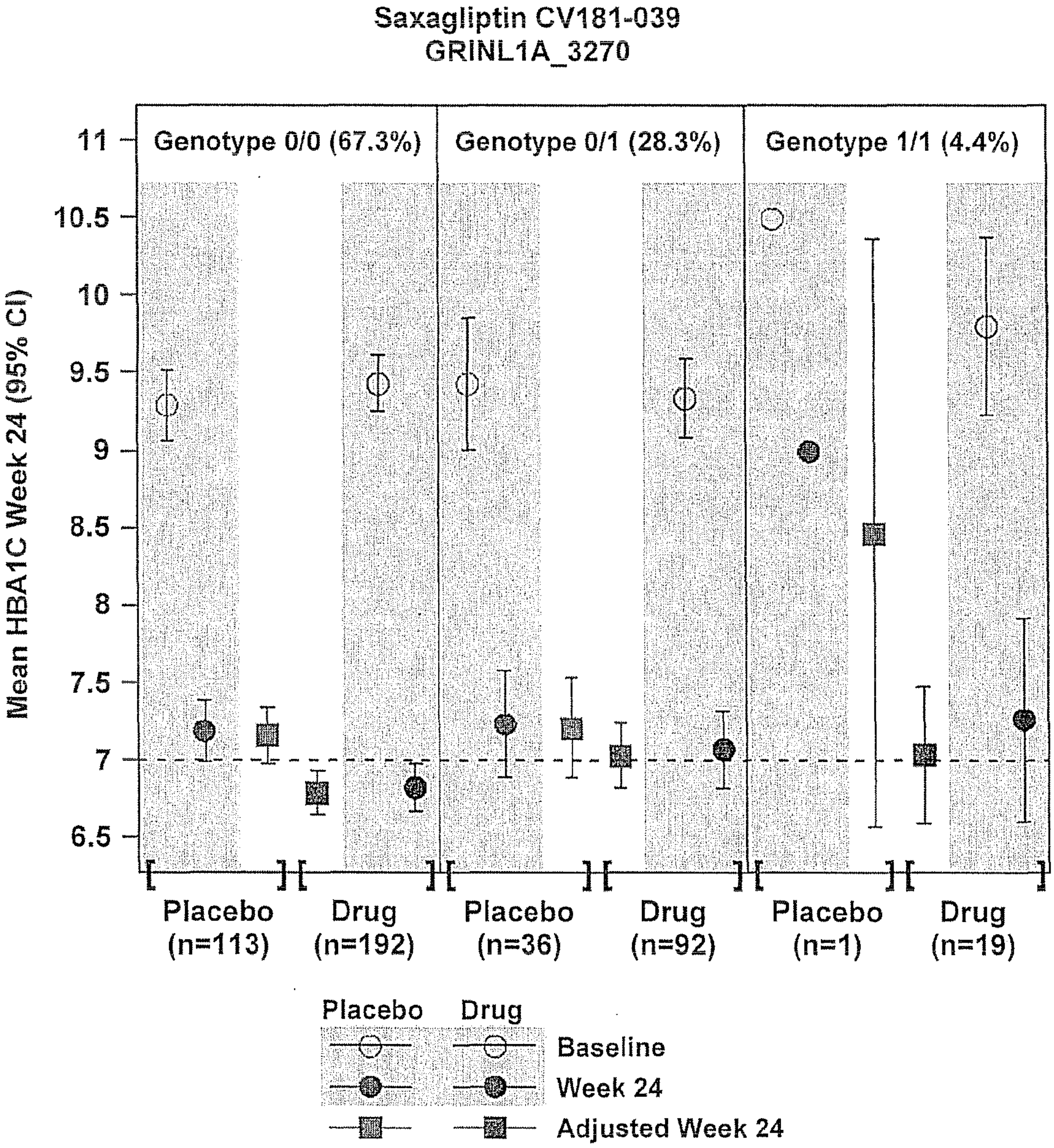


p-value = 0.0536

FIG. 21

Association of SNP rs11853270 in GRINL1A with Saxagliptin Response in Combination with Glyburide

Rs 11853270 represents position 87,712 of SEQ ID NOs:1, 2, and 75

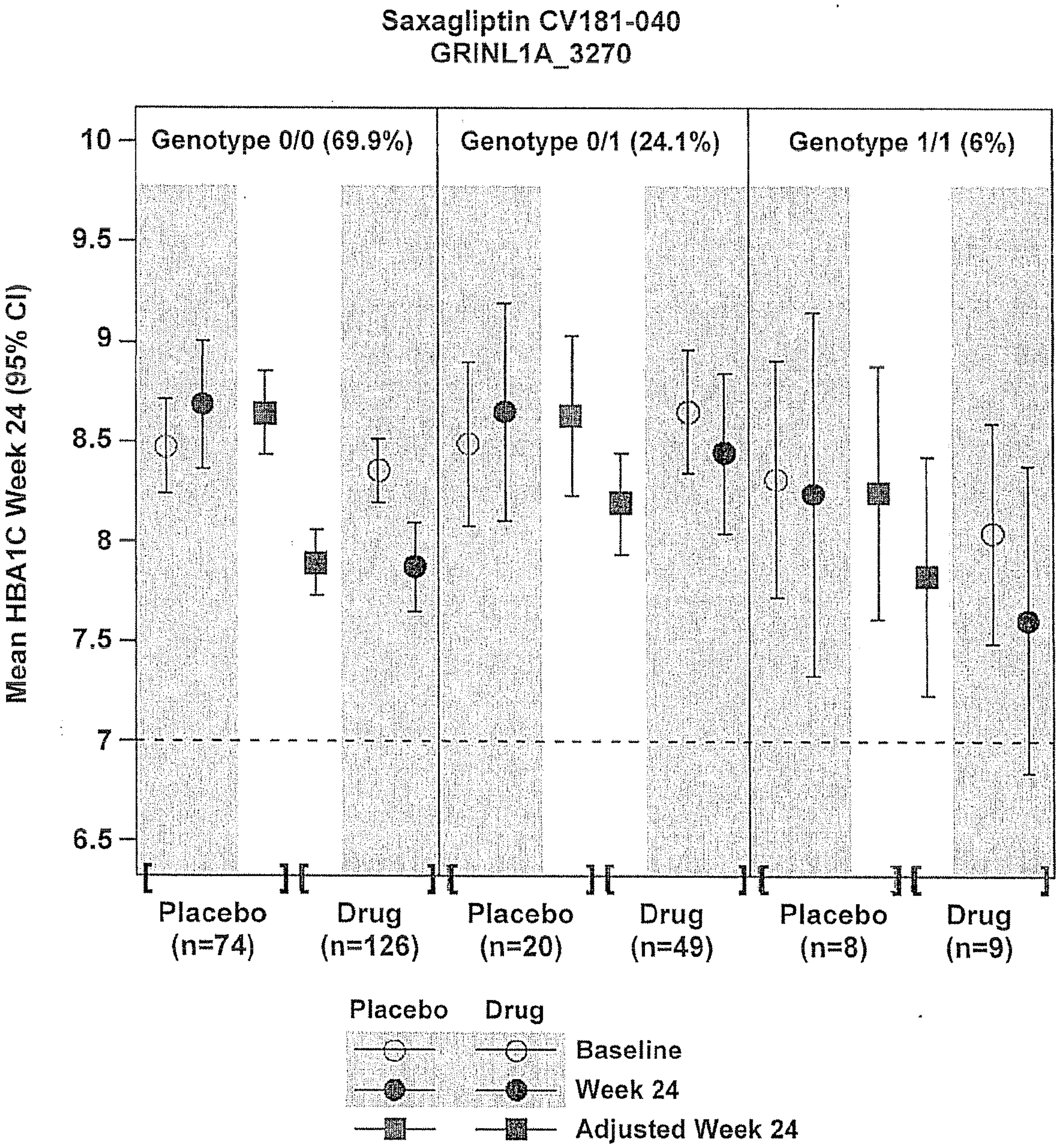


p-value = 0.0571

Europäisches Patentamt
European Patent Office
Office européen des brevets

# EUROPEAN SEARCH REPORT

Application Number
EP 15 17 5240

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE SNP [Online]<br><br>24 September 2001 (2001-09-24), XP002750701, retrieved from NCBI Database accession no. rs2541508 * the whole document * | 1-9 | INV. C12Q1/68 |
| A | DATABASE EMBL [Online]<br><br>21 May 1999 (1999-05-21), "Homo sapiens chromosome 16 clone RP11-109M19, complete sequence.", XP002750702, retrieved from EBI accession no. EM_STD:AC007595 Database accession no. AC007595 * sequence from nucleotides 108446 to 41005.; sequence * | 1-9 | |
| A | US 2005/042614 A1 (HUGHES THOMAS EDWARD [US] ET AL) 24 February 2005 (2005-02-24) * claims 1,2,5,6,47-53 * * paragraphs [0002], [0109], [0163] - [0167] * | 1-9 | TECHNICAL FIELDS SEARCHED (IPC)<br>C12Q<br>G01N |
| A,D | ROSENSTOCK J ET AL: "Glucose-lowering activity of the dipeptidyl peptidase-4 inhibitor saxagliptin in drug-naive patients with type 2 diabetes.", DIABETES, OBESITY & METABOLISM MAY 2008, vol. 10, no. 5, 18 March 2008 (2008-03-18), pages 376-386, XP002548609, ISSN: 1463-1326 * abstract * | 5-9 | |

-/--

The present search report has been drawn up for all claims

3

| Place of search | Date of completion of the search | Examiner |
|---|---|---|
| Munich | 10 November 2015 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 17 5240

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DEFRONZO RALPH A ET AL: "Saxagliptin added to metformin improves glycemic control in patients with type 2 diabetes", DIABETES, vol. 56, no. Suppl. 1, June 2007 (2007-06), page A74, XP009123660, & 67TH ANNUAL MEETING OF THE AMERICAN-DIABETES-ASSOCIATION; CHICAGO, IL, USA; JUNE 22 -26, 2007 ISSN: 0012-1797 * the whole document * ----- | 5-9 | |
| A | BARNETT A: "DPP-4 inhibitors and their potential role in the management of type 2 diabetes.", INTERNATIONAL JOURNAL OF CLINICAL PRACTICE NOV 2006, vol. 60, no. 11, November 2006 (2006-11), pages 1454-1470, XP002548611, ISSN: 1368-5031 * table 2 * * page 1463, column 1, last paragraph - column 2 * * page 1464, column 2, last paragraph - page 1465, column 2 * ----- | 5-9 | TECHNICAL FIELDS SEARCHED (IPC) |
| A,D | US 2002/019411 A1 (ROBL JEFFREY A [US] ET AL) 14 February 2002 (2002-02-14) * claims 12,14 * * example 60 * ----- | 5-9 | |

-/--

The present search report has been drawn up for all claims

3

| Place of search | Date of completion of the search | Examiner |
|---|---|---|
| Munich | 10 November 2015 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 17 5240

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CREPALDI G ET AL: "Dipeptidyl peptidase 4 (DPP-4) inhibitors and their role in Type 2 diabetes management.", JOURNAL OF ENDOCRINOLOGICAL INVESTIGATION 2007 JUL-AUG, vol. 30, no. 7, July 2007 (2007-07), pages 610-614, XP009123607, ISSN: 1720-8386 * abstract * * page 612, column 2, last paragraph - page 613, column 1, paragraph 1 * ----- | 5-9 | |
| A,P | WO 2008/098256 A1 (SQUIBB BRISTOL MYERS CO [US]; KOUSTUBH RANADE [US]) 14 August 2008 (2008-08-14) ----- | 1-9 | |
| A,P | DEACON CAROLYN F ET AL: "Saxagliptin: a new dipeptidyl peptidase-4 inhibitor for the treatment of type 2 diabetes", ADVANCES IN THERAPY, vol. 26, no. 5, 14 May 2009 (2009-05-14), pages 488-499, XP002548612, ISSN: 0741-238X * the whole document * ----- | 5-9 | TECHNICAL FIELDS SEARCHED (IPC) |
| A | US 2007/031846 A1 (CARGILL MICHELE [US] ET AL) 8 February 2007 (2007-02-08) * sequence 10554 * * paragraphs [0025], [0026], [0102], [0104], [0106] * -/-- | 1-9 | |

The present search report has been drawn up for all claims

3

| Place of search | Date of completion of the search | Examiner |
|---|---|---|
| Munich | 10 November 2015 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 17 5240

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | -& DATABASE EMBL [Online]<br><br>14 December 2010 (2010-12-14), "Sequence 10554 from patent US 7833706.", XP002750709, retrieved from EBI accession no. EM_PAT:GX773270<br>Database accession no. GX773270<br>* sequence *<br>----- | | |
| A | T. L. KARR: "Fruit flies and the sperm proteome", HUMAN MOLECULAR GENETICS, vol. 16, no. R2, 31 July 2007 (2007-07-31), pages R124-R133, XP055226995, gb<br>ISSN: 0964-6906, DOI: 10.1093/hmg/ddm252<br>* abstract *<br>* table 2 *<br>----- | 1-9 | |
| | | | TECHNICAL FIELDS SEARCHED (IPC) |

The present search report has been drawn up for all claims

3

| Place of search | Date of completion of the search | Examiner |
|---|---|---|
| Munich | 10 November 2015 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 17 5240

This annex lists the patent family members relating to the patent documents cited in the above-mentioned European search report. The members are as contained in the European Patent Office EDP file on
The European Patent Office is in no way liable for these particulars which are merely given for the purpose of information.

10-11-2015

| Patent document cited in search report | Publication date | Patent family member(s) | Publication date |
|---|---|---|---|
| US 2005042614 A1 | 24-02-2005 | BR 0213958 A | 08-09-2004 |
| | | CA 2464995 A1 | 08-05-2003 |
| | | CN 1604968 A | 06-04-2005 |
| | | EP 1470246 A2 | 27-10-2004 |
| | | JP 2005507261 A | 17-03-2005 |
| | | US 2005042614 A1 | 24-02-2005 |
| | | WO 03038123 A2 | 08-05-2003 |
| US 2002019411 A1 | 14-02-2002 | AR 027634 A1 | 02-04-2003 |
| | | AT 396176 T | 15-06-2008 |
| | | AU 4546601 A | 24-09-2001 |
| | | AU 2001245466 B2 | 12-05-2005 |
| | | BR 0109115 A | 30-12-2003 |
| | | CA 2402894 A1 | 20-09-2001 |
| | | CN 1427826 A | 02-07-2003 |
| | | CN 1698601 A | 23-11-2005 |
| | | CO 5280198 A1 | 30-05-2003 |
| | | CZ 304355 B6 | 26-03-2014 |
| | | DE 122010000008 I1 | 01-07-2010 |
| | | DE 122012000023 I1 | 09-08-2012 |
| | | DK 1261586 T3 | 29-09-2008 |
| | | EG 25854 A | 11-09-2012 |
| | | EP 1261586 A2 | 04-12-2002 |
| | | EP 1559710 A2 | 03-08-2005 |
| | | EP 2272825 A2 | 12-01-2011 |
| | | ES 2305062 T3 | 01-11-2008 |
| | | ES 2456667 T3 | 23-04-2014 |
| | | HK 1049330 A1 | 14-11-2008 |
| | | HU 0302792 A2 | 29-12-2003 |
| | | IL 151372 A | 24-12-2009 |
| | | IL 177019 A | 28-02-2013 |
| | | JP 4460205 B2 | 12-05-2010 |
| | | JP 5427047 B2 | 26-02-2014 |
| | | JP 2003531118 A | 21-10-2003 |
| | | JP 2010077163 A | 08-04-2010 |
| | | JP 2013040219 A | 28-02-2013 |
| | | JP 2014040486 A | 06-03-2014 |
| | | JP 2015134830 A | 27-07-2015 |
| | | KR 20030036140 A | 09-05-2003 |
| | | KR 20060026125 A | 22-03-2006 |
| | | LU 91650 I2 | 19-04-2010 |
| | | LU 91985 I2 | 25-06-2012 |
| | | MX PA02008837 A | 25-04-2003 |
| | | NL 300436 I1 | 01-04-2010 |
| | | NO 2010006 I1 | 03-05-2010 |
| | | NO 2012009 I1 | 04-06-2012 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 15 17 5240

This annex lists the patent family members relating to the patent documents cited in the above-mentioned European search report. The members are as contained in the European Patent Office EDP file on
The European Patent Office is in no way liable for these particulars which are merely given for the purpose of information.

10-11-2015

| Patent document cited in search report | Publication date | Patent family member(s) | Publication date |
|---|---|---|---|
| | | NO 20024295 A | 06-11-2002 |
| | | NZ 520821 A | 26-11-2004 |
| | | PE 07712002 A1 | 06-09-2002 |
| | | PL 365520 A1 | 10-01-2005 |
| | | PT 1261586 E | 04-08-2008 |
| | | RU 2286986 C2 | 10-11-2006 |
| | | SG 152030 A1 | 29-05-2009 |
| | | TW I258468 B | 21-07-2006 |
| | | US RE44186 E1 | 30-04-2013 |
| | | US 2002019411 A1 | 14-02-2002 |
| | | UY 26613 A1 | 25-10-2001 |
| | | UY 34691 A | 31-10-2014 |
| | | WO 0168603 A2 | 20-09-2001 |
| | | ZA 200206816 A | 26-11-2003 |
| WO 2008098256 A1 | 14-08-2008 | US 2011020797 A1 | 27-01-2011 |
| | | WO 2008098256 A1 | 14-08-2008 |
| US 2007031846 A1 | 08-02-2007 | CA 2514950 A1 | 12-08-2004 |
| | | EP 1590485 A2 | 02-11-2005 |
| | | JP 2009523404 A | 25-06-2009 |
| | | US 2007031846 A1 | 08-02-2007 |
| | | US 2010215645 A1 | 26-08-2010 |
| | | US 2014234291 A1 | 21-08-2014 |
| | | WO 2004067779 A2 | 12-08-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61054098 B
- US 6395767 B
- WO 2005095381 A
- US 4683195 A
- US 5837832 A
- US 5874219 A
- US 5856174 A
- US 5858659 A
- US 5856104 A
- EP 1007712 A
- US 4683202 A
- EP 235726 A, Dattagupta
- WO 8911548 A, Saiki
- WO 9511995 A
- WO 9322456 A
- US 6458540 B
- US 6440707 B


### Non-patent literature cited in the description


- **PEARSON et al.** *Diabet Med.,* 2000, vol. 17, 543-545
- **TOMLINSON et al.** 11ß-Hydroxysteroid Dehydrogenase Type 1: A Tissue-Specific Regulator of Glucocorticoid Response. *Endocrine Reviews,* 2004, vol. 25 (5), 831-866
- **DRAPER et al.** *J Clin Endocrinol Metab,* 2002, vol. 87, 4984-4990
- **ALBERTS et al.** *Diabetologia,* 2002, vol. 45, 1528-1532
- **ALBERTS et al.** *J Med Chem,* 2003, vol. 45, 3813-3815
- **SMITH et al.** *Biol Neonate,* 1982, vol. 42, 201-207
- **ANDREWS et al.** *J Clin Endocrinol Metab,* 2003, vol. 87, 5587-5593
- **RASK et al.** *J Clin Endocrinol Metab,* 2002, vol. 87, 3330-3336
- **STEWART et al.** *J Clin Endocrinol Metab,* 1999, vol. 84, 1022-1027
- **RASK et al.** *J Clin Endocrinol Metab,* 2001, vol. 86, 1418-1421
- **PATERSON JM et al.** *Proc Natl Acad Sci USA,* 2004, vol. 101, 7088-7093
- **NIELSEN et al.** *Science,* 1991, vol. 254, 1497-1500
- Glycohaemoglobin- A crucial Measurement in Modern Diabetes Management. Progress Towards Standardization and Improved Precision of Measurement. **COLMAN et al.** Consensus Statement from the Australian Diabetes Society. Royal College of Australia Association of Clinical Biochemists, 1-11
- **J. ROSENSTOCK et al.** *Diabetes, Obesity and Metabolism,* 2008, vol. 10 (5), 376-386
- **SAIKI et al.** *Science,* 1988, vol. 239, 487-491
- **MULLIS et al.** *Cold Spring Harbor Symp. Quant. Biol.,* 1987, vol. 51, 263
- PCR Technology. Stockton Press, 1989
- **EHRLICH et al.** *Science,* 1991, vol. 252, 1643-1650
- PCR Protocols, A Guide to Methods and Applications. Academic Press, 1990
- **HANDOUT.** *Adv. Mol. Biol.,* 13 September 2004
- **HAMES ; HIGGINS.** Nucleic Acid Hybridization, A Practical Approach. IPL Press, 97
- **WOLFE et al.** *Structure Fold Des.,* 2000, vol. 8, 739-50
- **KANG et al.** *J. Biol, Chem.,* 2000, vol. 275, 8742-8
- **WANG et al.** *Proc. Natl. Acad. Sci. U.S.A.,* 1999, vol. 96, 9568-73
- **MCCOLL et al.** *Proc. Natl. Acad. Sci. U.S.A.,* 1999, vol. 96, 9521-6
- **SEGAL et al.** *Proc. Natl. Acad. Sci. U.S.A.,* 1999, vol. 96, 2758-63
- **WOLFE et al.** *J. Molec. Biol.,* 1998, vol. 285, 1917-34
- **POMERANTZ et al.** *Biochemistry,* 1998, vol. 37, 965-70
- **LEON et al.** *Biol. Res.,* 2000, vol. 33, 21-30
- **BERG et al.** *Ann. Rev. Biophys. Biomol. Struct.,* 1997, vol. 26, 357-71
- **CLARK.** *Molec. Biol. Evol.,* 1990, vol. 7, 111-22
- **CLARK et al.** *Am J Hum Genet,* 1998, vol. 63, 595-612
- **FULLERTON et al.** *Am J Hum. Genet,* 2000, vol. 67, 881-900
- **TEMPLETON et al.** *Am J Hum Genet,* 2000, vol. 66, 69-83

- **EXCOFFIER ; SLATKIN.** *Mol Biol Evol,* 1995, vol. 12, 921-7
- **FALLIN ; SCHORK.** *Am J Hum Genet,* 2000, vol. 67, 947-59
- **LONG et al.** *Am J Hum Genet,* 1995, vol. 56, 799-810
- **SCHEET, P. ; STEPHENS, M.** *American Journal of Human Genetics,* 2006, vol. 78 (4), 629-644
- **STEPHENS, M. ; DONNELLY, P.** *American Journal of Human Genetics,* 2003, vol. 73 (5), 1162-1169
- **ZHANG, S. ; PAKSTIS, A. J. ; KIDD, K. K. ; ZHAO, H. ; STEPHENS, M. ; SMITH, N. J. et al.** *American Journal of Human Genetics,* 2011, vol. 69 (4), 906-914
- **KNAPP et al.** *Am J Hum Genet,* 1993, vol. 52, 1085-93
- **LI et al.** *Schizophr Res,* 1998, vol. 32, 87-92
- **MATISE.** *Genet Epidemiol,* 1995, vol. 12, 641-5
- **OTT, J.** *Genet Epidemiol,* 1989, vol. 6, 127-30
- **TERWILLIGER ; OTT.** *Hum Hered,* 1992, vol. 42, 337-46
- **NIELSEN et al.** *Science,* 1991, vol. 254, 1497
- **EGHOLM et al.** *Nature,* 1993, vol. 365, 666
- **PURCELL et al.** PLINK: a tool set for whole-genome association and population-based linkage analyses. *Amer. Journ. Hum Genet.,* 2007, vol. 81, 559-75
- **ROGINSKI et al.** *Genomics,* 2004, vol. 84 (2), 265-276
- **TANNIN et al.** *J. Biol. Chem.,* 1991, vol. 266 (25), 16653-16658
- **ONG et al.** *J. Biol. Chem.,* 1998, vol. 273 (9), 5190-5195
- **ONG et al.** *J. Biol. Chem.,* 1999, vol. 274 (36), 25608-25612
- **KANG et al.** *J. Biol. Chem.,* 2002, vol. 277 (38), 34766-34772
- **CHEN et al.** *Genome Res.,* 1999, vol. 9 (5), 492-498
- **SAMBROOK et al.** Molecular Cloning: A Laboratory Manual. Cold Spring Harbor Laboratory Press, 1989
- Current Protocols in Molecular Biology. John Wiley and Sons, Inc, 1995
- **ROZEN.** Bioinformatics Methods and Protocols in Methods of Molecular Biology. Humana Press, 2000, 365-386
- **ALTSCHUL, SF et al.** *J. Mol. Biol.,* 1990, vol. 215, 403-410
- Directed Mutagenesis Using the Polymerase Chain Reaction. **CORMACK, B.** Current Protocols in Molecular Biology. John Wiley & Sons, Inc, 2000, vol. 37, 8.5.1-8.5.10
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 4967
- PCR Methods and Applications 1. **ECKERT et al.** PCR. IRL Press, 1991
- **WU ; WALLACE.** *Genomics,* 1989, vol. 4, 560
- **LANDEGREN et al.** *Science,* 1988, vol. 241, 1077
- **KWOH et al.** *Proc. Natl. Acad. Sci. USA,* 1989, vol. 86, 1173
- **GUATELLI et al.** *Proc. Nat. Acad. Sci. USA,* 1990, vol. 87, 1874
- **SAIKI et al.** *Nature,* 1986, vol. 324, 163-166
- **GIBBS.** *Nucleic Acid Res.,* 1989, vol. 17, 2427-2448
- **SAMBROOK et al.** Molecular Cloning, A Laboratory Manual. CSHP, 1989
- **ZYSKIND et al.** Recombinant DNA Laboratory Manual. Acad. Press, 1988
- PCR Technology. Principles and Applications for DNA Amplification. W .H. Freeman and Co, 1992
- **ORITA et al.** *Proc. Nat. Acad. Sci.,* 1989, vol. 86, 2766-2770
- **CHEN et al.** *PNAS,* 1997, vol. 94, 10756-61
- **LANDEGREN, U. ; NILSSON, M. ; KWOK, P.** *Genome Res,* 1998, vol. 8, 769-776
- **KWOK, P.** *Pharmacogenomics,* 2000, vol. 1, 95-100
- **GUT, I.** *Hum Mutat,* 2001, vol. 17, 475-492
- **WHITCOMBE, D. ; NEWTON, C. ; LITTLE, S.** *Curr Opin Biotechnol,* 1998, vol. 9, 602-608
- **TILLIB, S. ; MIRZABEKOV, A.** *Curr Opin Biotechnol,* 2001, vol. 12, 53-58
- **WINZELER, E. et al.** *Science,* 1998, vol. 281, 1194-1197
- **LYAMICHEV, V. et al.** *Nat Biotechnol,* 1999, vol. 17, 292-296
- **HALL, J. et al.** *Proc Natl Acad Sci U S A,* 2000, vol. 97, 8272-8277
- **MEIN, C. et al.** *Genome Res,* 2000, vol. 10, 333-343
- **OHNISHI, Y. et al.** *J Hum Genet,* 2001, vol. 46, 471-477
- **NILSSON, M. et al.** *Science,* 1994, vol. 265, 2085-2088
- **BANER, J. ; NILSSON, M. ; MENDEL-HARTVIG, M. ; LANDEGREN, U.** *Nucleic Acids Res,* 1998, vol. 26, 5073-5078
- **BANER, J. et al.** *Curr Opin Biotechnol,* 2001, vol. 12, 11-15
- **HATCH, A. ; SANO, T. ; MISASI, J. ; SMITH, C.** *Genet Anal,* 1999, vol. 15, 35-40
- **LIZARDI, P. et al.** *Nat Genet,* 1998, vol. 19, 225-232

- **ZHONG, X. ; LIZARDI, P. ; HUANG, X. ; BRAY-WARD, P. ; WARD, D.** *Proc Natl Acad Sci U S A,* 2001, vol. 98, 3940-3945
- **FARUQI, F. et al.** *BMC Genomics,* 2001, vol. 2, 4
- **LIVAK, K.** *Gnet Anal,* 1999, vol. 14, 143-149
- **MARRAS, S. ; KRAMER, F. ; TYAGI, S.** *Genet Anal,* 1999, vol. 14, 151-156
- **RANADE, K. et al.** *Genome Res,* 2001, vol. 11, 1262-1268
- **MYAKISHEV, M. ; KHRIPIN, Y. ; HU, S. ; HAMER, D.** *Genome Re,* 2001, vol. 11, 163-169
- **BEAUDET, L. ; BEDARD, J. ; BRETON, B. ; MERCURI, R. ; BUDARF, M.** *Genome Res,* 2001, vol. 11, 600-608
- **CHEN, X. ; LEVINE, L. ; PY, K.** *Genome Res,* 1999, vol. 9, 492-498
- **GIBSON, N. et al.** *Clin Chem,* 1997, vol. 43, 1336-1341
- **LATIF, S. ; BAUER-SARDINA, I. ; RANADE, K. ; LIVAK, K. ; PY, K.** *Genome Res,* 2001, vol. 11, 436-440
- **HSU, T. ; LAW, S. ; DUAN, S. ; NERI, B. ; KWOK, P.** *Clin Chem,* 2001, vol. 47, 1373-1377
- **ALDERBORN, A. ; KRISTOFFERSON, A. ; HAMMERLING, U.** *Genome Res,* 2000, vol. 10, 1249-1258
- **RONAGHI, M. ; UHLEN, M. ; NYREN, P.** *Science,* 1998, vol. 281, 363, , 365
- **RONAGHI, M.** *Genome Res,* 2001, vol. 11, 3-11
- **PEASE, A. et al.** *Proc Natl Acad Sci U S A,* 1994, vol. 91, 5022-5026
- **SOUTHERN, E. ; MASKOS, U. ; ELDER, J.** *Genomics,* 1993, vol. 13, 1008-1017
- **WANG, D. et al.** *Science,* 1998, vol. 280, 1077-1082
- **BROWN, P. ; BOTSTEIN, D.** *Nat Genet,* 1999, vol. 21, 33-37
- **CARGILL, M. et al.** *Nat Genet,* 1999, vol. 22, 231-238
- **DONG, S. et al.** *Genome Res,* 2001, vol. 11, 1418-1424
- **HALUSHKA, M. et al.** *Nat Genet,* 1999, vol. 22, 239-247
- **HACIA, J.** *Nat Genet,* 1999, vol. 21, 42-47
- **LIPSHUTZ, R. ; FODOR, S. ; GINGERAS, T. ; LOCKHART, D.** *Nat Genet,* 1999, vol. 21, 20-24
- **SAPOLSKY, R. et al.** *Genet Anal,* 1999, vol. 14, 187-192
- **TSUCHIHASHI, Z. ; BROWN, P.** *J Virol,* 1994, vol. 68, 5863
- **HERSCHLAG, D.** *J Biol Chem,* 1995, vol. 270, 20871-20874
- **HEAD, S. et al.** *Nucleic Acids Res,* 1997, vol. 25, 5065-5071
- **NIKIFOROV, T. et al.** *Nucleic Acids Res,* 1994, vol. 22, 4167-4175
- **SYVANEN, A. et al.** *Genomics,* 1992, vol. 12, 590-595
- **SHUMAKER, J. ; METSPALU, A. ; CASKEY, C.** *Hum Mutat,* 1996, vol. 7, 346-354
- **LINDROOS, K. ; LILJEDAHL, U. ; RAITIO, M. ; SYVANEN, A.** *Nucleic Acids Res,* 2001, vol. 29, E69-9
- **LINDBLAD-TOH, K. et al.** *Nat Genet,* 2000, vol. 24, 381-386
- **PASTINEN, T. et al.** *Genome Res,* 2000, vol. 10, 1031-1042
- **FAN, J. et al.** *Genome Res,* 2000, vol. 10, 853-860
- **HIRSCHHORN, J. et al.** *Proc Natl Acad Sci U S A,* 2000, vol. 97, 12164-12169
- **BOUCHIE, A.** *Nat Biotechnol,* 2001, vol. 19, 704
- **HENSEL, M. et al.** *Science,* 1995, vol. 269, 400-403
- **SHOEMAKER, D. ; LASHKARI, D. ; MORRIS, D. ; MITTMANN, M. ; DAVIS, R.** *Nat Genet,* 1996, vol. 14, 450-456
- **GERRY, N. et al.** *J Mol Biol,* 1999, vol. 292, 251-262
- **LADNER, D. et al.** *Lab Invest,* 2001, vol. 81, 1079-1086
- **IANNONE, M. et al.** *Cytometry,* 2000, vol. 39, 131-140
- **FULTON, R. ; MCDADE, R. ; SMITH, P. ; KIENKER, L. ; KETTMAN, J. J.** *Clin Chem,* 1997, vol. 43, 1749-1756
- **ARMSTRONG, B. ; STEWART, M. ; MAZUMDER, A.** *Cytometry,* 2000, vol. 40, 102-108
- **CAI, H. et al.** *Genomics,* 2000, vol. 69, 395
- **CHEN, J. et al.** *Genome Res,* 2000, vol. 10, 549-557
- **YE, F. et al.** *Hum Mutat,* 2001, vol. 17, 305-316
- **MICHAEL, K. ; TAYLOR, L. ; SCHULTZ, S. ; WALT, D.** *Anal Chem,* 1998, vol. 70, 1242-1248
- **STEEMERS, F. ; FERGUSON, J. ; WALT, D.** *Nat Biotechnol,* 2000, vol. 18, 91-94
- **CHAN, W. ; NIE, S.** *Science,* 1998, vol. 281, 2016-2018
- **HAN, M. ; GAO, X. ; SU, J. ; NIE, S.** *Nat Biotechnol,* 2001, vol. 19, 631-635
- **GRIFFIN, T. ; SMITH, L.** *Trends Biotechnol,* 2000, vol. 18, 77-84
- **JACKSON, P. ; SCHOLL, P. ; GROOPMAN, J.** *Mol Med Today,* 2000, vol. 6, 271-276
- **HAFF, L. ; SMIRNOV, I.** *Genome Res,* 1997, vol. 7, 378-388
- **ROSS, P. ; HALL, L. ; SMIRNOV, I. ; HAFF, L.** *Nat Biotechnol,* 1998, vol. 16, 1347-1351
- **BRAY, M. ; BOERWINKLE, E. ; DORIS, P.** *Hum Mutat,* 2001, vol. 17, 296-304

- **SAUER, S. et al.** *Nucleic Acids Res,* 2000, vol. 28, E13
- **SAUER, S. et al.** *Nucleic Acids Res,* 2000, vol. 28, E100
- **SUN, X. ; DING, H. ; HUNG, K. ; GUO, B.** *Nucleic Acids Res,* 2000, vol. 28, E68
- **TANG, K. et al.** *Proc Natl Acad Sci U S A,* 1999, vol. 91, 10016-10020
- **LI, J. et al.** *Electrophoresis,* 1999, vol. 20, 1258-1265
- **LITTLE, D. ; BRAUN, A. ; O'DONNELL, M. ; KOSTER, H.** *Nat Med,* 1997, vol. 3, 1413-1416
- **LITTLE, D. et al.** *Anal Chem,* 1997, vol. 69, 4540-4546
- **GRIFFIN, T. ; TANG, W. ; SMITH, L.** *Nat Biotechnol,* 1997, vol. 15, 1368-1372
- **ROSS, P. ; LEE, K. ; BELGRADER, P.** *Anal Chem,* 1997, vol. 69, 4197-4202
- **JIANG-BAUCOM, P. ; GIRARD, J. ; BUTLER, J. ; BELGRADER, P.** *Anal Chem,* 1997, vol. 69, 4894-4898
- **GRIFFIN, T. ; HALL, J. ; PRUDENT, J. ; SMITH, L.** *Proc Natl Acad Sci U S A,* 1999, vol. 96, 6301-6306
- **KOKORIS, M. et al.** *Mol Diagn,* 2000, vol. 5, 329-340
- **TARANENKO, N. et al.** *Genet Anal,* 1996, vol. 13, 87-94
- **AOMORI, T. ; YAMAMOTO, K. ; OGUCHI-KATAYAMA, A. ; KAWAI, Y. ; ISHIDAO, T. ; MITANI, Y. et al.** *Clinical Chemistry,* 2009, vol. 55 (4), 804-812
- **KONSTANTOU, J. K. ; IOANNOU, P. C. ; CHRISTOPOULOS, T. K.** *European Journal of Human Genetics,* 2009, vol. 17 (1
- **LI, X. ; LUO, J. ; XIAO, P. ; SHI, X. ; TANG, C. ; LU, Z.** *Clinica Chimica Acta,* 2009, vol. 399 (1-2), 40-44
- **HUI, L. ; DELMONTE, T. ; RANADE, K.** *Current Protocols in Human Genetics,* 2008, 2.10.1-2.10.8
- **BROWNING, S. R. ; BROWNING, B. L.** *American Journal of Human Genetics,* 2007, vol. 81 (5), 1084-1097